# EUROPEAN PATENT SPECIFICATION

(11) **EP 2 640 845 B1**
(45) Date of publication and mention of the grant of the patent: **13.09.2017**
(21) Application number: 09726587.0
(22) Date of filing: 31.03.2009
(51) Int. Cl.: C12Q 1/68

(54) **IN VITRO DIAGNOSTICS METHOD FOR THE DIAGNOSIS OF SOMATIC AND OVARIAN CANCERS**
IN-VITRO-DIAGNOSEVERFAHREN FÜR DIE DIAGNOSE VON SOMATISCHEM KREBS UND EIERSTOCKKREBS
PROCÉDÉ DE DIAGNOSTIC IN VITRO POUR LE DIAGNOSTIC DE CANCERS SOMATIQUES ET OVARIENS

(30) Priority: 31.03.2008 EP 08290307
(43) Date of publication of application: 25.09.2013
(73) Proprietor: Université Grenoble Alpes, 38401 Saint-Martin-d'Hères (FR); INSERM (Institut National de la Santé et de la Recherche Médicale), 75654 Paris Cedex 13 (FR)
(72) Inventor: PISON-ROUSSEAUX, Sophie, F-38410 Saint Martin d'Uriage (FR); KHOCHBIN, Saadi, F-38240 Meylan (FR)
(74) Representative: Grosset-Fournier, Chantal Catherine
(86) International application number: PCT/EP2009/053809
(87) International publication number: WO 2009/121878

(56) References cited:
- EP-A- 1 316 560
- EP-A- 1 795 596
- WO-A-2006/029176
- WO-A-2007/100859
- WO-A-2008/103645
- US-A1- 2007 128 636
- CONDOMINES MAUD ET AL: "Gene expression profiling and real-time PCR analyses make it possible to identify novel potential cancer-testis antigens in multiple myeloma" BLOOD, vol. 110, no. 11, Part 1, November 2007 (2007-11), page 532A, XP008097197 & 49TH ANNUAL MEETING OF THE AMERICAN-SOCIETY-OF-HEMATOLOGY; ATLANTA, GA, USA; DECEMBER 08 -11, 2007 ISSN: 0006-4971
- PARMIGIANI RAPHAEL B ET AL: "Characterization of a cancer/testis (CT) antigen gene family capable of eliciting humoral response in cancer patients" PROCEEDINGS OF THE NATIONAL ACADEMY OF SCIENCES OF USA, NATIONAL ACADEMY OF SCIENCE, WASHINGTON, DC.; US, vol. 103, no. 48, 1 November 2006 (2006-11-01), pages 18066-18071, XP002451136 ISSN: 0027-8424
- SIMPSON A J G ET AL: "CANCER/TESTIS ANTIGENS, GAMETOGENESIS AND CANCER" NATURE REVIEWS. CANCER, NATUR PUBLISHING GROUP, LONDON, GB, vol. 5, no. 8, 1 August 2005 (2005-08-01), pages 615-625, XP008059983 ISSN: 1474-175X
- CHEN Y-T ET AL: "CANCER-TESTIS ANTIGENS: TARGETS FOR CANCER IMMUNOTHERAPY" CANCER JOURNAL FROM SCIENTIFIC AMERICAN, SCIENTIFIC AMERICAN, INC., NEW YORK, NY, US, vol. 5, no. 1, 1 January 1999 (1999-01-01), page 16/17, XP000985730 ISSN: 1081-4442
- DATABASE Geneseq [Online] 24 January 2008 (2008-01-24), "Human tissue-derived serum glycoprotein DNA SEQ ID NO:13291." XP002544606 retrieved from EBI accession no. GSN:AJL97636 Database accession no. AJL97636 & WO 2007/047796 A (INST SYSTEMS BIOLOGY [US]; ZHANG HUI [US]; AEBERSOLD RUDOLF H [CH]) 26 April 2007 (2007-04-26)
- DATABASE Geneseq [Online] 24 January 2008 (2008-01-24), "Human tissue-derived serum glycoprotein SEQ ID NO:8712." XP002544607 retrieved from EBI accession no. GSP:AJL93057 Database accession no. AJL93057
- DATABASE EMBL [Online] 20 June 2003 (2003-06-20), "Sequence 1666 from Patent EP1308459." XP002553973 retrieved from EBI accession no. EMBL:AX748141 Database accession no. AX748141 & EP 1 308 459 A (HELIX RES INST [JP]; RES ASS FOR BIOTECHNOLOGY [JP] RES ASS FOR BIOTEC) 7 May 2003 (2003-05-07)
- Helena Stabile ET AL: "Bone morphogenic protein antagonist Drm/gremlin is a novel proangiogenic factor", , 1 March 2007 (2007-03-01), pages 1834-1840, XP055121826, DOI: 10.1182/blood-2006- Retrieved from the Internet: URL:http://bloodjournal.hematologylibrary. org/content/bloodjournal/109/5/1834.full.p df#zoom=75 [retrieved on 2014-06-05]

## Description

The present invention relates to the *in vitro* diagnostic method for the diagnosis of somatic and ovarian cancers.

Spermatogenesis is a unique process of cell differentiation, involving the concerted action of a large number of factors, among which many show a testis-restricted expression pattern. Although several lists of testis-specific genes have been established for several species, including mouse (Chalmel et al. 2007; Schultz et al. 2003), until recently none was yet available for the human genes. Recently, two groups have proposed a list of human genes expressed in testis (Bock-Axelsen et al. 2007; Chen et al. 2005) . However the methods used did not allow sorting the genes according to their strict expression in testis.

Testis-specific (TS) genes are actively repressed in somatic cells. However, during cell transformation, it has been observed that some testis-specific genes are de-repressed, leading to the illegitimate expression of the encoded factors. These factors have been named "Cancer Testis" (CT) antigens, due to their ability to induce an immune response directed against them. Initially, CT factors were found deregulated in some somatic cancers. By contrast, CT factors are generally absent in the undifferentiated testicular tumors.

Actually, CT factors, coded by CT genes, correspond to genes with an expression restricted to germ cells of the testis (testis-specific genes; TS), and placenta (placenta-specific genes; PS). More particularly, their expression is confined to cells such as spermatogonia, spermatocytes, spermatids, and placental cells such as trophoblasts.

Some CTs can be expressed in nongametogenic tissues such as the pancreas, liver, and spleen at levels far below that observed in germ cells.

CT genes belong to families of genes that share common characteristics:
- they are expressed in a variety of malignant tumors,
   and
- they can be immunogenic.

More than 40 families of CT genes have been identified so far on immunogenic properties, expression profiles, and by bioinformatic methods (for reviews see (Costa et al. 2007; Kalejs and Erenpreisa 2005; Meklat et al. 2007; Scanlan et al. 2002; Scanlan et al. 2004; Simpson et al. 2005)), but little is known about their specific functions, and their functional connection with stem cell biology and cancer is widely unexplored.

CT genes are of particular interest. Their encoded factors have demonstrated their high potential as relevant diagnosis markers and therapeutic targets. Indeed these factors have been named "Cancer Testis Antigens", due to their ability to induce an immune response directed against them. To date, more than 83 families of CT genes have been identified (http://www.cta.lncc.br/, for reviews and, Chen et al. 2006 Genes Chromosomes Cancer 45: 392-400; Chen et al. 2005b Cancer Immun 5: 9; Costa et al. 2007 Stem Cells 25: 707-11; Heidebrecht et al. 2006 Clin Cancer Res 12: 4804-11; Kalejs and Erenpreisa 2005 Cancer Cell Int 5: 4; Meklat et al. 2007 Br J Haematol 136: 769-76; Scanlan et al. 2002; Scanlan et al. 2004; Simpson et al. 2005 Nat Rev Cancer 5: 615-25 ; Hofmann et al. 2008 Dec 23;105(51):20422-7).

The discovery and study of CTs have raised a lot of hope and interest, but their sporadic and unpredictable expression in cancer cells has hindered their large-scale use in cancer diagnosis and/or treatment.

Furthermore, studies have proposed strategies to identify large scale of CT genes in order to provide cancer diagnosis makers.

For example, EP 1 316 560, Condomines et al. (2007, Blood, vol. 110, no. 11, 532A) and Parmigiani et al. (2006, PNAS, vol. 103, no. 48, 18066-18071) discloses cancer/testis antigens applicable to diagnosis.

WO/2006/029176 (Scanlan et al.) relates to the use of the nucleic acid molecules, polypeptides and fragments thereof in methods and compositions for the diagnosis and treatment of diseases, such as cancer. Some putative CT testis-specific genes have been tested for their expression in somatic cancer tissues by a RT-PCR. However, this study identified too few CT genes, for use as a reliable marker of somatic cancers.

Bock-Axelsen et al. (PNAS, 2007, vol 204 pp13122-13127) have recently proposed a new method to identify genes overexpressed in human solid tumors, using a micro-array strategy. This document discloses that cancers overexpress only a few genes that are selectively expressed in the same tissue in which tumor is originated. In particular, Bock-Axelsen *et al.* describe some testis-specific genes mis-regulated in a panel of somatic tumors. Using a transcriptomic-based approach, Bock-Aselsen et al. Found testis-overexpressed genes, which are deregulated in somatic cancer, but, according to EST data (which they did not look at), most of the genes they have identified as "testis specific" or "CT" do not show a testis-restricted pattern of expression in normal cells.

In normal cells, the genome structural and functional differentiation involves epigenetic mechanisms, leading to the transcriptional silencing of many genes and the activation of a few of the tissue-specific genes (Bernstein BE, Meissner A, Lander ES (2007) Cell 128: 669-81; Li B, Carey M, Workman JL (2007) Cell 128: 707-19; Martin C, Zhang Y (2007) Curr Opin Cell Biol 19: 266-72; Rando OJ (2007) Curr Opin Genet Dev 17: 94-9). Cell transformation is associated with a global deregulation of epigenetic signalling pathways resulting in aberrant repression or de-repression of genes (Esteller 2007a Nat Rev Genet 8: 286-98; Fraga et al. 2005 Nat Genet 37: 391-400; Jones and Baylin 2007 Cell 128: 683-92). Furthermore, Schubeler and collaborators have systematically characterized the DNA methylation status of the promoter regions of the whole human genome in primary fibroblasts (representative of normal somatic cells) and in sperm cells (Weber et al. 2007 Apr;39(4):457-66). They observed that the promoters of most human genes are CpG rich (approximately 3/4 of all genes).

Whereas transcriptional silencing of critical cell regulators has clearly been involved in malignant cells transformation (Baylin 2005 Nat Clin Pract Oncol 2 Suppl 1: S4-11; Esteller 2007b Hum Mol Genet 16 Spec No 1: R50-9), the causes and consequences of the illegitimate activation of tissues-specific genes in cancer or pre-cancerous cells have not been well investigated yet.

In spite of these works, determining new TS genes as putative CT genes, no method gives satisfactory results about either the testis-specific restriction of expression of some genes, or their putative deregulation of expression of CT-genes.
So, the invention provides a reliable global identification of TS and PS liable to be miss-regulated in somatic tumor, i.e. CT genes, said CT genes being used as universal biomarkers of malignant somatic cell transformation.

The invention also provides simple, rapid and easy-to-use methods using nucleic acid molecules of CT genes, or the corresponding proteins, for the *in vitro* and *ex vivo* diagnosis of somatic and ovarian cancer.
The invention provides kits for the detection of ovarian and somatic cancers, using specific CT genes.
Moreover, the invention provides pharmaceutical compositions comprising nucleic acid molecules or proteins for the therapy of cancer.

The invention is illustrated by the use of one element chosen among:
- at least a nucleic acid molecule of the group comprising or constituted by :
   - a nucleotide sequence of the group consisting in SEQ ID NO 385 (old 641) to SEQ ID NO 414 (old 754), or,
   - a nucleotide sequence of the group consisting in SEQ ID NO 2q-1, q varying from 1 to 192 (old 320), coding for a protein comprising or constituted by an amino acid sequence belonging to the group consisting in SEQ ID NO 2q, q varying from 1 to 192 (old 320), or
   - the complementary sequence of the nucleic acid molecule thereof,
- at least a fragment of said nucleic acid molecule, said fragment comprising at least 15 contiguous nucleotides of said nucleic acid molecule,
- at least a variant of the said nucleic acid molecule, wherein the variant presents a sequence homology of at least 70%, particularly 80%, and more particularly 90% compared to said nucleic acid molecule,
for the *in vitro* or *ex vivo* diagnosis of any type of somatic or ovarian cancers, wherein at least one of any of the above described elements is abnormally expressed in cancer cells of at least one type of the somatic or ovarian cancers, and wherein each type of somatic or ovarian cancer cells abnormally expresses at least one of the above described elements.
In the invention, "a nucleotide sequence of the group consisting in SEQ ID NO 385 (old 641) to SEQ ID NO 414 (old 754)" means that the group of nucleotide comprising SEQ ID NO 385 to SEQ ID NO 754 corresponds to the group consisting in old sequences SEQ ID NO 641 to SEQ ID NO 754, disclosed in the priority document EP 08 290 307.1 filed on March 31, 2008.
In the invention, "a nucleotide sequence of the group consisting in SEQ ID NO 2q-1, q varying from 1 to 192 (old 320)" means that the group of nucleotide comprising SEQ ID NO 2q-1, q varying from 1 to 192 corresponds to the group consisting in old sequences SEQ ID NO 2q-1, q varying from 1 to 320, disclosed in the priority document EP 08 290 307.1 filed on March 31, 2008.
The prior art does not allow to determine a clear cut association between cancer and CT genes, i.e. any CT gene is miss-regulated in at least one cancer tissue and any cancer expresses at an abnormal level at least one CT gene.
The invention is illustrated by the use of at least one set of nucleic acid molecules chosen among:
- a set comprising at least 26 nucleic acid molecules chosen among the collection of 222 nucleic acid molecules represented by the nucleic acid sequences SEQ ID NO 2q-1, q varying from 1 to 192 and SEQ ID NO 385 to 414,
- a set comprising at least 26 complementary nucleic acid molecules of said at least 26 nucleic acid molecules,
- a set comprising at least one fragment of each of
   ▪ said at least 26 nucleic acid molecules, or
   ▪ said at least 26 complementary nucleic acid molecules,
   said fragments having a nucleic acid sequence comprising at least from 15 to 18 contiguous nucleotides of each of said at least 26 nucleic acid molecules, and
- a set comprising at least one variant of
   ▪ each of said at least 26 nucleic acid molecules, or
   ▪ each of said at least 26 complementary nucleic acid molecules
wherein the nucleic acid sequence of said variant presents a sequence homology of at least 70% compared to the nucleic acid sequence of said nucleic acid molecule,
said 26 nucleic acid molecules being represented by the nucleic acid sequences SEQ 2q-1, q varying from 1 to 26,
for the *in vitro* or *ex vivo* diagnosis of any type of somatic or ovarian cancers, said somatic cancers being solid tumors or hematological neoplasms,
wherein:
- cancer cells of each type of somatic or ovarian cancers abnormally express at least one nucleic acid molecule of the above sets of nucleic acid molecules, and
- at least one of nucleic acid molecule of the above sets of nucleic acid molecules is abnormally expressed in cancer cells of at least one type of somatic or ovarian cancers.

In one advantageous embodiment, the invention is illustrated by the use of at least one set of nucleic acid molecules chosen among:
- a set comprising at least 26 nucleic acid molecules chosen among the collection of 222 nucleic acid molecules represented by the nucleic acid sequences SEQ ID NO 2q-1, q varying from 1 to 192 and SEQ ID NO 385 to 414,
- a set comprising at least 26 complementary nucleic acid molecules of said at least 26 nucleic acid molecules,
- a set comprising at least one fragment of each of
   ▪ said at least 26 nucleic acid molecules, or
   ▪ said at least 26 complementary nucleic acid molecules,
   said fragments having a nucleic acid sequence comprising at least from 15 to 18 contiguous nucleotides of each of said at least 26 nucleic acid molecules, and
- a set comprising at least one variant of
   ▪ each of said at least 26 nucleic acid molecules, or
   ▪ each of said at least 26 complementary nucleic acid molecules
wherein the nucleic acid sequence of said variant presents a sequence homology of at least 70% compared to the nucleic acid sequence of said nucleic acid molecule,
said 26 nucleic acid molecules being represented by the nucleic acid sequences SEQ 2q-1, q varying from 1 to 26,
for the *in vitro* or *ex vivo* diagnosis of any type of somatic or ovarian cancers, said somatic cancers being solid tumors or hematological neoplasms,
wherein:
- at least one set of nucleic acid molecules of the above defined sets is abnormally expressed in cancer cells of at least one type of the somatic or ovarian cancers, and
- cancer cells each type of somatic or ovarian cancers abnormally express at least one set of nucleic acid molecules of the above defined sets.

The invention relates to the use of at least one set of nucleic acid molecules chosen among:
- a set comprising at least 26 nucleic acid molecules, said 26 nucleic acid molecules being represented by the nucleic acid sequences SEQ ID NO : 1, 3, 5, 7, 9, 11, 13, 15, 17, 19, 21, 23, 25, 27, 29, 31, 33, 35, 37, 39, 41, 43, 45, 47, 49 and 51,
- a set comprising at least 26 complementary nucleic acid molecules of said at least 26 nucleic acid molecules,
for the *in vitro* or *ex vivo* diagnosis of any type of somatic or ovarian cancers, said somatic cancers being solid tumors or hematological neoplasms,
wherein:
- cancer cells of each type of somatic or ovarian cancers abnormally express at least one nucleic acid molecule of the above sets of nucleic acid molecules, and
- at least one of nucleic acid molecule of the above sets of nucleic acid molecules is abnormally expressed in cancer cells of at least one type of somatic or ovarian cancers.

Another advantageous embodiment of the invention relates to the use of the set comprising at least 26 nucleic acid molecules chosen among the collection of 222 nucleic acid molecules represented by the nucleic acid sequences SEQ ID NO 2q-1, q varying from 1 to 192 and SEQ ID NO 385 to 414, said 26 nucleic acid molecules being represented by the nucleic acid sequences SEQ 2q-1, q varying from 1 to 26,
for the *in vitro* or *ex vivo* diagnosis of any type of somatic or ovarian cancers, said somatic cancers being solid tumors or hematological neoplasms,
wherein:
- cancer cells of each type of somatic or ovarian cancers abnormally express at least one nucleic acid molecule of the above set of nucleic acid molecules, and
- at least one of nucleic acid molecule of the above set of nucleic acid molecules is abnormally expressed in cancer cells of at least one type of somatic or ovarian cancers.

The invention is based on the unexpected observation that any CT gene is miss-regulated in at least one somatic and ovarian tumor, and reciprocally any somatic and ovarian cancer expresses at least a miss-regulated CT gene.
Also, the invention is based on the unexpected observation made by the inventors that a core minimal group of 26 CT genes among 222 CT genes are deregulated in at least one cancer and reciprocally any somatic and ovarian cancer expresses at least one of said 26 CT miss-regulated gene.
Also, the Inventors have shown that a subgroup of 26 genes among the collection of 222 CT genes are specific and allow to diagnose cancer with specific rate.

The results obtained by the groups of nucleic acid molecules disclosed herein and herafter in the invention are illustrated in Example 3.

According to the invention, terms "nucleic acid molecules", "nucleic acids", "oligonucleotides" and "polynucleotides" are uniformly used to define a chain of bases that characterizing a DNA molecules or an RNA molecule. These molecules are defined by the fact that they comprise or consist in a nucleic acid sequence, said sequence being a succession of bases covalently linked. The term "base" is used to define the components of the DNA or RNA, i.e. deoxyribonucleotides and ribonucleotides respectively. All the deoxyribonucleotides and ribonucleotides known in the art are concerned by the invention.
DNA molecules in the invention correspond to a gene, its transcripts when said gene is expressed, variants of said gene when they exist, or any other molecules constituted or comprising at least two bases. DNA molecules also concern the complementary nucleic acid molecules (cDNA), which result from the natural or artificial reverse transcription, i.e. DNA synthesis from RNA.
RNA molecules of the invention corresponds to a mRNA, rRNA, miRNA, or any other molecule constituted or comprising at least two bases that characterize RNA.

Preferably, the invention concerns mRNA molecules, that include, but is not limited to, full length mRNA corresponding to the complete transcription of a gene during the transcription process. All the variants, isoforms and fragments of said RNA are also considered in the invention.

According to the invention, a "variant" is defined as a polynucleotide molecule that differs from the reference polynucleotide molecule (the gene), but retains essential properties. The gene and its variants share similar polynucleotide sequences with, for example, 70 % of nucleic acids identity, preferably 80% of nucleic acids identity, more preferably or particularly 90% of nucleic acids identity, more preferably or particularly 92% of nucleic acids identity, more preferably or particularly 95% of nucleic acids identity, more preferably or particularly 98% of nucleic acids identity and more preferably or particularly 99% of nucleic acids identity. The variants of the invention can be also considered as isoforms. These variants can be the result of an alternative splicing, which result of an addition or deletion of one or more exons naturally contained in the nucleic acid sequence of the gene. Moreover, variants in the invention also concerns, but is not limited to, products of pseudo-genes, that have diverged in their sequence from the gene.
All the variants are characterized in that they have retained the essential properties of the nucleic acid molecule from which they derive.

According to the invention, fragments of nucleic acid molecule are defined by the fact that they contain at least from 15 to 18 contiguous nucleotides, advantageously they contain at least 20 nucleotides, preferably 30 nucleotides, more preferably 40 nucleotides, more preferably 60 nucleotides, more preferably 100 nucleotides. The most preferred fragments contain 60 nucleotides.
Fragments of a nucleic acid molecule can also correspond to the nucleic acid molecule corresponding to a gene wherein at least one nucleotide is suppressed. These fragments can retain some important genetic information of said nucleic acid molecule or simply can serve as oligonucleotides allowing DNA amplification, or oligonucleotide probes allowing nucleic acid molecule hybridization.
The "fragments" according to the invention corresponds then to a part of said nucleic acid molecule, and can also correspond to the complementary sequence of said part of said nucleic acid molecule. The complementarity is a concept well known in the art based on the possible interaction between purine and pyrimidine bases.
In the invention, the above mentioned molecules, fragments, variant or complementary molecules are assembled in sets. The specific set that consists in all the 222 genes of the invention is also called collection.

According to the invention, "cancer" relates to an abnormal proliferation of the cells of a determined organe. For instance, a lung cancer corresponds to an abnormal proliferation of any of the cells that form lung.
Also, in the invention, "type of cancer" designates the type of abnormal proliferation that may occur in a cancer. For instance, a lung cancer can be divided in some types such as non-small cells lung cancer or small cells lung cancer.

In the invention "cancer cells of each type of somatic or ovarian cancers abnormally express at least one nucleic acid molecule of the above set of nucleic acid molecules" means that for a determined cancer, and its types, at least one nucleic acid of the set comprising at least 26 nucleic acid molecules chosen among the nucleic acid of the collection of 222 nucleic acid molecules is abnormally expressed.

Also, for two different cancers, for instance lung cancer and pancreas cancer, the at least one nucleic acid molecule defined above can be deregulated in either lung cancer, or pancreas cancer, or deregulated in both cancer.
Moreover, a type of cancer can abnormally express two or more nucleic acid molecules defined above.

In the invention "at least one of nucleic acid molecule of the above set of nucleic acid molecules is abnormally expressed in cancer cells of at least one type of somatic or ovarian cancers" a nucleic acid molecule, or more, of the above defined group is abnormally expressed in every cancer, and in particular in every type of cancer.

According to the invention, "abnormally expressed in cancer cells" means that the above-mentioned elements are expressed at a level which is not the normal level of expression of said elements. The normal level of expression is determined in individual not afflicted by pathologies.
In the invention, the elements mentioned above are expressed specifically in testis or placenta. Their expression can be measured by commonly used methods known in the art. For example, expression level of nucleic acid molecules can be measured by methods such as Reverse-Transcription Quantitative PCR (RT-QPCR) or Northern Blotting according to a routine protocol These methods allow measuring the levels of mRNA corresponding to a particular gene (or sequence). In the first approach, the RNA from the sample (total or polyA, the latter corresponding to mRNA) is submitted to reverse transcription, in order to obtain the DNA corresponding to the complementary sequences. In Q-PCR, this DNA is then amplified by PCR, in conditions allowing a quantification of the initial amount of DNA. By using specific primers the amount of DNA corresponding to a particular sequence can be quantified. Northern blotting involves the electrophoretic separation of the RNA molecules, followed by the detection of specific sequences by hybridizing complementary sequences, used as probes (these probes are labeled).
In the testicular or placental cells of a healthy individual, said elements are expressed at a level which corresponds to a "normal level". According to the invention, said elements are not expressed in the corresponding somatic cells of said healthy individual, their expression level is null.
When somatic cells become malignant, according to the invention said malignant somatic cells express the previously described elements, which are normally not expressed in the corresponding normal somatic cells. Therefore, said elements have an expression level in malignant somatic cells higher than zero. So, in malignant somatic cells, when an element is absent in a healthy condition, its expression in a malignant condition is considered as abnormal.

According to the invention, terms "abnormally regulated", "miss-regulated" and "deregulated" are uniformly used hereafter to define a regulation in an abnormal condition, i.e. a cancer. Also, a normal condition, which refers to a normal regulation, corresponds to a condition in which cells are healthy.

According to the invention, nucleic acid molecules are characterized by their nucleic acid sequence among the nucleic acids sequences consisting in SEQ ID NO 2q-1, q varying from 1 to 192, and SEQ ID NO 385 to SEQ ID NO 414.
These nucleic acid molecules mentioned above are expressed either in testis, or in placenta, in a healthy condition.
The above-mentioned nucleic acid sequences SEQ ID NO 2q-1, q varying from 1 to 192, correspond to the following nucleic acid sequences: SEQ ID NO 1, SEQ ID NO 3, SEQ ID NO 5, SEQ ID NO 7, SEQ ID NO 9, SEQ ID NO 11, SEQ ID NO 13, SEQ ID NO 15, SEQ ID NO 17, SEQ ID NO 19, SEQ ID NO 21, SEQ ID NO 23, SEQ ID NO 25, SEQ ID NO 27, SEQ ID NO 29, SEQ ID NO 31, SEQ ID NO 33, SEQ ID NO 35, SEQ ID NO 37, SEQ ID NO 39, SEQ ID NO 41, SEQ ID NO 43, SEQ ID NO 45, SEQ ID NO 47, SEQ ID NO 49, SEQ ID NO 51, SEQ ID NO 53, SEQ ID NO 55, SEQ ID NO 57, SEQ ID NO 59, SEQ ID NO 61, SEQ ID NO 63, SEQ ID NO 65, SEQ ID NO 67, SEQ ID NO 69, SEQ ID NO 71, SEQ ID NO 73, SEQ ID NO 75, SEQ ID NO 77, SEQ ID NO 79, SEQ ID NO 81, SEQ ID NO 83, SEQ ID NO 85, SEQ ID NO 87, SEQ ID NO 89, SEQ ID NO 91, SEQ ID NO 93, SEQ ID NO 95, SEQ ID NO 97, SEQ ID NO 99, SEQ ID NO 101, SEQ ID NO 103, SEQ ID NO 105, SEQ ID NO 107, SEQ ID NO 109, SEQ ID NO 111, SEQ ID NO 113, SEQ ID NO 115, SEQ ID NO 117, SEQ ID NO 119, SEQ ID NO 121, SEQ ID NO 123, SEQ ID NO 125, SEQ ID NO 127, SEQ ID NO 129, SEQ ID NO 131, SEQ ID NO 133, SEQ ID NO 135, SEQ ID NO 137, SEQ ID NO 139, SEQ ID NO 141, SEQ ID NO 143, SEQ ID NO 145, SEQ ID NO 147, SEQ ID NO 149, SEQ ID NO 151, SEQ ID NO 153, SEQ ID NO 155, SEQ ID NO 157, SEQ ID NO 159, SEQ ID NO 161, SEQ ID NO 163, SEQ ID NO 165, SEQ ID NO 167, SEQ ID NO 169, SEQ ID NO 171, SEQ ID NO 173, SEQ ID NO 175, SEQ ID NO 177, SEQ ID NO 179, SEQ ID NO 181, SEQ ID NO 183, SEQ ID NO 185, SEQ ID NO 187, SEQ ID NO 189, SEQ ID NO 191, SEQ ID NO 193, SEQ ID NO 195, SEQ ID NO 197, SEQ ID NO 199, SEQ ID NO 201, SEQ ID NO 203, SEQ ID NO 205, SEQ ID NO 207, SEQ ID NO 209, SEQ ID NO 211, SEQ ID NO 213, SEQ ID NO 215, SEQ ID NO 217, SEQ ID NO 219, SEQ ID NO 221, SEQ ID NO 223, SEQ ID NO 225, SEQ ID NO 227, SEQ ID NO 229, SEQ ID NO 231, SEQ ID NO 233, SEQ ID NO 235, SEQ ID NO 237, SEQ ID NO 239, SEQ ID NO 241, SEQ ID NO 243, SEQ ID NO 245, SEQ ID NO 247, SEQ ID NO 249, SEQ ID NO 251, SEQ ID NO 253, SEQ ID NO 255, SEQ ID NO 257, SEQ ID NO 259, SEQ ID NO 261, SEQ ID NO 263, SEQ ID NO 265, SEQ ID NO 267, SEQ ID NO 269, SEQ ID NO 271, SEQ ID NO 273, SEQ ID NO 275, SEQ ID NO 277, SEQ ID NO 279, SEQ ID NO 281, SEQ ID NO 283, SEQ ID NO 285, SEQ ID NO 287, SEQ ID NO 289, SEQ ID NO 291, SEQ ID NO 293, SEQ ID NO 295, SEQ ID NO 297, SEQ ID NO 299, SEQ ID NO 301, SEQ ID NO 303, SEQ ID NO 305, SEQ ID NO 307, SEQ ID NO 309, SEQ ID NO 311, SEQ ID NO 313, SEQ ID NO 315, SEQ ID NO 317, SEQ ID NO 319, SEQ ID NO 321, SEQ ID NO 323, SEQ ID NO 325, SEQ ID NO 327, SEQ ID NO 329, SEQ ID NO 331, SEQ ID NO 333, SEQ ID NO 335, SEQ ID NO 337, SEQ ID NO 339, SEQ ID NO 341, SEQ ID NO 343, SEQ ID NO 345, SEQ ID NO 347, SEQ ID NO 349, SEQ ID NO 351, SEQ ID NO 353, SEQ ID NO 355, SEQ ID NO 357, SEQ ID NO 359, SEQ ID NO 361, SEQ ID NO 363, SEQ ID NO 365, SEQ ID NO 367, SEQ ID NO 369, SEQ ID NO 371, SEQ ID NO 373, SEQ ID NO 375, SEQ ID NO 377, SEQ ID NO 379, SEQ ID NO 381and SEQ ID NO 383,The definition of SEQ ID NO 2q-1 applies for all the groups according to the invention and mentioned hereafter.
The above-mentioned nucleic acid sequences SEQ ID NO 385 to SEQ ID NO 414 correspond to the following sequences: SEQ ID NO 385; SEQ ID NO 386; SEQ ID NO 387; SEQ ID NO 388; SEQ ID NO 389; SEQ ID NO 390; SEQ ID NO 391; SEQ ID NO 392; SEQ ID NO 393; SEQ ID NO 394; SEQ ID NO 395; SEQ ID NO 396; SEQ ID NO 397; SEQ ID NO 398; SEQ ID NO 399; SEQ ID NO 400; SEQ ID NO 401; SEQ ID NO 402; SEQ ID NO 403; SEQ ID NO 404; SEQ ID NO 405; SEQ ID NO 406; SEQ ID NO 407; SEQ ID NO 408; SEQ ID NO 409; SEQ ID NO 410; SEQ ID NO 411; SEQ ID NO 412; SEQ ID NO 413 and SEQ ID NO 414.
The above sequences correspond to CT genes as defined above.
Since these nucleic acid molecules they are expressed in normal cells in testis or placenta.

The following table 1 recapitulates the sequence numbers of the 222 CT genes according to the invention and the corresponding tissues expression in testis (TS) or placenta (PS).
Also, the numbering corresponding to the priority document EP 08 290 307.1 filed on March 31, 2008, is indicated in parentheses.

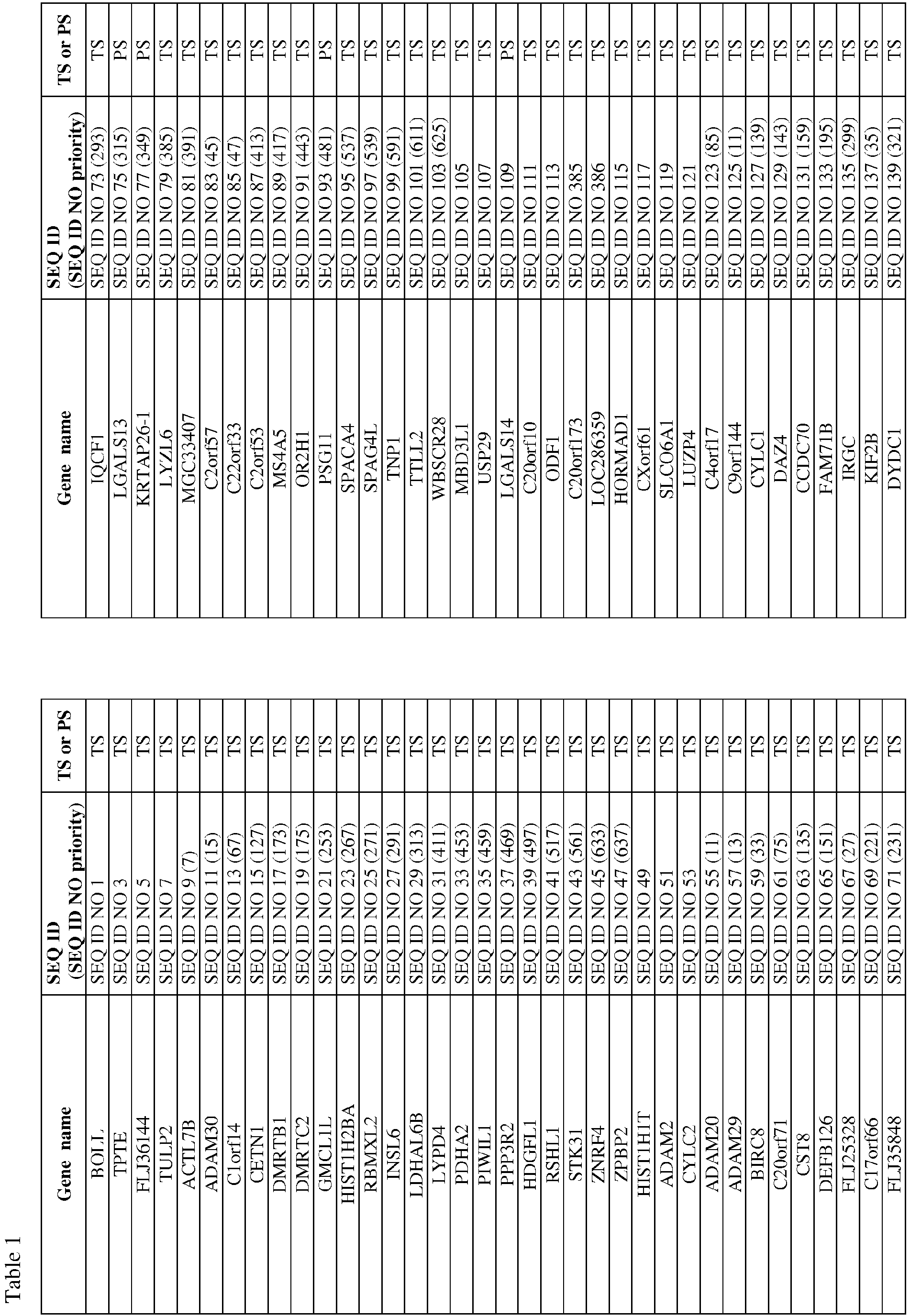

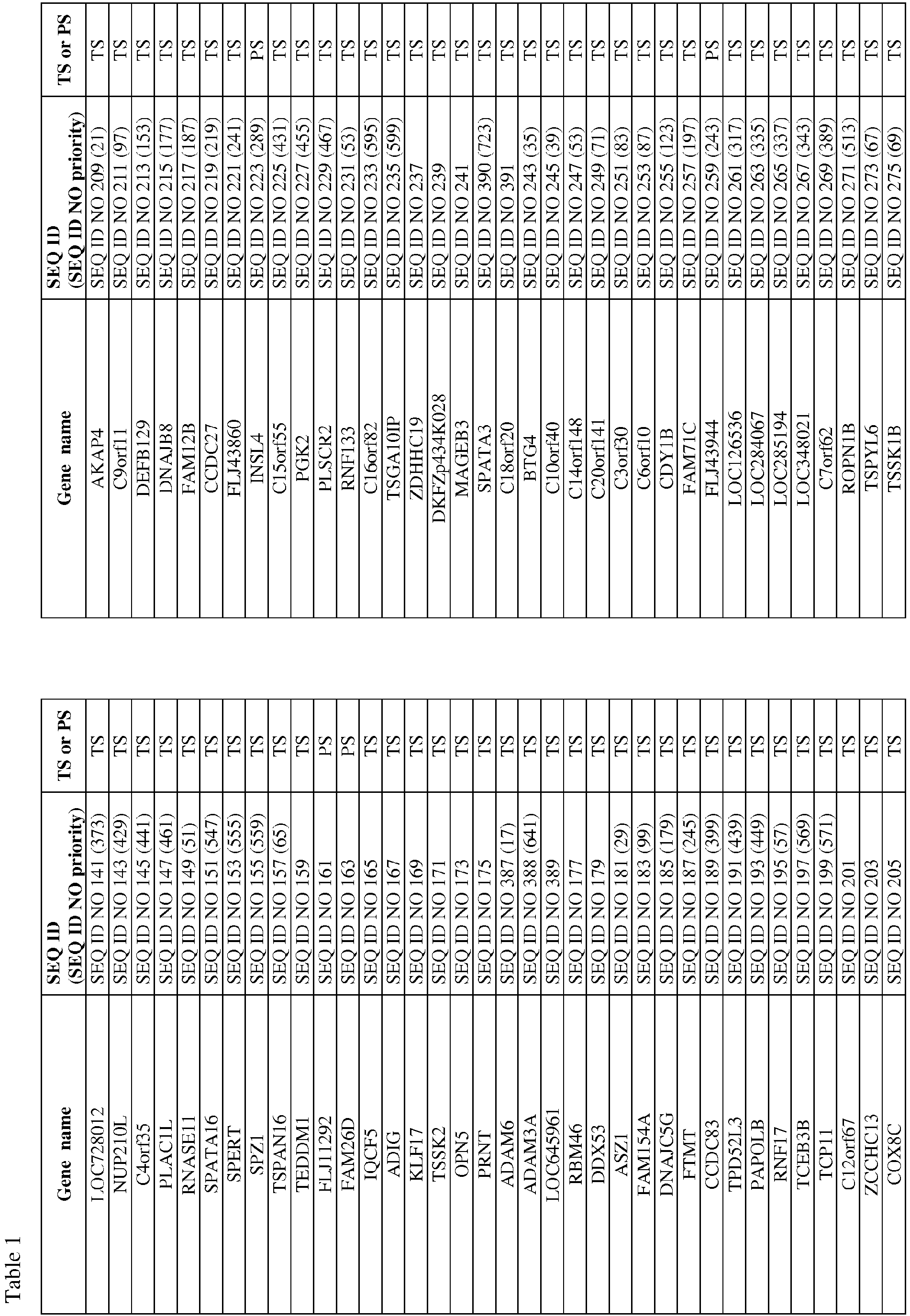

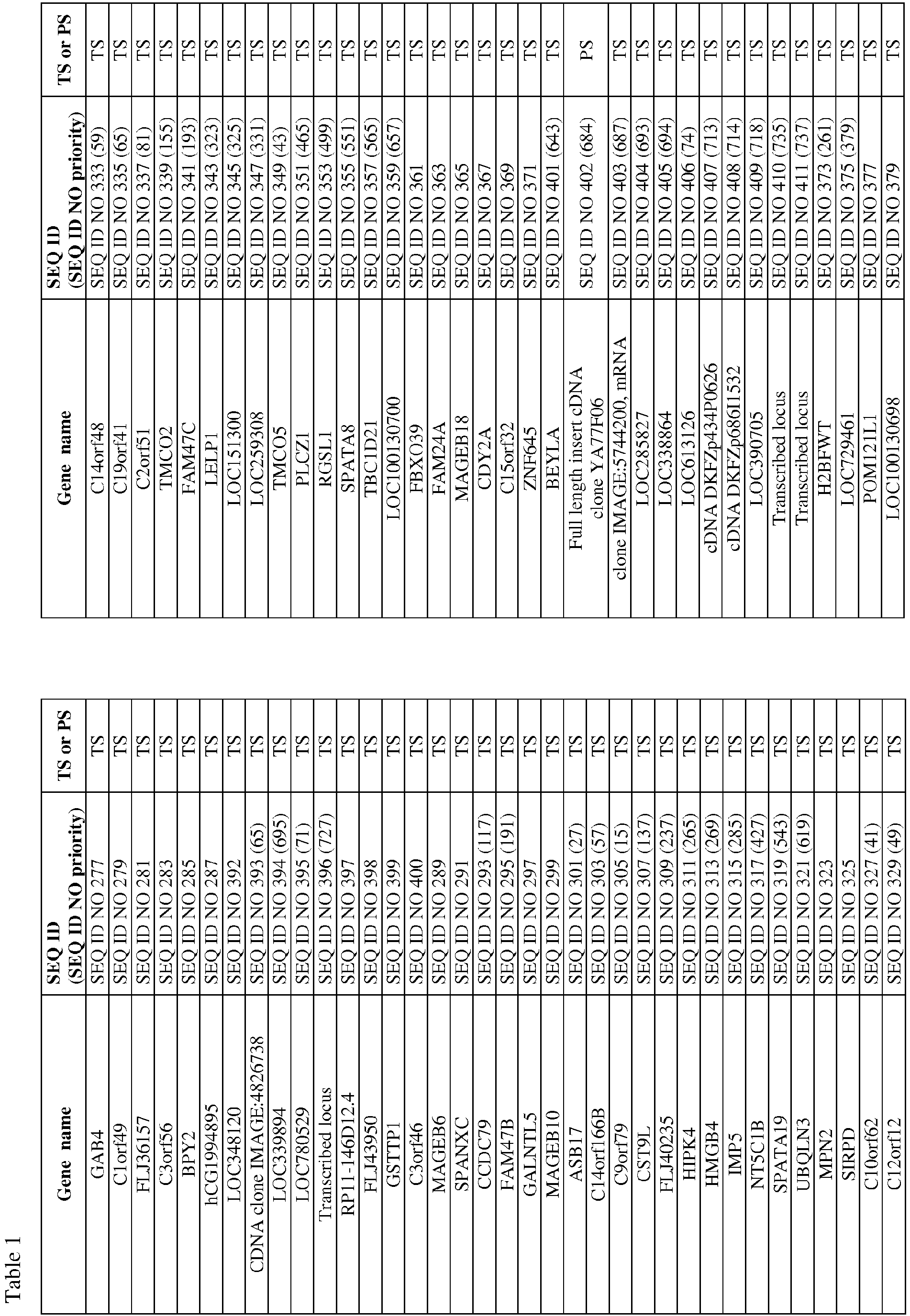

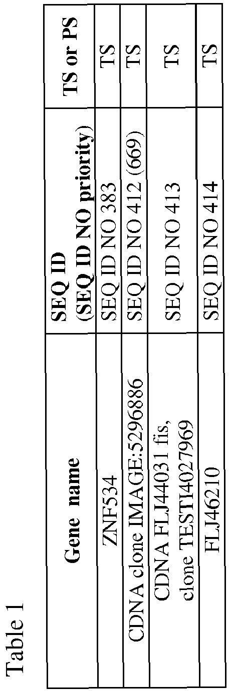

According to the invention, nucleic acids molecules characterized by the nucleic acid sequence chosen among the group consisting in SEQ ID NO 2q-1, q varying from 1 to 192, are able to code for proteins. Said proteins are characterized by their amino acids sequences chosen among the group consisting in SEQ ID NO 2q, q varying from 1 to 192.
The above-mentioned amino acid sequences SEQ ID NO 2q, q varying from 1 to 192, correspond to the following amino acid sequences: SEQ ID NO 2, SEQ ID NO 4, SEQ ID NO 6, SEQ ID NO 8, SEQ ID NO 10, SEQ ID NO 12, SEQ ID NO 14, SEQ ID NO 16, SEQ ID NO 18, SEQ ID NO 20, SEQ ID NO 22, SEQ ID NO 24, SEQ ID NO 26, SEQ ID NO 28, SEQ ID NO 30, SEQ ID NO 32, SEQ ID NO 34, SEQ ID NO 36, SEQ ID NO 38, SEQ ID NO 40, SEQ ID NO 42, SEQ ID NO 44, SEQ ID NO 46, SEQ ID NO 48, SEQ ID NO 50, SEQ ID NO 52, SEQ ID NO 54, SEQ ID NO 56, SEQ ID NO 58, SEQ ID NO 60, SEQ ID NO 62, SEQ ID NO 64, SEQ ID NO 66, SEQ ID NO 68, SEQ ID NO 70, SEQ ID NO 72, SEQ ID NO 74, SEQ ID NO 76, SEQ ID NO 78, SEQ ID NO 80, SEQ ID NO 82, SEQ ID NO 84, SEQ ID NO 86, SEQ ID NO 88, SEQ ID NO 90, SEQ ID NO 92, SEQ ID NO 94, SEQ ID NO 96, SEQ ID NO 98, SEQ ID NO 100, SEQ ID NO 102, SEQ ID NO 104, SEQ ID NO 106, SEQ ID NO 108, SEQ ID NO 110, SEQ ID NO 112, SEQ ID NO 114, SEQ ID NO 116, SEQ ID NO 118, SEQ ID NO 120, SEQ ID NO 122, SEQ ID NO 124, SEQ ID NO 126, SEQ ID NO 128, SEQ ID NO 130, SEQ ID NO 132, SEQ ID NO 134, SEQ ID NO 136, SEQ ID NO 138, SEQ ID NO 140, SEQ ID NO 142, SEQ ID NO 144, SEQ ID NO 146, SEQ ID NO 148, SEQ ID NO 150, SEQ ID NO 152, SEQ ID NO 154, SEQ ID NO 156, SEQ ID NO 158, SEQ ID NO 160, SEQ ID NO 162, SEQ ID NO 164, SEQ ID NO 166, SEQ ID NO 168, SEQ ID NO 170, SEQ ID NO 172, SEQ ID NO 174, SEQ ID NO 176, SEQ ID NO 178, SEQ ID NO 180, SEQ ID NO 182, SEQ ID NO 184, SEQ ID NO 186, SEQ ID NO 188, SEQ ID NO 190, SEQ ID NO 192, SEQ ID NO 194, SEQ ID NO 196, SEQ ID NO 198, SEQ ID NO 200, SEQ ID NO 202, SEQ ID NO 204, SEQ ID NO 206, SEQ ID NO 208, SEQ ID NO 210, SEQ ID NO 212, SEQ ID NO 214, SEQ ID NO 216, SEQ ID NO 218, SEQ ID NO 220, SEQ ID NO 222, SEQ ID NO 224, SEQ ID NO 226, SEQ ID NO 228, SEQ ID NO 230, SEQ ID NO 232, SEQ ID NO 234, SEQ ID NO 236, SEQ ID NO 238, SEQ ID NO 240, SEQ ID NO 242, SEQ ID NO 244, SEQ ID NO 246, SEQ ID NO 248, SEQ ID NO 250, SEQ ID NO 252, SEQ ID NO 254, SEQ ID NO 256, SEQ ID NO 258, SEQ ID NO 260, SEQ ID NO 262, SEQ ID NO 264, SEQ ID NO 266, SEQ ID NO 268, SEQ ID NO 270, SEQ ID NO 272, SEQ ID NO 274, SEQ ID NO 276, SEQ ID NO 278, SEQ ID NO 280, SEQ ID NO 282, SEQ ID NO 284, SEQ ID NO 286, SEQ ID NO 288, SEQ ID NO 290, SEQ ID NO 292, SEQ ID NO 294, SEQ ID NO 296, SEQ ID NO 298, SEQ ID NO 300, SEQ ID NO 302, SEQ ID NO 304, SEQ ID NO 306, SEQ ID NO 308, SEQ ID NO 310, SEQ ID NO 312, SEQ ID NO 314, SEQ ID NO 316, SEQ ID NO 318, SEQ ID NO 320, SEQ ID NO 322, SEQ ID NO 324, SEQ ID NO 326, SEQ ID NO 328, SEQ ID NO 330, SEQ ID NO 332, SEQ ID NO 334, SEQ ID NO 336, SEQ ID NO 338, SEQ ID NO 340, SEQ ID NO 342, SEQ ID NO 344, SEQ ID NO 346, SEQ ID NO 348, SEQ ID NO 350, SEQ ID NO 352, SEQ ID NO 354, SEQ ID NO 356, SEQ ID NO 358, SEQ ID NO 360, SEQ ID NO 362, SEQ ID NO 364, SEQ ID NO 366, SEQ ID NO 368, SEQ ID NO 370, SEQ ID NO 372, SEQ ID NO 374, SEQ ID NO 376, SEQ ID NO 378, SEQ ID NO 380, SEQ ID NO 382 and SEQ ID NO 384.
According to the invention, "any type of somatic and ovarian cancers" means "any type of somatic cancers" and "any type of ovarian cancer". By the way, the invention does not relate to the male gonad cancer, i.e. testicular cancer.

The term "diagnosis" means in the invention the process of identifying a medical condition or disease by its signs, symptoms, and from the results of various diagnostic procedures. It means also the recognition of a disease or condition by its outward signs and symptoms. Diagnosis corresponds also to the analysis of the underlying physiological/biochemical cause(s) of a disease or condition.
According to the invention, in vitro or ex vivo diagnosis also concerns the characterization of the type or the stage or the therapeutic follow-up of somatic and ovarian cancer.

The Inventors have unexpectedly showed that the deregulation of the expression of a group of 222 CT genes is substantially sufficient to detect all cancers and type of cancers.
Also, the study of the expression level of these above 222 CT genes can be used to
- diagnose a cancer in an individual without symptoms, or
- possibly predict the evolution of an identified tumor by, for instance, histology analysis.

The invention relates in one advantageous embodiment to the use of at least one set of nucleic acid sequences as defined above,
wherein said set of nucleic acid molecules comprises at least 59 nucleic acid molecules, said at least 59 nucleic acid molecules being represented by the nucleic acid sequences SEQ ID NO : 1, 3, 5, 7, 9, 11, 13, 15, 17, 19, 21, 23, 25, 27, 29, 31, 33, 35, 37, 39, 41, 43, 45, 47, 49, 51, 53, 55, 57, 59, 61, 63, 65, 67, 69, 71, 73, 75, 77, 79, 81, 83, 85, 87, 89, 91, 93, 95, 97, 99, 101, 103, 105, 107, 109, 111, 113, 385 and 386,
optionally, wherein said set of nucleic acid molecules comprises at least 93 nucleic acid molecules, said at least 93 nucleic acid molecules being represented by the nucleic acid sequences SEQ ID NO : 1, 3, 5, 7, 9, 11, 13, 15, 17, 19, 21, 23, 25, 27, 29, 31, 33, 35, 37, 39, 41, 43, 45, 47, 49, 51, 53, 55, 57, 59, 61, 63, 65, 67, 69, 71, 73, 75, 77, 79, 81, 83, 85, 87, 89, 91, 93, 95, 97, 99, 101, 103, 105, 107, 109, 111, 113, 385, 386, 115, 117, 119, 121, 123, 125, 127, 129, 131, 133, 135, 137, 139, 141, 143, 145, 147, 149, 151, 153, 155, 157, 159, 161, 163, 165, 167, 169, 171, 173, 175, 387, 388 and 389,
optionally, wherein said set of nucleic acid molecules comprises at least 108 nucleic acid molecules, said at least 108 nucleic acid molecules being represented by the nucleic acid sequences SEQ ID NO : 1, 3, 5, 7, 9, 11, 13, 15, 17, 19, 21, 23, 25, 27, 29, 31, 33, 35, 37, 39, 41, 43, 45, 47, 49, 51, 53, 55, 57, 59, 61, 63, 65, 67, 69, 71, 73, 75, 77, 79, 81, 83, 85, 87, 89, 91, 93, 95, 97, 99, 101, 103, 105, 107, 109, 111, 113, 385, 386, 115, 117, 119, 121, 123, 125, 127, 129, 131, 133, 135, 137, 139, 141, 143, 145, 147, 149, 151, 153, 155, 157, 159, 161, 163, 165, 167, 169, 171, 173, 175, 387, 388, 389, 177, 179, 181, 183, 185, 187, 189, 191, 193, 195, 197, 199, 201, 203 and 205,
optionally, wherein said set of nucleic acid molecules comprises at least 128 nucleic acid molecules, said at least 128 nucleic acid molecules being represented by the nucleic acid sequences SEQ ID NO : 1, 3, 5, 7, 9, 11, 13, 15, 17, 19, 21, 23, 25, 27, 29, 31, 33, 35, 37, 39, 41, 43, 45, 47, 49, 51, 53, 55, 57, 59, 61, 63, 65, 67, 69, 71, 73, 75, 77, 79, 81, 83, 85, 87, 89, 91, 93, 95, 97, 99, 101, 103, 105, 107, 109, 111, 113, 385, 386, 115, 117, 119, 121, 123, 125, 127, 129, 131, 133, 135, 137, 139, 141, 143, 145, 147, 149, 151, 153, 155, 157, 159, 161, 163, 165, 167, 169, 171, 173, 175, 387, 388, 389, 177, 179, 181, 183, 185, 187, 189, 191, 193, 195, 197, 199, 201, 203, 205, 207, 209, 211, 213, 215, 217, 219, 221, 223, 225, 227, 229, 231, 233, 235, 237, 239, 241, 390 and 391,
optionally, wherein said set of nucleic acid molecules comprises at least 160 nucleic acid molecules, said at least 160 nucleic acid molecules being represented by the nucleic acid sequences SEQ ID NO : 1, 3, 5, 7, 9, 11, 13, 15, 17, 19, 21, 23, 25, 27, 29, 31, 33, 35, 37, 39, 41, 43, 45, 47, 49, 51, 53, 55, 57, 59, 61, 63, 65, 67, 69, 71, 73, 75, 77, 79, 81, 83, 85, 87, 89, 91, 93, 95, 97, 99, 101, 103, 105, 107, 109, 111, 113, 385, 386, 115, 117, 119, 121, 123, 125, 127, 129, 131, 133, 135, 137, 139, 141, 143, 145, 147, 149, 151, 153, 155, 157, 159, 161, 163, 165, 167, 169, 171, 173, 175, 387, 388, 389, 177, 179, 181, 183, 185, 187, 189, 191, 193, 195, 197, 199, 201, 203, 205, 207, 209, 211, 213, 215, 217, 219, 221, 223, 225, 227, 229, 231, 233, 235, 237, 239, 241, 390, 391, 243, 245, 247, 249, 251, 253, 255, 257, 259, 261, 263, 265, 267, 269, 271, 273, 275, 277, 279, 281, 283, 285, 287, 392, 393, 394, 395, 396, 397, 398, 399 and 400,
optionally, wherein said set of nucleic acid molecules comprises at least 166 nucleic acid molecules, said at least 166 nucleic acid molecules being represented by the nucleic acid sequences SEQ ID NO : 1, 3, 5, 7, 9, 11, 13, 15, 17, 19, 21, 23, 25, 27, 29, 31, 33, 35, 37, 39, 41, 43, 45, 47, 49, 51, 53, 55, 57, 59, 61, 63, 65, 67, 69, 71, 73, 75, 77, 79, 81, 83, 85, 87, 89, 91, 93, 95, 97, 99, 101, 103, 105, 107, 109, 111, 113, 385, 386, 115, 117, 119, 121, 123, 125, 127, 129, 131, 133, 135, 137, 139, 141, 143, 145, 147, 149, 151, 153, 155, 157, 159, 161, 163, 165, 167, 169, 171, 173, 175, 387, 388, 389, 177, 179, 181, 183, 185, 187, 189, 191, 193, 195, 197, 199, 201, 203, 205, 207, 209, 211, 213, 215, 217, 219, 221, 223, 225, 227, 229, 231, 233, 235, 237, 239, 241, 390, 391, 243, 245, 247, 249, 251, 253, 255, 257, 259, 261, 263, 265, 267, 269, 271, 273, 275, 277, 279, 281, 283, 285, 287, 392, 393, 394, 395, 396, 397, 398, 399, 400, 289, 291, 293, 295, 297 and 299,
optionally, wherein said set of nucleic acid molecules comprises at least 179 nucleic acid molecules, said at least 179 nucleic acid molecules being represented by the nucleic acid sequences SEQ ID NO : 1, 3, 5, 7, 9, 11, 13, 15, 17, 19, 21, 23, 25, 27, 29, 31, 33, 35, 37, 39, 41, 43, 45, 47, 49, 51, 53, 55, 57, 59, 61, 63, 65, 67, 69, 71, 73, 75, 77, 79, 81, 83, 85, 87, 89, 91, 93, 95, 97, 99, 101, 103, 105, 107, 109, 111, 113, 385, 386, 115, 117, 119, 121, 123, 125, 127, 129, 131, 133, 135, 137, 139, 141, 143, 145, 147, 149, 151, 153, 155, 157, 159, 161, 163, 165, 167, 169, 171, 173, 175, 387, 388, 389, 177, 179, 181, 183, 185, 187, 189, 191, 193, 195, 197, 199, 201, 203, 205, 207, 209, 211, 213, 215, 217, 219, 221, 223, 225, 227, 229, 231, 233, 235, 237, 239, 241, 390, 391, 243, 245, 247, 249, 251, 253, 255, 257, 259, 261, 263, 265, 267, 269, 271, 273, 275, 277, 279, 281, 283, 285, 287, 392, 393, 394, 395, 396, 397, 398, 399, 400, 289, 291, 293, 295, 297, 299, 301, 303, 305, 307, 309, 311, 313, 315, 317, 319, 321, 323 and 325,
optionally, wherein said set of nucleic acid molecules comprises at least 213 nucleic acid molecules, said at least 213 nucleic acid molecules being represented by the nucleic acid sequences SEQ ID NO : 1, 3, 5, 7, 9, 11, 13, 15, 17, 19, 21, 23, 25, 27, 29, 31, 33, 35, 37, 39, 41, 43, 45, 47, 49, 51, 53, 55, 57, 59, 61, 63, 65, 67, 69, 71, 73, 75, 77, 79, 81, 83, 85, 87, 89, 91, 93, 95, 97, 99, 101, 103, 105, 107, 109, 111, 113, 385, 386, 115, 117, 119, 121, 123, 125, 127, 129, 131, 133, 135, 137, 139, 141, 143, 145, 147, 149, 151, 153, 155, 157, 159, 161, 163, 165, 167, 169, 171, 173, 175, 387, 388, 389, 177, 179, 181, 183, 185, 187, 189, 191, 193, 195, 197, 199, 201, 203, 205, 207, 209, 211, 213, 215, 217, 219, 221, 223, 225, 227, 229, 231, 233, 235, 237, 239, 241, 390, 391, 243, 245, 247, 249, 251, 253, 255, 257, 259, 261, 263, 265, 267, 269, 271, 273, 275, 277, 279, 281, 283, 285, 287, 392, 393, 394, 395, 396, 397, 398, 399, 400, 289, 291, 293, 295, 297, 299, 301, 303, 305, 307, 309, 311, 313, 315, 317, 319, 321, 323, 325, 327, 329, 331, 333, 335, 337, 339, 341, 343, 345, 347, 349, 351, 353, 355, 357, 359, 361, 363, 365, 367, 369, 371, 401, 402, 403, 404, 405, 406, 407, 408, 409, 410 and 411,
optionally, wherein said set of nucleic acid molecules comprises 222 nucleic acid molecules, said at least 222 nucleic acid molecules being represented by the nucleic acid sequences SEQ ID NO : 1, 3, 5, 7, 9, 11, 13, 15, 17, 19, 21, 23, 25, 27, 29, 31, 33, 35, 37, 39, 41, 43, 45, 47, 49, 51, 53, 55, 57, 59, 61, 63, 65, 67, 69, 71, 73, 75, 77, 79, 81, 83, 85, 87, 89, 91, 93, 95, 97, 99, 101, 103, 105, 107, 109, 111, 113, 385, 386, 115, 117, 119, 121, 123, 125, 127, 129, 131, 133, 135, 137, 139, 141, 143, 145, 147, 149, 151, 153, 155, 157, 159, 161, 163, 165, 167, 169, 171, 173, 175, 387, 388, 389, 177, 179, 181, 183, 185, 187, 189, 191, 193, 195, 197, 199, 201, 203, 205, 207, 209, 211, 213, 215, 217, 219, 221, 223, 225, 227, 229, 231, 233, 235, 237, 239, 241, 390, 391, 243, 245, 247, 249, 251, 253, 255, 257, 259, 261, 263, 265, 267, 269, 271, 273, 275, 277, 279, 281, 283, 285, 287, 392, 393, 394, 395, 396, 397, 398, 399, 400, 289, 291, 293, 295, 297, 299, 301, 303, 305, 307, 309, 311, 313, 315, 317, 319, 321, 323, 325, 327, 329, 331, 333, 335, 337, 339, 341, 343, 345, 347, 349, 351, 353, 355, 357, 359, 361, 363, 365, 367, 369, 371, 401, 402, 403, 404, 405, 406, 407, 408, 409, 410, 411, 373, 375, 377, 379, 381, 383, 412, 413 and 414.

The invention relates in one advantageous embodiment to the use of at least one set of nucleic acid sequences as defined above,
wherein said set of nucleic acid molecules comprises at least 59 nucleic acid molecules, said at least 59 nucleic acid molecules being represented by the nucleic acid sequences SEQ ID NO 2q-1, q varying from 1 to 57 and SEQ ID NO 385 to SEQ ID NO 386,
preferably, wherein said set of nucleic acid molecules comprises at least 93 nucleic acid molecules, said at least 93 nucleic acid molecules being represented by the nucleic acid sequences SEQ ID NO 2q-1, q varying from 1 to 88 and SEQ ID NO 385 to SEQ ID NO 389,
more preferably, wherein said set of nucleic acid molecules comprises at least 108 nucleic acid molecules, said at least 108 nucleic acid molecules being represented by the nucleic acid sequences SEQ ID NO 2q-1, q varying from 1 to 103 and SEQ ID NO 385 to SEQ ID NO 389,
more preferably wherein said set of nucleic acid molecules comprises at least 128 nucleic acid molecules, said at least 128 nucleic acid molecules being represented by the nucleic acid sequences SEQ ID NO 2q-1, q varying from 1 to 121 and SEQ ID NO 385 to SEQ ID NO 391, more preferably wherein said set of nucleic acid molecules comprises at least 160 nucleic acid molecules, said at least 160 nucleic acid molecules being represented by the nucleic acid sequences SEQ ID NO 2q-1, q varying from 1 to 144 and SEQ ID NO 385 to SEQ ID NO 400, more preferably wherein said set of nucleic acid molecules comprises at least 166 nucleic acid molecules, said at least 166 nucleic acid molecules being represented by the nucleic acid sequences SEQ ID NO 2q-1, q varying from 1 to 150 and SEQ ID NO 385 to SEQ ID NO 400, more preferably, wherein said set of nucleic acid molecules comprises at least 179 nucleic acid molecules, said at least 179 nucleic acid molecules being represented by the nucleic acid sequences SEQ ID NO 2q-1, q varying from 1 to 163 and SEQ ID NO 385 to SEQ ID NO 400, more preferably wherein said set of nucleic acid molecules comprises at least 213 nucleic acid molecules, said at least 213 nucleic acid molecules being represented by the nucleic acid sequences SEQ ID NO 2q-1, q varying from 1 to 186 and SEQ ID NO 385 to SEQ ID NO 411, in particular wherein said set of nucleic acid molecules comprises all the 222 nucleic acid molecules of said group of 222 nucleic acid molecules.

According to the invention, the above mentioned group of at least 26 (group 1), at least 59 (groups 1+2), at least 93 (groups 1+2+3), at least 108 (groups 1+2+3+4), at least 128 (group 1+2+3+4+5), at least 160 (groups 1+2+3+4+5+6), at least 166 (group 1+2+3+4+5+6+7), at least 179 (groups 1+2+3+4+5+6+7+8) and at least 213 (groups 1+2+3+4+5+6+7+8+9) nucleic acid molecules chosen among the collection of 222 CT genes (groups 1+2+3+4+5+6+7+8+9+0) have a specific methylation profile.

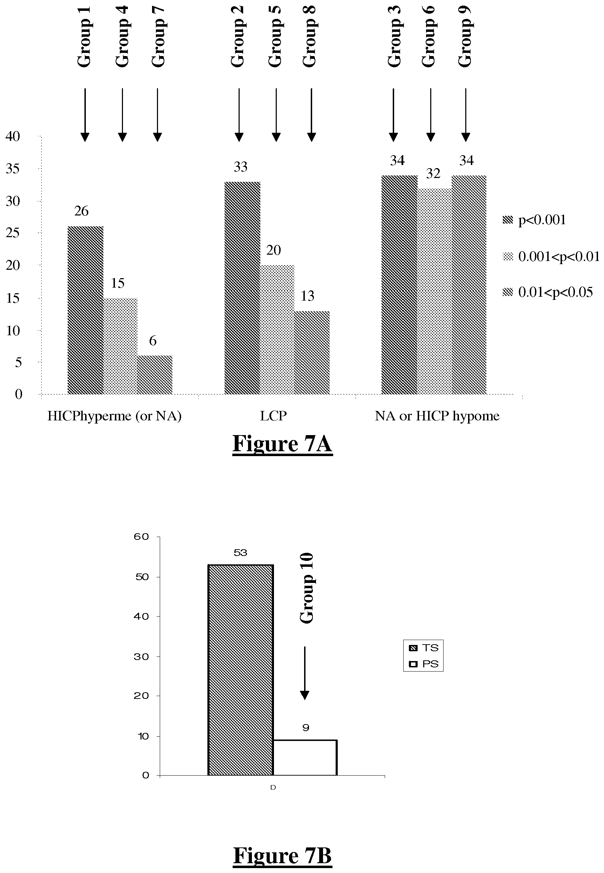

The proportion of nucleic acid molecules belonging to the groups (1-10) and the corresponding epigenetic status are indicated in figure 7.

Groups 1-10 are defined such as:
Group 1: comprises genes with CpG-rich promoters hypermethylated in somatic cells, found overexpressed in at least one oncomine study with p<0.001, n=26.
Group 2: comprises genes with CpG-poor promoters, found overexpressed in at least one oncomine study with p<0.001, n=33.
Group 3: comprises genes for which no evidence of germline cell specific epigenetic feature, found overexpressed in at least one oncomine study with p<0.001, n=34.
Group 4: comprises genes with CpG-rich promoters hypermethylated in somatic cells, found overexpressed in at least one oncomine study with 0.001<p<0.01, n=15.
Group 5: comprises genes with CpG-poor promoters, found overexpressed in at least one oncomine study with 0.001<p<0.01, n=20.
Group 6: comprises genes for which no evidence of germline cell specific epigenetic feature, found overexpressed in at least one oncomine study with 0.001<p<0.01, n=32.
Group 7: comprises genes with CpG-rich promoters hypermethylated in somatic cells, found overexpressed in at least one oncomine study with 0.01<p<0.05, n=6.
Group 8: comprises genes with CpG-poor promoters, found overexpressed in at least one oncomine study with 0.01<p<0.05, n=13.
Group 9: comprises genes for which no evidence of germline cell specific epigenetic feature, found overexpressed in at least one oncomine study with 0.01<p<0.05, n=34.
Group 10: comprises genes not available or not overexpressed in any of the selected oncomine studies but found expressed in one cancer sample on the microarray (n=9) as defined hereafter.

The invention is illustrated by the use of at least one set of amino acid molecules chosen among :
∘ a set comprising at least 26 proteins chosen among the collection of 192 proteins represented by the amino acid sequence SEQ ID NO 2q, q varying from 1 to 192,
∘ a set comprising at least one variant of each of said at least 26 proteins, wherein the amino acid sequence of said variant presents a sequence homology of at least 70% compared to the amino acid sequence of said protein,
∘ a set comprising at least one fragment of each of
   ▪ said at least 26 proteins, or
   ▪ said at least variant of each of said at least 26 proteins,
   said fragment being able to be recognized by an antibody specifically directed against an protein from which said fragment derives,
said at least 26 proteins being coded by at least at least 26 nucleic acid molecules as defined above, and said at least 26 proteins being represented by the amino acid sequences SEQ ID NO 2q, q varying from 1 to 26,
each amino acid molecule contained in a given set above-defined being specifically recognized by at least one specific antibody, and said specific antibody being able to specifically recognize one amino acid molecule of a given set above-defined,
for the *in vitro* or *ex vivo* diagnosis of any type of somatic or ovarian cancers, said somatic cancers being solid tumors or hematological neoplasms,
wherein:
- a biological sample of a patient afflicted by any type of somatic or ovarian cancer presents an abnormal amount of at least one antibody that specifically recognizes an amino acid molecule of the above sets of amino acid molecules, and
- at least one antibody that specifically recognizes an amino acid molecule of the above sets of amino acid molecules is present in an abnormal amount in a biological sample of a patient afflicted by at least one type of somatic or ovarian cancer.

In one advantageous embodiment, the invention is illustrated by the use of at least one set of amino acid molecules chosen among :
∘ a set comprising at least 26 proteins chosen among the collection of 192 proteins represented by the amino acid sequence SEQ ID NO 2q, q varying from 1 to 192,
∘ a set comprising at least one variant of each of said at least 26 proteins, wherein the amino acid sequence of said variant presents a sequence homology of at least 70% compared to the amino acid sequence of said protein,
∘ a set comprising at least one fragment of each of
   ▪ said at least 26 proteins, or
   ▪ said at least variant of each of said at least 26 proteins,
   said fragment being able to be recognized by an antibody specifically directed against an protein from which said fragment derives,
said at least 26 proteins being coded by at least at least 26 nucleic acid molecules as defined above, and said at least 26 proteins being represented by the amino acid sequences SEQ ID NO 2q, q varying from 1 to 26,
each amino acid molecule contained in a given set above-defined being specifically recognized by at least one specific antibody, and said specific antibody being able to specifically recognize one amino acid molecule of a given set above-defined,
for the *in vitro* or *ex vivo* diagnosis of any type of somatic or ovarian cancers, said somatic cancers being solid tumors or hematological neoplasms,
wherein:
- a biological sample of a patient afflicted by any type of somatic or ovarian cancer present an abnormal amount of at least a set of antibodies that specifically recognize a set of amino acid molecules of the above sets of amino acid molecules, and
- at least one a set of antibodies that specifically recognize a set of an amino acid molecule of the above sets of amino acid molecules is present in an abnormal amount in a biological sample of a patient afflicted by at least one type of somatic or ovarian cancer.

In another aspect, the invention relates to the use of at least one set of amino acid molecules chosen among:
∘ a set comprising at least 26 proteins,
∘ a set comprising at least one fragment of each of
said at least 26 proteins, said fragment being able to be recognized by an antibody specifically directed against a protein from which said fragment derives,
said at least 26 proteins being coded by at least 26 nucleic acid molecules according to claim 1, and said at least 26 proteins being represented by the amino acid sequences SEQ ID NO : 2, 4, 6, 8, 10, 12, 14, 16, 18, 20, 22, 24, 26, 28, 30, 32, 34, 36, 38, 40, 42, 44, 46, 48, 50 and 52,
each amino acid molecule contained in a given set above-defined being specifically recognized by at least one specific antibody,
for the *in vitro* or *ex vivo* diagnosis of any type of somatic or ovarian cancers, said somatic cancers being solid tumors or hematological neoplasms,
wherein:
- a biological sample of a patient afflicted by any type of somatic or ovarian cancer presents an abnormal amount of at least one antibody that specifically recognizes an amino acid molecule of the above set of amino acid molecules, and
- at least one antibody that specifically recognizes an amino acid molecule of the above sets of amino acid molecules is present in an abnormal amount in a biological sample of a patient afflicted by at least one type of somatic or ovarian cancer.

Another advantageous embodiment of the invention relates to the use of at least a set of amino acid molecule as defined above,
wherein said set of proteins comprises at least 57 proteins, said at least 57 proteins being represented by the amino acid sequences SEQ ID NO : 2, 4, 6, 8, 10, 12, 14, 16, 18, 20, 22, 24, 26, 28, 30, 32, 34, 36, 38, 40, 42, 44, 46, 48, 50, 52, 54, 56, 58, 60, 62, 64, 66, 68, 70, 72, 74, 76, 78, 80, 82, 84, 86, 88, 90, 92, 94, 96, 98, 100, 102, 104, 106, 108, 110, 112 and 114,
optionally, wherein said set of proteins comprises at least 88 proteins, said at least 88 proteins being represented by the amino acid sequences SEQ ID NO : 2, 4, 6, 8, 10, 12, 14, 16, 18, 20, 22, 24, 26, 28, 30, 32, 34, 36, 38, 40, 42, 44, 46, 48, 50, 52, 54, 56, 58, 60, 62, 64, 66, 68, 70, 72, 74, 76, 78, 80, 82, 84, 86, 88, 90, 92, 94, 96, 98, 100, 102, 104, 106, 108, 110, 112, 114, 116, 118, 120, 122, 124, 126, 128, 130, 132, 134, 136, 138, 140, 142, 144, 146, 148, 150, 152, 154, 156, 158, 160, 162, 164, 166, 168, 170, 172, 174 and 176,
optionally, wherein said set of proteins comprises at least 103 proteins, said at least 103 proteins being represented by the amino acid sequences SEQ ID NO : 2, 4, 6, 8, 10, 12, 14, 16, 18, 20, 22, 24, 26, 28, 30, 32, 34, 36, 38, 40, 42, 44, 46, 48, 50, 52, 54, 56, 58, 60, 62, 64, 66, 68, 70, 72, 74, 76, 78, 80, 82, 84, 86, 88, 90, 92, 94, 96, 98, 100, 102, 104, 106, 108, 110, 112, 114, 116, 118, 120, 122, 124, 126, 128, 130, 132, 134, 136, 138, 140, 142, 144, 146, 148, 150, 152, 154, 156, 158, 160, 162, 164, 166, 168, 170, 172, 174, 176, 178, 180, 182, 184, 186, 188, 190, 192, 194, 196, 198, 200, 202, 204 and 206,
optionally, wherein said set of proteins comprises at least 121 proteins, said at least 121 proteins being represented by the amino acid sequences SEQ ID NO : 2, 4, 6, 8, 10, 12, 14, 16, 18, 20, 22, 24, 26, 28, 30, 32, 34, 36, 38, 40, 42, 44, 46, 48, 50, 52, 54, 56, 58, 60, 62, 64, 66, 68, 70, 72, 74, 76, 78, 80, 82, 84, 86, 88, 90, 92, 94, 96, 98, 100, 102, 104, 106, 108, 110, 112, 114, 116, 118, 120, 122, 124, 126, 128, 130, 132, 134, 136, 138, 140, 142, 144, 146, 148, 150, 152, 154, 156, 158, 160, 162, 164, 166, 168, 170, 172, 174, 176, 178, 180, 182, 184, 186, 188, 190, 192, 194, 196, 198, 200, 202, 204, 206, 208, 210, 212, 214, 216, 218, 220, 222, 224, 226, 228, 230, 232, 234, 236, 238, 240 and 242,
optionally, wherein said set of proteins comprises at least 144 proteins, said at least 144 proteins being represented by the amino acid sequences SEQ ID NO : 2, 4, 6, 8, 10, 12, 14, 16, 18, 20, 22, 24, 26, 28, 30, 32, 34, 36, 38, 40, 42, 44, 46, 48, 50, 52, 54, 56, 58, 60, 62, 64, 66, 68, 70, 72, 74, 76, 78, 80, 82, 84, 86, 88, 90, 92, 94, 96, 98, 100, 102, 104, 106, 108, 110, 112, 114, 116, 118, 120, 122, 124, 126, 128, 130, 132, 134, 136, 138, 140, 142, 144, 146, 148, 150, 152, 154, 156, 158, 160, 162, 164, 166, 168, 170, 172, 174, 176, 178, 180, 182, 184, 186, 188, 190, 192, 194, 196, 198, 200, 202, 204, 206, 208, 210, 212, 214, 216, 218, 220, 222, 224, 226, 228, 230, 232, 234, 236, 238, 240, 242, 244, 246, 248, 250, 252, 254, 256, 258, 260, 262, 264, 266, 268, 270, 272, 274, 276, 278, 280, 282, 284, 286 and 288,
optionally, wherein said set of proteins comprises at least 150 proteins, said at least 150 proteins being represented by the amino acid sequences SEQ ID NO : 2, 4, 6, 8, 10, 12, 14, 16, 18, 20, 22, 24, 26, 28, 30, 32, 34, 36, 38, 40, 42, 44, 46, 48, 50, 52, 54, 56, 58, 60, 62, 64, 66, 68, 70, 72, 74, 76, 78, 80, 82, 84, 86, 88, 90, 92, 94, 96, 98, 100, 102, 104, 106, 108, 110, 112, 114, 116, 118, 120, 122, 124, 126, 128, 130, 132, 134, 136, 138, 140, 142, 144, 146, 148, 150, 152, 154, 156, 158, 160, 162, 164, 166, 168, 170, 172, 174, 176, 178, 180, 182, 184, 186, 188, 190, 192, 194, 196, 198, 200, 202, 204, 206, 208, 210, 212, 214, 216, 218, 220, 222, 224, 226, 228, 230, 232, 234, 236, 238, 240, 242, 244, 246, 248, 250, 252, 254, 256, 258, 260, 262, 264, 266, 268, 270, 272, 274, 276, 278, 280, 282, 284, 286, 288, 290, 292, 294, 296, 298 and 300,
optionally, wherein said set of proteins comprises at least 163 proteins, said at least 163 proteins being represented by the amino acid sequences SEQ ID NO : 2, 4, 6, 8, 10, 12, 14, 16, 18, 20, 22, 24, 26, 28, 30, 32, 34, 36, 38, 40, 42, 44, 46, 48, 50, 52, 54, 56, 58, 60, 62, 64, 66, 68, 70, 72, 74, 76, 78, 80, 82, 84, 86, 88, 90, 92, 94, 96, 98, 100, 102, 104, 106, 108, 110, 112, 114, 116, 118, 120, 122, 124, 126, 128, 130, 132, 134, 136, 138, 140, 142, 144, 146, 148, 150, 152, 154, 156, 158, 160, 162, 164, 166, 168, 170, 172, 174, 176, 178, 180, 182, 184, 186, 188, 190, 192, 194, 196, 198, 200, 202, 204, 206, 208, 210, 212, 214, 216, 218, 220, 222, 224, 226, 228, 230, 232, 234, 236, 238, 240, 242, 244, 246, 248, 250, 252, 254, 256, 258, 260, 262, 264, 266, 268, 270, 272, 274, 276, 278, 280, 282, 284, 286, 288, 290, 292, 294, 296, 298, 300, 302, 304, 306, 308, 310, 312, 314, 316, 318, 320, 322, 324 and 326,
optionally, wherein said set of proteins comprises at least 186 proteins, said at least 186 proteins being represented by the amino acid sequences SEQ ID NO : 2, 4, 6, 8, 10, 12, 14, 16, 18, 20, 22, 24, 26, 28, 30, 32, 34, 36, 38, 40, 42, 44, 46, 48, 50, 52, 54, 56, 58, 60, 62, 64, 66, 68, 70, 72, 74, 76, 78, 80, 82, 84, 86, 88, 90, 92, 94, 96, 98, 100, 102, 104, 106, 108, 110, 112, 114, 116, 118, 120, 122, 124, 126, 128, 130, 132, 134, 136, 138, 140, 142, 144, 146, 148, 150, 152, 154, 156, 158, 160, 162, 164, 166, 168, 170, 172, 174, 176, 178, 180, 182, 184, 186, 188, 190, 192, 194, 196, 198, 200, 202, 204, 206, 208, 210, 212, 214, 216, 218, 220, 222, 224, 226, 228, 230, 232, 234, 236, 238, 240, 242, 244, 246, 248, 250, 252, 254, 256, 258, 260, 262, 264, 266, 268, 270, 272, 274, 276, 278, 280, 282, 284, 286, 288, 290, 292, 294, 296, 298, 300, 302, 304, 306, 308, 310, 312, 314, 316, 318, 320, 322, 324, 326, 328, 330, 332, 334, 336, 338, 340, 342, 344, 346, 348, 350, 352, 354, 356, 358, 360, 362, 364, 366, 368, 370 and 372,
optionally, wherein said set of proteins comprises 192 proteins, said proteins being represented by the amino acid sequences SEQ ID NO : 2, 4, 6, 8, 10, 12, 14, 16, 18, 20, 22, 24, 26, 28, 30, 32, 34, 36, 38, 40, 42, 44, 46, 48, 50, 52, 54, 56, 58, 60, 62, 64, 66, 68, 70, 72, 74, 76, 78, 80, 82, 84, 86, 88, 90, 92, 94, 96, 98, 100, 102, 104, 106, 108, 110, 112, 114, 116, 118, 120, 122, 124, 126, 128, 130, 132, 134, 136, 138, 140, 142, 144, 146, 148, 150, 152, 154, 156, 158, 160, 162, 164, 166, 168, 170, 172, 174, 176, 178, 180, 182, 184, 186, 188, 190, 192, 194, 196, 198, 200, 202, 204, 206, 208, 210, 212, 214, 216, 218, 220, 222, 224, 226, 228, 230, 232, 234, 236, 238, 240, 242, 244, 246, 248, 250, 252, 254, 256, 258, 260, 262, 264, 266, 268, 270, 272, 274, 276, 278, 280, 282, 284, 286, 288, 290, 292, 294, 296, 298, 300, 302, 304, 306, 308, 310, 312, 314, 316, 318, 320, 322, 324, 326, 328, 330, 332, 334, 336, 338, 340, 342, 344, 346, 348, 350, 352, 354, 356, 358, 360, 362, 364, 366, 368, 370, 372, 374, 376, 378, 380, 382 and 384.

Another advantageous embodiment of the invention relates to the use of at least a set of amino acid molecule as defined above
wherein said set of proteins comprises at least 57 proteins, said at least 57 proteins being represented by the amino acid sequences SEQ ID NO 2q, q varying from 1 to 57,
preferably, wherein said set of proteins comprises at least 88 proteins, said at least 88 proteins being represented by the amino acid sequences SEQ ID NO 2q, q varying from 1 to 88,
more preferably, wherein said set of proteins comprises at least 103 proteins, said at least 103 proteins being represented by the amino acid sequences SEQ ID NO 2q, q varying from 1 to 103,
more preferably wherein said set of proteins comprises at least 121 proteins, said at least 121 proteins being represented by the amino acid sequences SEQ ID NO 2q, q varying from 1 to 121,
more preferably wherein said set of proteins comprises at least 144 proteins, said at least 144 proteins being represented by the amino acid sequences SEQ ID NO 2q, q varying from 1 to 144,
more preferably wherein said set of proteins comprises at least 150 proteins, said at least 150 proteins being represented by the amino acid sequences SEQ ID NO 2q, q varying from 1 to 150,
more preferably, wherein said set of proteins comprises at least 163 proteins, said at least 163 proteins being represented by the amino acid sequences SEQ ID NO 2q, q varying from 1 to 163,
more preferably wherein said set of proteins comprises at least 186 proteins, said at least 186 proteins being represented by the amino acid sequences SEQ ID NO 2q, q varying from 1 to 186,
in particular wherein said set of proteins comprises all the 192 proteins of said group of 192 proteins.

The above mentioned protein SEQ ID NO 2q, q varying from 1 to 192 (old 320), correspond to a protein coding by the nucleic acid molecules SEQ ID NO 2q-1, q varying from 1 to 192 (old 320).
As examples, according to the invention, said proteins can be defined such that SEQ ID NO 2 coded by nucleic acid molecule SEQ ID NO 1, SEQ ID NO 4 coded by nucleic acid molecule SEQ ID NO 3, SEQ ID NO 6 coded by nucleic acid molecule SEQ ID NO 5, SEQ ID NO 8 coded by nucleic acid molecule SEQ ID NO 7, etc...

According to the invention terms "amino acid molecules" and "proteins" are uniformly used to define a chain of amino acids. These molecules are defined by the fact that they comprise or consist in an amino acid sequence, said sequence being a succession of amino acids covalently linked.
According to the invention, a "variant" is defined as an amino acid molecule that differs from the reference amino acid molecule (the protein), but retains essential properties. The protein and its variants share similar amino acid sequences with, for example, 70 % of amino acids identity, preferably 80% of amino acids identity, more preferably or particularly 90% of amino acids identity, more preferably or particularly 92% of amino acids identity, more preferably or particularly 95% of amino acids identity, more preferably or particularly 98% of amino acids identity and more preferably or particularly 99% of amino acids identity. The variants of the invention can be also considered as isoforms. These variants can be the result of an alternative splicing, which result of an addition or deletion of one or more exons naturally contained in the nucleic acid sequence of the gene coding for a protein.
All the variants are characterized in that they have retained the essential properties of the amino acid molecule from which they derive.

According to the invention, the proteins or amino acid molecules are able to be recognized by specific antibodies, the interaction between an amino acid molecule and its specific antibody forming an immune complex. The interaction is called "specific" since an antibody recognizes a protein, or a variant of said protein, but is not able to recognize another different protein.

By "a biological sample of a patient afflicted by any type of somatic or ovarian cancer presents an abnormal amount of at least one antibody that specifically recognizes an amino acid molecule" it is defined in the invention that the proteins of the sets are able to detect antibodies liable to present in a biological sample of a subject afflicted by a cancer, or liable to be present in an amount different to the amount of said antibody in a biological sample of an healthy individual.

Thus, in a set of protein according to the invention, each protein is able to recognize at least one antibody of the individual's sample, and each antibody contained in the sample is able to be recognized by one protein of the set.

The following table 2 summarizes the correspondence between nucleic acid molecules SEQ ID NO 2q-1 and the corresponding protein SEQ ID NO 2q, coded by said nucleic acids.
The table 2 also describes the cells wherein nucleic acid molecules and protein are normally expressed. PS: Placental-specific genes, TS Testis-specific genes.

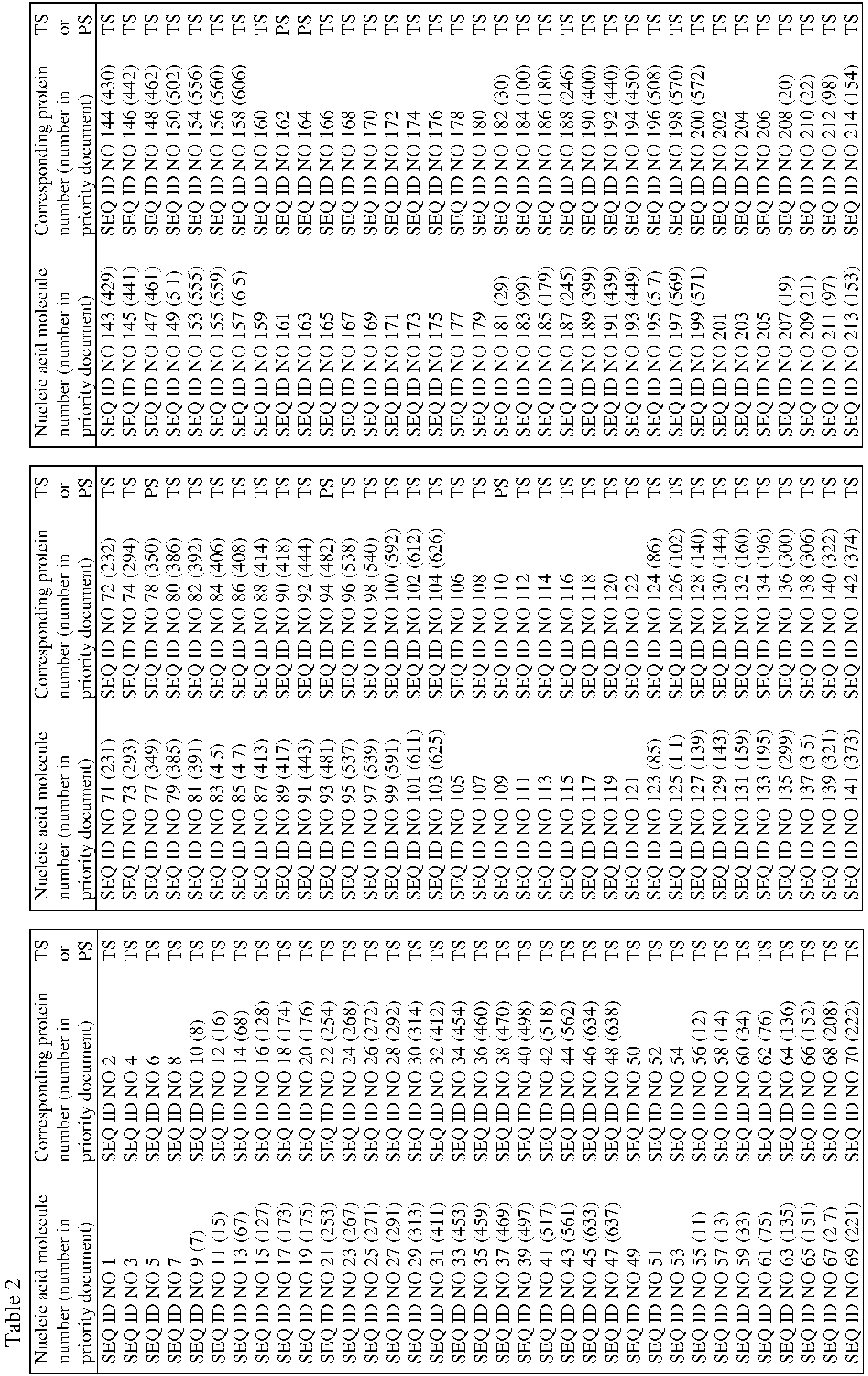

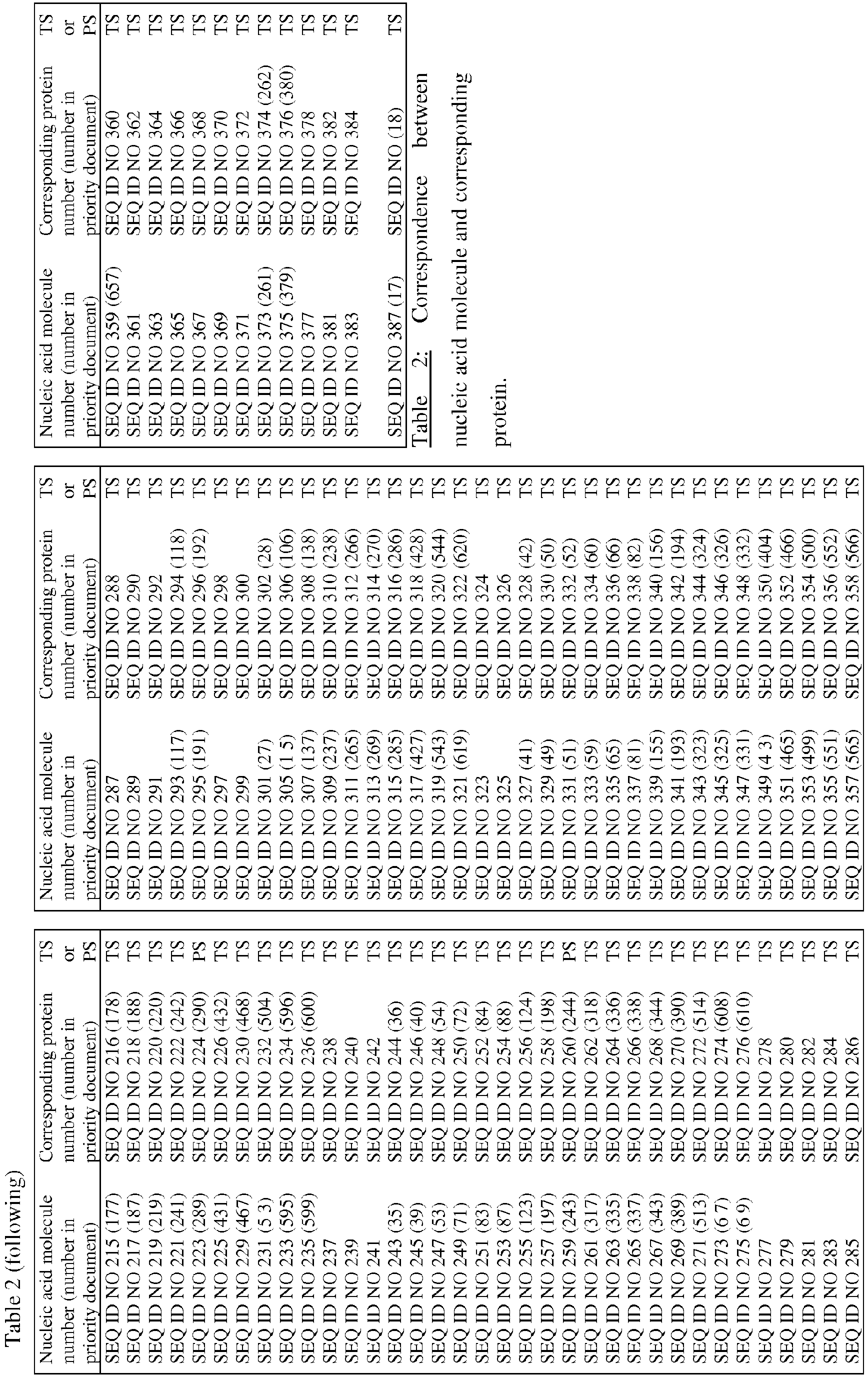

In the invention, the least 26, at least 57, at least 88, at least 103, at least 121, at least 144, at least 150, at least 163 and at least 186 amino acid molecules chosen among the collection of 192 CT amino acid molecules refers to the amino acid molecules that are expressed by the least 26 (group 1), at least 59 (group 2), at least 93 (group 3), at least 108 (group 4), at least 128 (group 5), at least 160 (group 6), at least 166 (group 7), at least 179 (group 8) and at least 213 (group 9) nucleic acid molecules chosen among the collection of 222 CT genes (group 10) respectively, as defined above.

The invention also relates to the use of a set of at least 26 antibodies, optionally a set of 57 antibodies, optionally a set of 88 antibodies, optionally a set of 103 antibodies, optionally a set of 121 antibodies, optionally a set of 150 antibodies, optionally a set of 163 antibodies, optionally a set of 186 antibodies, optionally a set of 192 antibodies characterized in that it each antibody of a given mentioned set of antibodies specifically recognizes an amino acid molecule of a set of amino acid molecules as defined above, and each amino acid molecules of a given set of amino acid molecules as defined above is specifically recognized by an antibody of said given set of antibodies,
for the *in vitro* or *ex vivo* diagnosis of any type of somatic or ovarian cancers,
wherein:
   - cancer cells of each type of somatic or ovarian cancer abnormally expresses at least one amino acid molecule recognized by an antibody of the above sets of antibodies, and
   - at least one of amino acid molecule recognized by an antibody of the above sets of antibodies is abnormally expressed in cancer cells of at least one type of somatic or ovarian cancers.

Another advantageous embodiment of the invention relates to the use of a set of at least 26 antibodies, preferably a set of 57 antibodies, more preferably a set of 88 antibodies, more preferably a set of 103 antibodies, more preferably a set of 121 antibodies, more preferably a set of 150 antibodies, more preferably a set of 163 antibodies, more preferably a set of 186 antibodies, in particular a set of 192 antibodies characterized in that it each antibody of a given mentioned set of antibodies specifically recognizes an amino acid molecule of a set of amino acid molecules as defined above, and each amino acid molecule of a given set of amino acid molecules as defined above is specifically recognized by an antibody of said given set of antibodies,
for the *in vitro* or *ex vivo* diagnosis of any type of somatic or ovarian cancers,
wherein:
- each type of somatic or ovarian cancer cells abnormally expresses at least a set of amino acid molecules recognized by a set of antibodies of the above sets of antibodies, and
- at least one a set of amino acid molecules recognized by a set of antibodies of the above sets of antibodies is abnormally expressed in cancer cells of at least one type of somatic or ovarian cancers.

By antibody it is defined in the invention, all the immunological molecules produced by B-cell: immunoglobulins (Ig). Then, according to the invention, all the soluble and insoluble immunoglobulins, such as IgG, IgM, IgA and IgD are considered. According to the invention IgG antibodies are prefered.
Also, according to the invention, antibodies can be represented by their "immunological" part, i.e. the variable chain. Thus, antibodies can be also considered as fragments such as Fab, F(ab)'₂ or scFv fragments.
By "antibody specifically recognize an amino acid molecule" it is meant in the invention that antibodies are able to form a specific immune complex with a determined protein, but not with another protein.
Also, in the invention "amino acid molecule is specifically recognized by an antibody" means that a protein is recognized by one specific antibody.
Thus, in a set of protein according to the invention, each antibody is able to recognize at least one protein of the individual's sample, and each protein contained in the sample is able to be recognized by one antibody of the set.

According to the invention, "neoplasm" describes an abnormal proliferation of genetically altered cells. Neoplasms can be benign or malignant.
According to the invention, "tumors" means any abnormal swelling, lump or mass.
As commonly used in the art, according to the invention, terms "tumor" and "neoplasm" are synonymous with cancer.

Cancers are classified by the type of cell that resembles the tumor and, therefore, the tissue presumed to be the origin of the tumor. Examples of general categories include:
- Carcinoma: Malignant tumors derived from epithelial cells. This group represents the most common cancers, including the common forms of breast, prostate, lung and colon cancer.
- Sarcoma: Malignant tumors derived from connective tissue, or mesenchymal cells,
- Germ cell tumor: Tumors derived from totipotent cells. In adults most often found in the testicle and ovary. However, the invention does no relate to the testicle cancer,
- Blastic tumor: A tumor (usually malignant) which resembles an immature or embryonic tissue. Many of these tumors are most common in children,
- Lymphoma and leukemia: Malignancies derived from hematopoietic (blood-forming) cells.

According to the invention, "solid tumors" concern tumors derived from organs, and in particular concern lung cancer, including small cell lung cancer and non-small lung cancer, pancreas cancer, bladder cancer, breast cancer, brain cancer, including glioblastomas medulloblastomas and neuroblastomas, cervical cancer, gastric cancer, colon cancer, including colorectal carcinoma, endometrial cancer, esophageal cancer, biliary tract cancer, head and neck cancer, oral cancer, including squamous cell carcinoma, liver cancer, including hepatocarcinoma, ovarian cancer, including those arising from epithelial cells, stromal cells, germ cells and mesenchymal cells, pancreatic cancer, prostate cancer, rectal cancer, sarcomas, including leiomyosarcoma, rhabdomyosarcoma, liposarcoma, fibrosarcoma, synovial sarcoma, neurosarcoma, chondrosarcoma, Ewing sarcoma, malignant fibrous histocytoma, glioma, hepatoma and osteosarcoma, skin cancer, including melanomas, Kaposi's sarcoma, basocellular cancer and squamous cell cancer, thyroid cancer, including thyroid adenocarcinoma and medullar carcinoma, kidney cancer, including adenocarcinoma and Wilms tumthe, intraepithelial neoplasms, including Bowen's disease and Paget's disease, and placental cancer or choriocarcinoma.
According to the invention, "hematological neoplasms" concern all the neoplasms derived from blood cells or progeny of blood cells, and in particular concern: acute lymphocytic leukemias, acute myelogenous leukemias, multiple myelomas, AIDS-associated leukemias, and adult T-cell leukemia lymphomas
The invention concerns also lymphomas such as Hodgkin's disease, lymphocytic lymphoma and mantle cell lymphoma.

In another aspect, the invention relates to a microarray consisting of at least 32 oligonucleotide probes represented by the oligonucleotide sequences SEQ ID NO 415 to 446, each of said at least 32 oligonucleotide probes specifically hybridizing with one nucleic acid molecule of at least a set of nucleic acid molecules as defined above, the correspondence between oligonucleotide probes and their corresponding nucleic acid sequence being represented in Table 3a.

The invention is illustrated by a microarray comprising at least 32 oligonucleotide probes represented by the oligonucleotide sequences SEQ ID NO 415 to 446, each of said at least 32 oligonucleotide probes specifically hybridizing with one nucleic acid molecule of at least a set of nucleic acid molecules as defined above, preferably with one nucleic acid molecule of at least 26 nucleic acid molecules represented by the nucleic acid sequences SEQ ID NO 2q-1, q varying from 1 to 26, the correspondence between oligonucleotide probes and their corresponding nucleic acid sequence being represented in Table 3a.

The following table 3a indicates the correspondence between nucleic acid molecules and the corresponding polynucleotide molecules.
Gene # indicates the SEQ ID corresponding to nucleic acid sequence;(Gene # priority) indicates the SEQ ID corresponding to nucleic acid sequence of the priority document, Prob1#, Prob2# and Prob#3 indicates the corresponding to nucleic acid sequence of the probe.

| **Gene # (Gene # priority**)) | **Prob1#** (priority) | **Prob2#** (priority) | **Prob3#** (priority) |
|---|---|---|---|
| **1** | 415 | 416 | |
| **3** | 417 | 418 | |
| **5** | 419 | | |
| **7** | 420 | | |
| **9** (7) | 421 (758) | | |
| **11** (15) | 422 | | |
| **13** (67) | 423 (787) | | |
| **15** (127) | 424(812) | | |
| **17** (173) | 425 (836) | 426 (837) | |
| **19** (175) | 427 (838) | | |
| **21** (253) | 428 | | |
| **23** (267) | 429 (886) | | |
| **25** (271) | 430 (888) | | |
| **27** (291) | 431 (898) | | |
| **29** (313) | 432 (908) | | |
| **31** (411) | 433 | | |
| **33** (453) | 434 (964) | | |
| **35** (459) | 435 (967) | | |
| **37** (469) | 436 (972) | 437 (971) | |
| **39** (497) | 438 | | |
| **41** (517) | 439 | 440 | |
| **43** (561) | 441 | 442 | |
| **45** (633) | 443 | | |
| **47** (637) | 444 (1055) | | |
| **49** | 445 | | |
| **51** | 446 | | |

Thus, for instance, SEQ ID NO 1 gene is detected by the polynucleotide probes SEQ ID NO 415 and 416, or SEQ ID NO 9 (corresponding to SEQ ID NO 7 gene in the priority document) is detected by probe SEQ ID NO 421 (corresponding to SEQ ID NO 758 gene in the priority document).

In one advantageous embodiment, the invention relates to a microarray as defined above, comprising at least 70 oligonucleotide probes represented by the oligonucleotide sequences SEQ ID NO 415 to 484, each of said at least 70 oligonucleotide probes specifically hybridizing with one nucleic acid molecule of at least 59 nucleic acid molecules represented by the nucleic acid sequences SEQ ID NO 2q-1, q varying from 1 to 57 and SEQ ID NO 385 to SEQ ID NO 386,
optionally, comprising at least 110 oligonucleotide probes represented by the oligonucleotide sequences SEQ ID NO 415 to 524, each of said at least 110 oligonucleotide probes specifically hybridizing with one nucleic acid molecule of at least 93 nucleic acid molecules represented by the nucleic acid sequences SEQ ID NO 2q-1, q varying from 1 to 88 and SEQ ID NO 385 to SEQ ID NO 389,
optionally, comprising at least 130 oligonucleotide probes represented by the oligonucleotide sequences SEQ ID NO 415 to 544, each of said at least 130 oligonucleotide probes specifically hybridizing with one nucleic acid molecule of at least 108 nucleic acid molecules represented by the nucleic acid sequences SEQ ID NO 2q-1, q varying from 1 to 103 and SEQ ID NO 385 to SEQ ID NO 389,
optionally, comprising at least 154 oligonucleotide probes represented by the oligonucleotide sequences SEQ ID NO 415 to 568, each of said at least 154 oligonucleotide probes specifically hybridizing with one nucleic acid molecule of at least 128 nucleic acid molecules represented by the nucleic acid sequences SEQ ID NO 2q-1, q varying from 1 to 121 and SEQ ID NO 385 to SEQ ID NO 391,
optionally, comprising at least 197 oligonucleotide probes represented by the oligonucleotide sequences SEQ ID NO 415 to 611, each of said at least 197 oligonucleotide probes specifically hybridizing with one nucleic acid molecule of at least 160 nucleic acid molecules represented by the nucleic acid sequences SEQ ID NO 2q-1, q varying from 1 to 144 and SEQ ID NO 385 to SEQ ID NO 400,
optionally, comprising at least 204 oligonucleotide probes represented by the oligonucleotide sequences SEQ ID NO 415 to 618, each of said at least 204 oligonucleotide probes specifically hybridizing with one nucleic acid molecule of at least 166 nucleic acid molecules represented by the nucleic acid sequences SEQ ID NO 2q-1, q varying from 1 to 150 and SEQ ID NO 385 to SEQ ID NO 400,
optionally, comprising at least 220 oligonucleotide probes represented by the oligonucleotide sequences SEQ ID NO 415 to 634, each of said at least 220 oligonucleotide probes specifically hybridizing with one nucleic acid molecule of at least 179 nucleic acid molecules represented by the nucleic acid sequences SEQ ID NO 2q-1, q varying from 1 to 163 and SEQ ID NO 385 to SEQ ID NO 400,
optionally, comprising at least 261 oligonucleotide probes represented by the oligonucleotide sequences SEQ ID NO 415 to 675, each of said at least 261 oligonucleotide probes specifically hybridizing with one nucleic acid molecule of at least 213 nucleic acid molecules represented by the nucleic acid sequences SEQ ID NO 2q-1, q varying from 1 to 186 and SEQ ID NO 385 to SEQ ID NO 411,
optionally, comprising at least 270 oligonucleotide probes represented by the oligonucleotide sequences SEQ ID NO 415 to 684, each of said at least 270 oligonucleotide probes specifically hybridizing with one nucleic acid molecule of the 222 nucleic acid molecules of the collection of 222 nucleic acid molecules represented by the nucleic acid sequences SEQ ID NO 2q-1, q varying from 1 to 192 and SEQ ID NO 385 to 414,
the correspondence between oligonucleotide probes and their corresponding gene being represented in Table 3b,
said microarray possibly comprising positive and negative oligonucleotide probes specifically hybridizing with positive and negative control nucleic acid molecules.

By "positive and negative oligonucleotide probes specifically hybridizing with positive and negative control nucleic acid molecules" it is meant in the invention "positive oligonucleotide probes specifically hybridizing with positive control nucleic acid molecules and negative oligonucleotide probes specifically hybridizing with negative control nucleic acid molecules". The "negative probes" of the invention designate probes that detect the expression of genes that are expressed ubiquituously, i.e. in all types of healthy or malignant cells. "Negative nucleic acid molecules" thus define genes that are expressed ubiquituously.
The "positive probes" of the invention designate probes that detect the expression of genes that are expressed specifically in one or more tissues but not expressed in testis or placenta, said tissues being constituded by healthy or malignant cells.
"Positive nucleic acid molecules" thus define genes that are expressed specifically in one or more tissues but not expressed in testis or placenta.

The following Table 3b indicates the correspondence between nucleic acid molecules and the corresponding polynucleotide molecules.

Gene # indicates the SEQ ID corresponding to nucleic acid sequence;(Gene # priority) indicates the SEQ ID corresponding to nucleic acid sequence of the priority document, Prob1#, Prob2# and Prob#3 indicate the corresponding nucleic acid sequences of the probes.

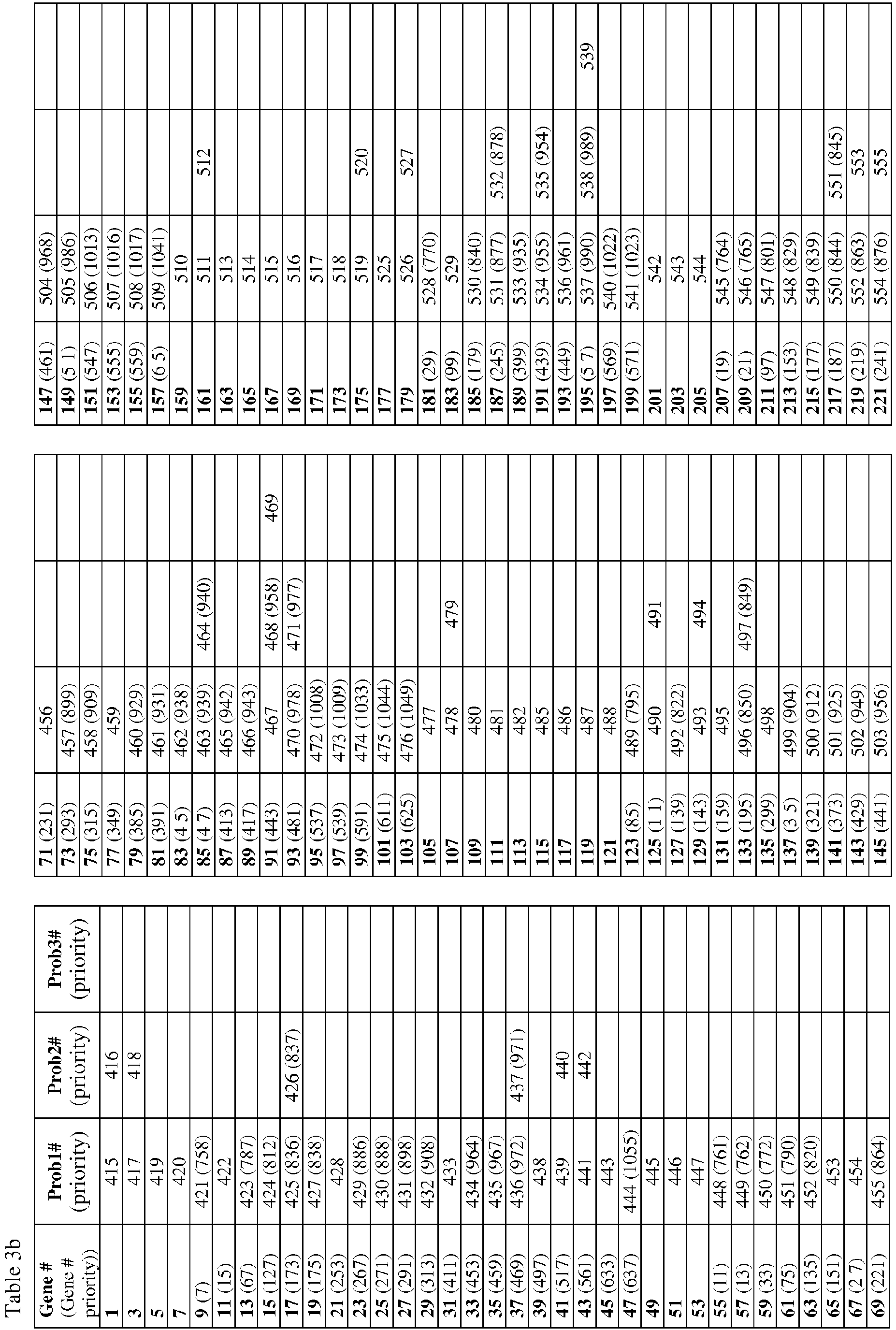

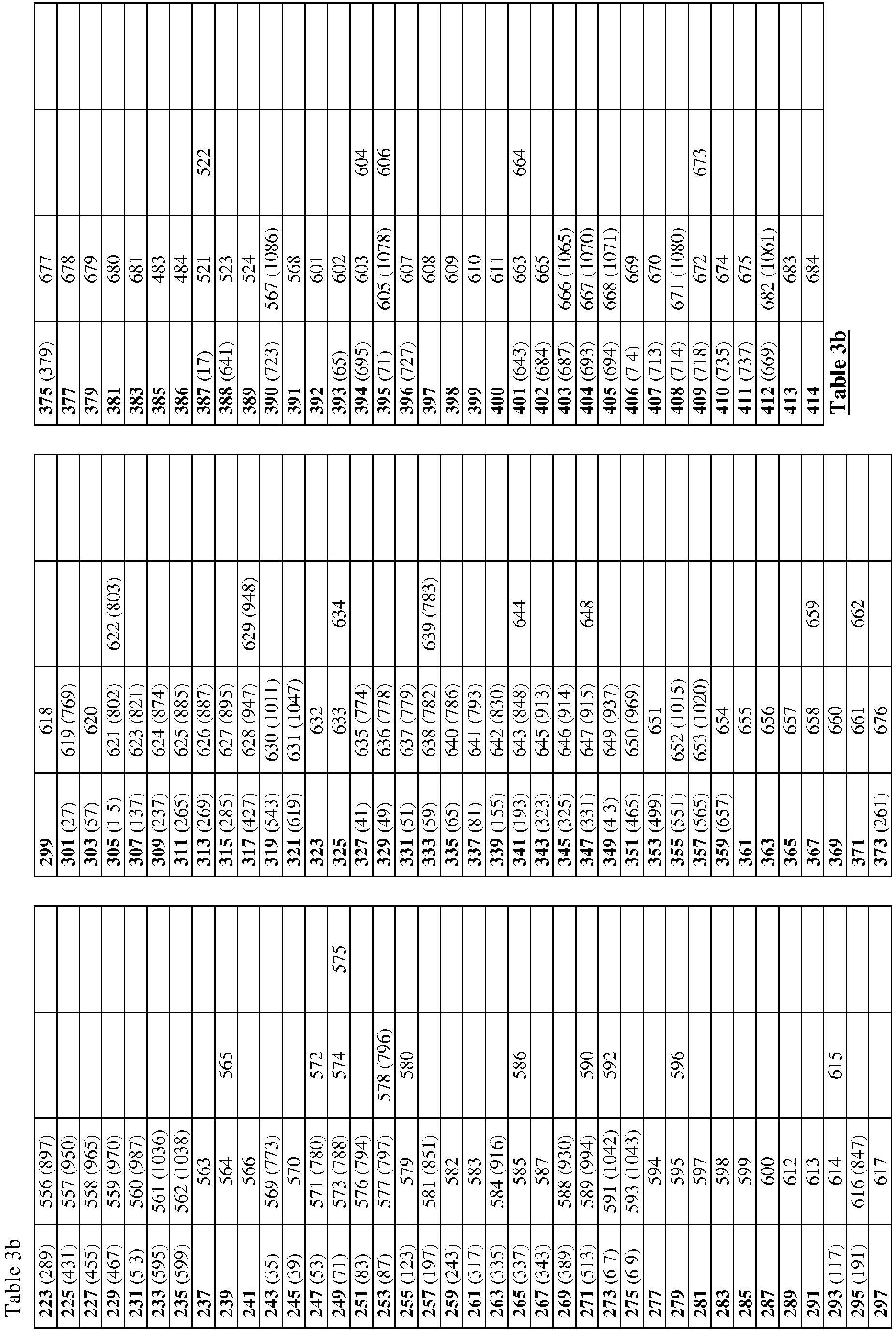

Another advantageous embodiment of the invention relates to a microarray as defined above, comprising the oligonucleotide probes represented by the oligonucleotide sequences SEQ ID NO 415 to SEQ ID NO 684, optionally comprising the oligonucleotide probes represented by the oligonucleotide sequences SEQ ID NO 415 to SEQ ID NO 1617, optionally comprising or consisting in the oligonucleotide probes represented by the oligonucleotide sequences SEQ ID NO 415 to SEQ ID NO 2989.

In the invention the microarray comprising oligonucleotide probes represented by the oligonucleotide sequences SEQ ID NO 415 to SEQ ID NO 684 is able to detect the variation of expression of the 222 CT genes represented by the nucleic acid sequences SEQ ID NO 2q-1, q varying from 1 to 192, and SEQ ID NO 385 to 414.

The microarray comprising oligonucleotide probes represented by the oligonucleotide sequences SEQ ID NO 415 to SEQ ID NO 1617 is able to detect the variation of expression of the 222 CT genes represented by the nucleic acid sequences SEQ ID NO 2q-1, q varying from 1 to 192, and SEQ ID NO 385 to 414, and the variation of expression of genes that are expressed in a tissue specific manner, but are not expressed in testis or placenta.

The microarray comprising oligonucleotide probes represented by the oligonucleotide sequences SEQ ID NO 415 to SEQ ID NO 2989 is able to detect the variation of expression of the 222 CT genes represented by the nucleic acid sequences SEQ ID NO 2q-1, q varying from 1 to 192, and SEQ ID NO 385 to 414, and the variation of expression of genes that are expressed in a tissue specific manner, but are not expressed in testis or placenta, the expression of ubiquitous genes, and poorly specific testis and placenta expressed genes.
For instance, poorly specific testis and placenta expressed genes are defined hereafter by TEPEc and d genes (see Example 3).

The invention is also illustrated by a microarray comprising at least 26 amino acid molecules, or a fragments thereof, represented by the amino acid sequences SEQ ID NO 2q, q varying from 1 to 26, chosen among the collection of 192 amino acid molecules, or fragments thereof, represented by the amino acid sequences SEQ ID NO 2q, q varying from 1 to 192, each of said at least 26 amino acid molecules, or fragments thereof, specifically hybridizing with at least one antibody, said antibody being able to specifically interact with a determined amino acid molecule, or fragment thereof, and not being able to interact with another amino acid molecule.

The invention is also illustrated by a microarray comprising at least 26 antibodies, chosen among a group of 192 antibodies, said at least 26 antibodies specifically interacting with at least 26 amino acid molecules, or a fragments thereof, represented by the amino acid sequences SEQ ID NO 2q, q varying from 1 to 26, chosen among the collection of 192 amino acid molecules, or fragments thereof, represented by the amino acid sequences SEQ ID NO 2q, q varying from 1 to 192.

The invention is illustrated by a method for the *in vitro* and/or *ex vivo* cancer diagnosis in a subject, by determining the presence or the variation of the amount of at least one nucleic acid molecule comprising or constituted by a nucleotide acid sequence consisting in SEQ ID NO 2q-1, q varying from 1 to 192 and SEQ ID NO 385 to SEQ ID NO 414, or a fragment thereof, among nucleic acids from a biological sample from the subject,
said presence or variation of the amount of said nucleic acid molecule being assessed with respect to the absence or the given amount of said nucleic acid molecule from a sample isolated from a healthy subject, comprising:
- contacting nucleic acids from the biological sample with an agent, said agent being at least one nucleic acid molecule, or a complementary nucleic acid molecule of at least one nucleic acid molecule, comprising or constituted by the group consisting in SEQ ID NO 2q-1, q varying from 1 to 192 and SEQ ID NO 385 to SEQ ID NO 414 or a fragment thereof, and the said agent being able to selectively hybridize with at least one nucleic acid molecule comprising or constituted by the group consisting in SEQ ID NO 2q-1, q varying from 1 to 192 and SEQ ID NO 385 to SEQ ID NO 414 liable to be present among nucleic acids from the biological sample, to form a nucleic acid complex,
- determining the presence or the variation of amount of said nucleic acid complex indicating the fact that the subject is afflicted by cancer.

In another aspect, the invention relates to a method for the *in vitro* and/or *ex vivo* somatic or ovarian cancer diagnosis in a subject, by determining the presence or the variation of the amount of at least one nucleic acid molecule of a group of at least 26 nucleic acid molecules chosen among the collection of 222 nucleic acid molecules represented by the nucleic acid sequences SEQ ID NO 2q-1, q varying from 1 to 192 and SEQ ID NO 385 to 414, or a fragment thereof
said 26 nucleic acid molecules being represented by the nucleic acid sequences SEQ ID NO 2q-1, q varying from 1 to 26, among nucleic acids from a biological sample from the subject,
said presence or variation of the amount of said nucleic acid molecule being assessed with respect to the absence or the given amount of said nucleic acid molecule from a sample isolated from a healthy subject, comprising:
- contacting nucleic acids from the biological sample with an agent to allow the formation of at least one nucleic acid complex between said agent and said at least one nucleic acid molecule from the biological sample of the subject,
   - said agent comprising at least:
      ∘ one nucleic acid molecule,
      ∘ a complementary molecule of said nucleic acid sequence,
      ∘ oa fragment of said nucleic acid molecule, or
      ∘ a fragment of said complementary molecule,
      of each of at least 26 nucleic acid molecules chosen among the 222 nucleic acid molecules represented by the nucleic acid sequences SEQ ID NO 2q-1, q varying from 1 to 192 and SEQ ID NO 385 to 414, said at least 26 nucleic acid molecules being represented by the nucleic acid sequences SEQ ID NO 2q-1, q varying from 1 to 26, and
   - the nucleic acid sequences, the complementary sequences of said nucleic acid sequences, or the fragments thereof, contained in said agent being able to selectively hybridize with said at least 26 nucleic acid molecules,
- determining the presence or the variation of amount of at least one nucleic acid complex indicating the fact that the subject is afflicted with cancer.

In one preferred embodiment, the invention relates to a method for the *in vitro* and/or *ex vivo* somatic or ovarian cancer diagnosis as defined above, by determining the presence or the variation of amount of at least one nucleic acid molecule of a group of at least 59 nucleic acid molecules chosen among the collection of 222 nucleic acid molecules represented by the nucleic acid sequences SEQ ID NO 2q-1, q varying from 1 to 192 and SEQ ID NO 385 to 414, or a fragment thereof, said 59 nucleic acid molecules being represented by the nucleic acid sequences SEQ ID NO 2q-1, q varying from 1 to 57 and SEQ ID NO 385 to SEQ ID NO 386.

In one preferred embodiment, the invention relates to a method for the *in vitro* and/or *ex vivo* somatic or ovarian cancer diagnosis as defined above, by determining the presence or the variation of amount of at least one nucleic acid molecule of a group of at least 93 nucleic acid molecules chosen among the collection of 222 nucleic acid molecules represented by the nucleic acid sequences SEQ ID NO 2q-1, q varying from 1 to 192 and SEQ ID NO 385 to 414, or a fragment thereof, said at least 93 nucleic acid molecules being represented by the nucleic acid sequences SEQ ID NO 2q-1, q varying from 1 to 88 and SEQ ID NO 385 to SEQ ID NO 389.

In one preferred embodiment, the invention relates to a method for the *in vitro* and/or *ex vivo* somatic or ovarian cancer diagnosis as defined above, by determining the presence or the variation of amount of at least one nucleic acid molecule of a group of at least 108 nucleic acid molecules chosen among the collection of 222 nucleic acid molecules represented by the nucleic acid sequences SEQ ID NO 2q-1, q varying from 1 to 192 and SEQ ID NO 385 to 414, or a fragment thereof, said at least 108 nucleic acid molecules being represented by the nucleic acid sequences SEQ ID NO 2q-1, q varying from 1 to 103 and SEQ ID NO 385 to SEQ ID NO 389.

In one preferred embodiment, the invention relates to a method for the *in vitro* and/or *ex vivo* somatic or ovarian cancer diagnosis as defined above, by determining the presence or the variation of amount of at least one nucleic acid molecule of a group of at least 128 nucleic acid molecules chosen among the collection of 222 nucleic acid molecules represented by the nucleic acid sequences SEQ ID NO 2q-1, q varying from 1 to 192 and SEQ ID NO 385 to 414, or a fragment thereof, said at least 128 nucleic acid molecules being represented by the nucleic acid sequences SEQ ID NO 2q-1, q varying from 1 to 121 and SEQ ID NO 385 to SEQ ID NO 391.

In one preferred embodiment, the invention relates to a method for the *in vitro* and/or *ex vivo* somatic or ovarian cancer diagnosis as defined above, by determining the presence or the variation of amount of at least one nucleic acid molecule of a group of at least 160 nucleic acid molecules chosen among the collection of 222 nucleic acid molecules represented by the nucleic acid sequences SEQ ID NO 2q-1, q varying from 1 to 192 and SEQ ID NO 385 to 414, or a fragment thereof, said at least 160 nucleic acid molecules being represented by the nucleic acid sequences SEQ ID NO 2q-1, q varying from 1 to 144 and SEQ ID NO 385 to SEQ ID NO 400.

In one preferred embodiment, the invention relates to a method for the *in vitro* and/or *ex vivo* somatic or ovarian cancer diagnosis as defined above, by determining the presence or the variation of amount of at least one nucleic acid molecule of a group of at least 166 nucleic acid molecules chosen among the collection of 222 nucleic acid molecules represented by the nucleic acid sequences SEQ ID NO 2q-1, q varying from 1 to 192 and SEQ ID NO 385 to 414, or a fragment thereof, said at least 166 nucleic acid molecules being represented by the nucleic acid sequences SEQ ID NO 2q-1, q varying from 1 to 150 and SEQ ID NO 385 to SEQ ID NO 400.

In one preferred embodiment, the invention relates to a method for the *in vitro* and/or *ex vivo* somatic or ovarian cancer diagnosis as defined above, by determining the presence or the variation of amount of at least one nucleic acid molecule of a group of at least 179 nucleic acid molecules chosen among the collection of 222 nucleic acid molecules represented by the nucleic acid sequences SEQ ID NO 2q-1, q varying from 1 to 192 and SEQ ID NO 385 to 414, or a fragment thereof, said at least 179 nucleic acid molecules being represented by the nucleic acid sequences SEQ ID NO 2q-1, q varying from 1 to 163 and SEQ ID NO 385 to SEQ ID NO 400.

In one preferred embodiment, the invention relates to a method for the *in vitro* and/or *ex vivo* somatic or ovarian cancer diagnosis as defined above, by determining the presence or the variation of amount of at least one nucleic acid molecule of a group of at least 213 nucleic acid molecules chosen among the collection of 222 nucleic acid molecules represented by the nucleic acid sequences SEQ ID NO 2q-1, q varying from 1 to 192 and SEQ ID NO 385 to 414, or a fragment thereof, said at least 213 nucleic acid molecules being represented by the nucleic acid sequences SEQ ID NO 2q-1, q varying from 1 to 186 and SEQ ID NO 385 to SEQ ID NO 411.

In one preferred embodiment, the invention relates to a method for the *in vitro* and/or *ex vivo* somatic or ovarian cancer diagnosis as defined above, by determining the presence or the variation of amount of at least one nucleic acid molecule of the collection of 222 nucleic acid molecules represented by the nucleic acid sequences SEQ ID NO 2q-1, q varying from 1 to 192 and SEQ ID NO 385 to 414, or a fragment thereof.

According to the invention, the "determination of the presence" of at least one nucleic acid molecule indicates that if a nucleic acid molecule can be detected in a biological sample, said nucleic acid molecule is considered as present in the biological sample. On the contrary, if said nucleic acid molecule can not be detected by the method of the invention, the nucleic acid molecule is considered as absent from the biological sample.
According to the invention, the "determination of variation of amount" of at least one nucleic acid molecule means that the quantity of said nucleic acid molecule is measured. The amount of nucleic acid molecule is measured using classical protocol of quantification, wherein the amount of nucleic acid molecule is compared with at least two control samples. These control samples are represented by at least a negative sample and a positive control sample. The value associated to the measure of the quantity of nucleic acid molecule is null in the control negative sample, and value associated to the measure of the quantity of nucleic acid molecule is positive in the control positive sample.
The negative sample corresponds to a biological sample of a healthy individual, or patient, wherein said nucleic acid molecule is either absent or present at a known level, said known level being defined as the standard level.
So, if the nucleic acid molecule is absent of the biologic sample, the value of the quantification is null. On the other hand, if the nucleic acid molecule is present, the value of the quantification is superior to zero.
The presence or amount of nucleic acid molecule may be determined by any routine protocols commonly used in the art. In particular, the nucleic acid molecule is detected by commonly used techniques based on the nucleic acid hybridization, such as Southern blot and Northern blot.
The extraction of the nucleic acid molecules of the samples is managed by a routine protocol used in the art. Advantageously, nucleic acid molecules extracted from the biological sample are RNA.
According to the invention, said agent comprising and/or being constituted by at least one polynucleotide molecule preferably corresponding to a fragment of said nucleic acid molecule, said polynucleotide molecule being such that it is able to specifically hybridize with said nucleic acid molecule, according to the base complementarity. Preferably in the invention, said polynucleotide molecule, also called hereafter nucleic acid, is a DNA molecule.

Then, the method of the invention consists in contacting nucleic acid molecules extracted from the biological sample of a subject, with an agent. Contact between nucleic acid molecule, when present, and agent allows to form a nucleic acid complex.

Preferably, before contacting the agent with the nucleic acid molecules, said nucleic acid molecules being labeled with any known labeling molecules (radioisotopes, enzymes, fluorescent molecules...). The hybridization is made according a standard procedure, by modulating if necessary saline concentration and temperature. The protocol used for hybridization is well known by a skilled person.
Alternatively, said nucleic acid complex can be detected using known labeling molecules (e.g. fluorescent molecules) that specifically detect the formation of a double strand nucleic acid molecule, as the result of the hybridization.
The presence or amount of the formed nucleic acid complex is detected, by the detection of hybridized nucleic acid molecules with a specific detection method fitting to the used labeling molecule.
The presence or amount of nucleic acid molecule, compared with at least the absence or the amount of said nucleic acid molecule, allows defining if the individual from whom nucleic acid molecules derive from is afflicted by cancer.

In an advantageous embodiment, the invention relates to a method described above, wherein the above-defined agent is preferably immobilized on a micro-array, said micro-array comprising at least one nucleic acid comprising or consisting by a nucleic acid sequence of the group comprising SEQ ID NO 421, SEQ ID NO 423, SEQ ID NO 424, SEQ ID NO 425, SEQ ID NO 426, SEQ ID NO , SEQ ID NO 427, SEQ ID NO 429, SEQ ID NO 430, SEQ ID NO 431, SEQ ID NO 432, SEQ ID NO 434, SEQ ID NO 435, SEQ ID NO 436, SEQ ID NO 437, SEQ ID NO , SEQ ID NO 444, SEQ ID NO 448, SEQ ID NO 449, SEQ ID NO 450, SEQ ID NO 451, SEQ ID NO 452, SEQ ID NO 455, SEQ ID NO 457, SEQ ID NO 458, SEQ ID NO 460, SEQ ID NO 461, SEQ ID NO 462, SEQ ID NO 463, SEQ ID NO 464, SEQ ID NO , SEQ ID NO 465, SEQ ID NO 466, SEQ ID NO 470, SEQ ID NO 471, SEQ ID NO , SEQ ID NO 472, SEQ ID NO 473, SEQ ID NO 474, SEQ ID NO 475, SEQ ID NO 476, SEQ ID NO 489, SEQ ID NO 492, SEQ ID NO 496, SEQ ID NO 497, SEQ ID NO , SEQ ID NO 499, SEQ ID NO 500, SEQ ID NO 501, SEQ ID NO 502, SEQ ID NO 503, SEQ ID NO 504, SEQ ID NO 505, SEQ ID NO 506, SEQ ID NO 507, SEQ ID NO 508, SEQ ID NO 509, SEQ ID NO 528, SEQ ID NO 530, SEQ ID NO 531, SEQ ID NO 532, SEQ ID NO , SEQ ID NO 533, SEQ ID NO 534, SEQ ID NO 535, SEQ ID NO , SEQ ID NO 536, SEQ ID NO 537, SEQ ID NO 538, SEQ ID NO , SEQ ID NO 540, SEQ ID NO 541, SEQ ID NO 545, SEQ ID NO 546, SEQ ID NO 547, SEQ ID NO 548, SEQ ID NO 549, SEQ ID NO 550, SEQ ID NO 551, SEQ ID NO , SEQ ID NO 552, SEQ ID NO 554, SEQ ID NO 556, SEQ ID NO 557, SEQ ID NO 558, SEQ ID NO 559, SEQ ID NO 560, SEQ ID NO 561, SEQ ID NO 562, SEQ ID NO 569, SEQ ID NO 571, SEQ ID NO 573, SEQ ID NO 576, SEQ ID NO 577, SEQ ID NO 578, SEQ ID NO , SEQ ID NO 581, SEQ ID NO 584, SEQ ID NO 588, SEQ ID NO 589, SEQ ID NO 591, SEQ ID NO 593, SEQ ID NO 616, SEQ ID NO 619, SEQ ID NO 621, SEQ ID NO 622, SEQ ID NO , SEQ ID NO 623, SEQ ID NO 624, SEQ ID NO 625, SEQ ID NO 626, SEQ ID NO 627, SEQ ID NO 628, SEQ ID NO 629, SEQ ID NO , SEQ ID NO 630, SEQ ID NO 631, SEQ ID NO 635, SEQ ID NO 636, SEQ ID NO 637, SEQ ID NO 638, SEQ ID NO 639, SEQ ID NO , SEQ ID NO 640, SEQ ID NO 641, SEQ ID NO 642, SEQ ID NO 643, SEQ ID NO 645, SEQ ID NO 646, SEQ ID NO 647, SEQ ID NO 649, SEQ ID NO 650, SEQ ID NO 652, SEQ ID NO 653, SEQ ID NO 567, SEQ ID NO 605, SEQ ID NO 666, SEQ ID NO 667, SEQ ID NO 668, SEQ ID NO 671 and SEQ ID NO 682. The above mentioned sequences are contained in the group disclosed in the priority document (group constituted by SEQ ID NO 755 to SEQ ID NO 1088 in the priority document).

According to the invention, the above-mentioned polynucleotide molecule, or nucleic acid are also called polynucleotidic probes.

In another advantageous embodiment, the invention relates to a method defined above, wherein said agent contains nucleic acid sequences that allow a PCR amplification of a fragment of at least one nucleic acid sequence of said at least 26 nucleic acid molecules liable to be present in an amount different from the given amount of said at least 26 nucleic acid molecules from a sample isolated from an healthy subject, said PCR amplification being preferably reverse transcription-quantitative PCR, or PCR array.

In another advantageous embodiment, the invention relates to a method defined above, wherein said agent contains nucleic acid sequences that allow a PCR amplification of a fragment of at least one nucleic acid sequence of said at least 26 nucleic acid molecules, said PCR amplification being preferably reverse transcription-quantitative PCR, or PCR array.

Another advantageous embodiment of the invention relates to a method described above, that allow a PCR amplification of a fragment of a nucleic acid sequence of said at least 59 nucleic acid molecules liable to be present in an amount different from the given amount of said at least 59 molecules from a sample isolated from an healthy subject.
Another advantageous embodiment of the invention relates to a method described above, that allow a PCR amplification of a fragment of a nucleic acid sequence of said at least 93 nucleic acid molecules liable to be present in an amount different from the given amount of said at least 93 molecules from a sample isolated from an healthy subject.
Another advantageous embodiment of the invention relates to a method described above, that allow a PCR amplification of a fragment of a nucleic acid sequence of said at least 108 nucleic acid molecules liable to be present in an amount different from the given amount of said at least 108 molecules from a sample isolated from an healthy subject.
Another advantageous embodiment of the invention relates to a method described above, that allow a PCR amplification of a fragment of a nucleic acid sequence of said at least 128 nucleic acid molecules liable to be present in an amount different from the given amount of said at least 128 molecules from a sample isolated from an healthy subject.
Another advantageous embodiment of the invention relates to a method described above, that allow a PCR amplification of a fragment of a nucleic acid sequence of said at least 160 nucleic acid molecules liable to be present in an amount different from the given amount of said at least 160 molecules from a sample isolated from an healthy subject.
Another advantageous embodiment of the invention relates to a method described above, that allow a PCR amplification of a fragment of a nucleic acid sequence of said at least 166 nucleic acid molecules liable to be present in an amount different from the given amount of said at least 166 molecules from a sample isolated from an healthy subject.
Another advantageous embodiment of the invention relates to a method described above, that allow a PCR amplification of a fragment of a nucleic acid sequence of said at least 179 nucleic acid molecules liable to be present in an amount different from the given amount of said at least 179 molecules from a sample isolated from an healthy subject.
Another advantageous embodiment of the invention relates to a method described above, that allow a PCR amplification of a fragment of a nucleic acid sequence of said at least 213 nucleic acid molecules liable to be present in an amount different from the given amount of said at least 213 molecules from a sample isolated from an healthy subject.
Another advantageous embodiment of the invention relates to a method described above, that allow a PCR amplification of a fragment of a nucleic acid sequence of said 222 nucleic acid molecules liable to be present in an amount different from the given amount of said 222 molecules from a sample isolated from an healthy subject.

In one other advantageous embodiment, the invention relates to a method as defined above, comprising
- contacting nucleic acids from the biological sample with an agent, said agent being a microarray such as defined above, to allow the formation of at least one nucleic acid complex, between said agent and at least one nucleic acid from a sample of a subject,
- determining the presence or the variation of the amount of at least one nucleic acid complex indicating the fact that the subject is afflicted with cancer.

In another aspect, the invention relates to a method as defined above, by determining the presence or the variation of amount of at least one protein, or a fragment thereof, of a group of at least 26 proteins as defined above, in the biological sample from the subject,
said presence or variation of amount of said protein being assessed with respect to the absence or the given amount of said protein in a sample isolated from a healthy subject, comprising:
- contacting proteins from the biological sample with an agent to allow the formation of at least one immune complex between said agent and said at least one protein from the biological sample of the subject,
   said agent consisting of at least one antibody specifically hybridizing with one protein of each of said at least 26 proteins,
- determining the presence or the variation of amount of said at least one immune complex indicating the fact that the subject is afflicted by cancer, said immune complex being determined preferably by immunohistochemistry, immunocytochemistry, immunofluorescence, western blotting and immunoprecipitation.

In an embodiment, the invention relates to a method as defined above, by determining the presence or the variation of amount of at least one antibody among a group of at least 26 antibodies as defined above that specifically recognizes at least 26 proteins as defined in above, in a biological sample from the subject,
said presence or variation of amount of said antibody being assessed with respect to the absence or the given amount of said antibody in a sample isolated from a healthy subject, comprising:
- contacting the biological sample from the subject with an agent, to allow the formation of at least one immune complex between said agent and at least one antibody from the biological sample of the subject,
   said agent consisting of at least 26 proteins that are able to specifically hybridize with said at least 26 antibodies,
- determining the presence or the variation of amount of said at least one immune complex indicating the fact that the subject is afflicted by cancer, said immune complex being determined preferably by immunohistochemistry, immunocytochemistry, immunofluorescence, western blotting and immunoprecipitation.

The invention is also illustrated by a method for the *in vitro* and/or *ex vivo* cancer diagnosis in a subject, by determining the presence or the variation of amount of at least one protein comprising or constituted by an amino acid sequence consisting in SEQ ID NO 2q, q varying from 1 to 192, or a fragment thereof, among polypeptides from a biological sample from the subject, said presence or variation of amount of said protein being assessed with respect to the absence or the given amount of said protein from a sample isolated from an healthy subject, comprising:
- contacting polypeptides from the biological sample with an agent, said agent being able to recognize at least one protein comprising or constituted by the group consisting in SEQ ID NO 2q, q varying from 1 to 192, or a fragment thereof, liable to be present among polypeptides from the biological sample, to form an immune complex,
- determining the presence or the variation of amount of said immune complex indicating the fact that the subject is afflicted by cancer.

In another preferred embodiment, the invention is illustrated by a method for the *in vitro* and/or *ex vivo* cancer diagnosis, wherein immune complex results from the specific recognition of a protein comprising or constituted by an amino acid sequence consisting in SEQ ID NO 2q, q varying from 1 to 192 , or a fragment thereof, by said agent, said immune complex being liable to be determined for instance by immunohistochemistry, immunocytochemistry, immunofluorescence, western blotting and immunoprecipitation.

The invention is also illustrated, in an advantageous embodiment, by a method for the *in vitro* and/or *ex vivo* cancer diagnosis in a subject, by determining the presence or the variation of amount of at least one protein, or a fragment thereof, of a group of at least 26 proteins chosen among 192 proteins comprising or constituted by an amino acid sequence consisting in SEQ ID NO 2q, q varying from 1 to 192,
said at least 26 proteins being constituted by the amino acid sequences in SEQ ID NO 2q, q varying from 1 to 26,
each protein of said at least 26 proteins being specifically recognized by at least one specific antibody, and said specific antibody being able to specifically recognize one protein of said at least 26 proteins,
among polypeptides from a biological sample from the subject, said presence or variation of amount of said protein being assessed with respect to the absence or the given amount of said protein from a sample isolated from an healthy subject, comprising:
- contacting polypeptides from the biological sample with an agent to allow the formation of at least one immune complex between said agent and at least one protein from a sample of a subject,
   said agent comprising at least one antibodies specifically hybridizing with one protein of each of said at least 26 proteins, and each protein of said at least 26 proteins being specifically recognized by at least one antibody,
   said at least 26 proteins being liable to be present in an amount different from the given amount of said at least 26 proteins from a sample isolated from an healthy subject
- determining the presence or the variation of amount of at least one immune complex indicating the fact that the subject is afflicted by cancer, said immune complex being liable to be determined preferably by immunohistochemistry, immunocytochemistry, immunofluorescence, western blotting and immunoprecipitation.

The method described above allows the determination of the presence or the variation of amount of at least one protein, or a fragment thereof, of a group of at least 57 proteins, or 88 proteins, or 103 proteins, or 121 proteins, or 150 proteins, or 163 proteins, or 186 proteins, or 192 proteins chosen among 192 proteins comprising or constituted by an amino acid sequence consisting in SEQ ID NO 2q, q varying from 1 to 192, or among the group of 192 proteins previously defined.

The invention is illustrated by a method for the *in vitro* and/or *ex vivo* cancer diagnosis in a subject, by determining the presence or the variation of amount of at least one antibody that specifically recognizes a protein comprising or constituted by an amino acid sequence consisting in SEQ ID NO 2q, q varying from 1 to 192, or a fragment thereof, among antibodies that specifically recognize polypeptides from a biological sample from the subject, said presence or variation of amount of said antibody that specifically recognizes protein being assessed with respect to the absence or the given amount of said antibody that specifically recognizes protein from a sample isolated from an healthy subject, comprising:
- contacting antibodies that specifically recognize polypeptides from the biological sample with an agent, said agent being able to recognize at least one antibody that specifically recognize protein comprising or constituted by the group consisting in SEQ ID NO 2q, q varying from 1 to 320, or a fragment thereof, liable to be present among antibodies that specifically recognize polypeptides from the biological sample, to form an immune complex,
- determining the presence or the variation of amount of said immune complex indicating the fact that the subject is afflicted by cancer.

The invention is also illustrated by a method for the *in vitro* and/or *ex vivo* cancer diagnosis in a subject, by determining the presence or the variation of amount of at least one antibody among a group of at least 26 antibodies that specifically recognizes at least 26 proteins or a fragment thereof, chosen among 192 proteins comprising or constituted by an amino acid sequence consisting in SEQ ID NO 2q, q varying from 1 to 192,
said at least 26 proteins being constituted by the amino acid sequences in SEQ ID NO 2q, q varying from 1 to 26,
among antibodies that specifically recognize polypeptides from a biological sample from the subject, said presence or variation of amount of said antibody that specifically recognizes protein being assessed with respect to the absence or the given amount of said antibody that specifically recognizes protein from a sample isolated from an healthy subject, comprising:
   - contacting sample of a subject liable to contain antibodies that specifically recognize polypeptides from the biological sample with an agent to allow the formation of at least one immune complex between said agent and at least one antibody from a sample of a subject said agent comprising said at least 26 proteins that are able to specifically hybridize with said at least 26 antibodies, each protein of said at least 26 protein being able to specifically hybridize with at least one antibody, and each antibody specifically hybridizing with one protein of said at least 26 proteins,
      said at least 26 antibodies being liable to be present in an amount different from the given amount of said at least 26 antibodies from a sample isolated from an healthy subject
   - determining the presence or the variation of amount of at least one immune complex indicating the fact that the subject is afflicted by cancer, said immune complex being liable to be determined preferably by immunohistochemistry, immunocytochemistry, immunofluorescence, western blotting and immunoprecipitation.

The method described above allows the determination of the presence or the variation of amount of at least one antibody among a group of at least 57, or 88, or 103, or 121, or 144, or 150, or 163, or 186 antibodies that specifically recognizes respectively at least 57, or 88, or 103, or 121, or 144, or 150, or 163, or 186 proteins or a fragment thereof, chosen among 192 proteins comprising or constituted by an amino acid sequence consisting in SEQ ID NO 2q, q varying from 1 to 192, or among the group of 192 proteins previously defined.
at least one antibody among a group of at least 26 antibodies that specifically recognizes at least 26 proteins or a fragment thereof, chosen among 192 proteins comprising or constituted by an amino acid sequence consisting in SEQ ID NO 2q, q varying from 1 to 192,

According to the invention, the determination of the presence of at least one antibody indicates that if an antibody can be detected in a biological sample, the antibody is considered as present in the biological sample. On the contrary, if the said antibody can not be detected by the method of the invention, the antibody is considered as absent from the biological sample.
By antibody, it is defined in the invention all the immunological molecules produced by B-cell: immunoglobulins (Ig). Then, according to the invention, all the soluble and insoluble immunoglobulins, such as IgG, IgM, IgA, and IgD, can be detected.
With regard to the determination of the quantification of amount of at least an antibody, it is heard in the invention, that the quantity of said antibody is measured.
The amount of antibody is measured using a classical protocol of quantification, wherein the amount of antibody is compared with at least two control samples. These control samples are represented by at least a negative sample and a positive control sample. The value associated to the measure of the quantity of antibody is null in the control negative sample, and value associated to the measure of the quantity of antibody is positive in the control positive sample.

So, if the antibody is absent of the biologic sample, the value of the quantification is null. On the other hand, if the antibody is present, the value of the quantification is superior to zero. The presence or amount of antibodies may be determined by any routine protocols commonly used in the art.

According to the method of the invention, polypeptides are recognized specifically by at least one antibody liable to be present in a biological sample of a subject. When the antibody is present, the recognition is said specific, which means that the antibody only interact with said polypeptide, or the variants or isoforms of the polypeptides, but does not interact with another polypeptide.

The invention also is illustrated by a method for the *in vitro* and/or *ex vivo* cancer diagnosis in a subject, by determining the presence of an immune response in a biological sample from the subject comprising:
- contacting a biological sample from the subject with an agent, to allow the achievement of an immune response
   said agent comprising at least one antibodies specifically hybridizing with one protein of each of said at least 26 proteins, or a fragment thereof, and each protein of said at least 26 proteins being specifically recognized by at least one antibody,
   said at least 26 proteins being liable to be present in an amount different from the given amount of said at least 26 proteins from a sample isolated from an healthy subject
   said at least 26 proteins being liable to be presented by the MCH molecules of T-cells
- determining the presence of said immune response indicating the fact that the subject is afflicted by cancer.

In one advantageous embodiment, the invention relates to any methods described above, wherein the sample is a body fluid, a body effusion, a cell, a tissue or a tumor.

The invention also relates to a kit for the *in vitro* and/or *ex vivo* cancer diagnosis comprising:
- a microarray such as defined above,
   - optionally material for preparation of nucleic acids of the biological sample from a patient suspected to be afflicted by cancer, in particular the preparation of cDNAs,
   - optionally labelled molecules for labelling said nucleic nucleic acids,
   - optionally a negative control corresponding to nucleic acids from a biological sample from a healthy subject.

The invention also relates to a kit for the *in vitro* and/or *ex vivo* cancer diagnosis comprising:
it for the *in vitro* and/or *ex vivo* cancer diagnosis comprising:
   - ELISA support consisting of at least 26 proteins as defined above, or fragments thereof,
   - optionally labelled antibodies directed against antibodies that recognizes specifically said proteins, said protein being liable to be present in a sample from a patient suspected to be afflicted by cancer,
   - optionally a negative control corresponding to antibodies from a sample from a healthy subject.

The invention is illustrated by a kit for the *in vitro* and/or *ex vivo* cancer diagnosis comprising:
- ELISA support comprising or constituted by at least 26 proteins chosen among 192 proteins comprising or constituted by an amino acid sequence consisting in SEQ ID NO 2q, q varying from 1 to 192, or a fragment thereof, said at least 26 proteins being constituted by the amino acid sequences in SEQ ID NO 2q, q varying from 1 to 26, or fragment thereof,
- possibly labelled antibodies directed against antibody that recognizes specifically said protein, said protein being liable to be present among polypeptides from a sample from a patient suspected to be afflicted by cancer,
- possibly a negative control corresponding to antibodies, or sera, from a sample from an healthy subject.

The invention also relates to a kit for the *in vitro* and/or *ex vivo* cancer diagnosis comprising:
- ELISA support consisting of at least 26 antibodies that specifically recognize at least 26 proteins as defined above, or fragments thereof,
- optionally labelled antibody directed against a protein specifically recognized by said antibody, said antibody being liable to be present in a sample from a patient suspected to be afflicted by cancer,
- optionally a negative control corresponding to polypeptides from a sample from a healthy subject.

The invention is illustrated by a kit for the *in vitro* and/or *ex vivo* cancer diagnosis comprising:
- ELISA support comprising or constituted by at least 26 antibodies that specifically recognize at least 26 proteins chosen among 192 proteins comprising or constituted by an amino acid sequence consisting in SEQ ID NO 2q, q varying from 1 to 192, or a fragment thereof, said at least 26 proteins being constituted by the amino acid sequences in SEQ ID NO 2q, q varying from 1 to 26, or fragment thereof,
- possibly labelled antibody directed against a protein specifically recognized by said antibody, said antibody being liable to be present among antibodies from a sample from a patient suspected to be afflicted by cancer,
- possibly a negative control corresponding to polypeptides from a sample from an healthy subject.

The above mentioned kit contains also
- either at least 57, or 88, or 103, or 121, or 144, or 150, or 163, or 186 antibodies that specifically recognize respectively at least 57, or 88, or 103, or 121, or 144, or 150, or 163, or 186 proteins chosen among 192 proteins comprising or constituted by an amino acid sequence consisting in SEQ ID NO 2q, q varying from 1 to 192, or a fragment thereof, said at least 57, or 88, or 103, or 121, or 144, or 150, or 163, or 186 proteins as defined above, or 222 antibodies specifically recognizing the 222 proteins defined above, or
- at least 57, or 88, or 103, or 121, or 144, or 150, or 163, or 186 proteins chosen among 192 proteins, or a fragment thereof, said at least 26 proteins, or the 222 proteins as defined above.

The invention is illustrated by a pharmaceutical composition comprising at least, as active substance, one of the elements chosen among the group consisting in:
- a nucleic acid molecule described above,
- a protein described above, and
- an antibody described above,
in association with a pharmaceutically acceptable vehicle.

The invention is illustrated by a vaccine composition comprising as active ingredient an antibody, fragments or derivatives thereof described above, in association with a pharmaceutically acceptable vehicle.

The invention is illustrated by a pharmaceutical composition for the treatment of cancers comprising as active ingredient is at least one RNAi molecule, said RNAi molecule being able to hybridize with a nucleic acid molecule described above, in association with a pharmaceutically acceptable vehicle.

RNA interference (RNAi) is a mechanism that inhibits gene expression by causing the degradation of specific RNA molecules or hindering the transcription of specific genes. RNAi plays a role in regulating development and genome maintenance. Small interfering RNA strands (siRNA) are keys to the RNAi process, and have complementary nucleotide sequences to the targeted RNA strand. Specific RNAi pathway proteins are guided by the siRNA to the targeted messenger RNA (mRNA), where they "cleave" the target, breaking it down into smaller portions that can no longer be translated into protein.

In an advantageous embodiment, the invention is illustrated by a pharmaceutical composition described previously, wherein said RNAi specifically hybridize to at least a nucleic acid molecule of the group comprising or constituted by a nucleotide sequence of the group consisting in SEQ ID NO 1 to SEQ ID NO 476, or at least a nucleotide acid molecule coding for protein comprising or constituted by an amino acid sequence belonging to the group consisting in SEQ ID NO 2q, q varying from 1 to 320, said RNAi containing a 17-25 nucleotide sense sequence (siRNA).

In another advantageous embodiment, the invention is illustrated by a pharmaceutical composition previously described, wherein said RNAi specifically binds to at least a nucleic acid molecule of the group comprising or constituted by a nucleotide sequence of the group consisting in SEQ ID NO 1 to SEQ ID NO 476, or at least a nucleotide acid molecule coding for protein comprising or constituted by an amino acid sequence belonging to the group consisting SEQ ID NO 2q, q varying from 1 to 320, said RNAi containing an oligonucleotide composed 17-25 nucleotides sense sequence, a 7-11 nucleotides hairpin loop sequence and an antisense sequence binding complementarily to the sense sequence (shRNA), said shRNA being contained in an expression vector allowing shRNA expression in mammalian cells.

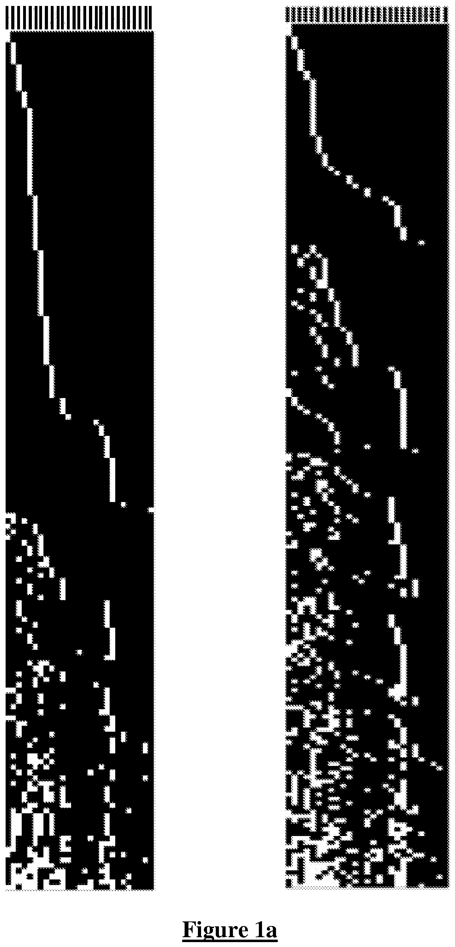

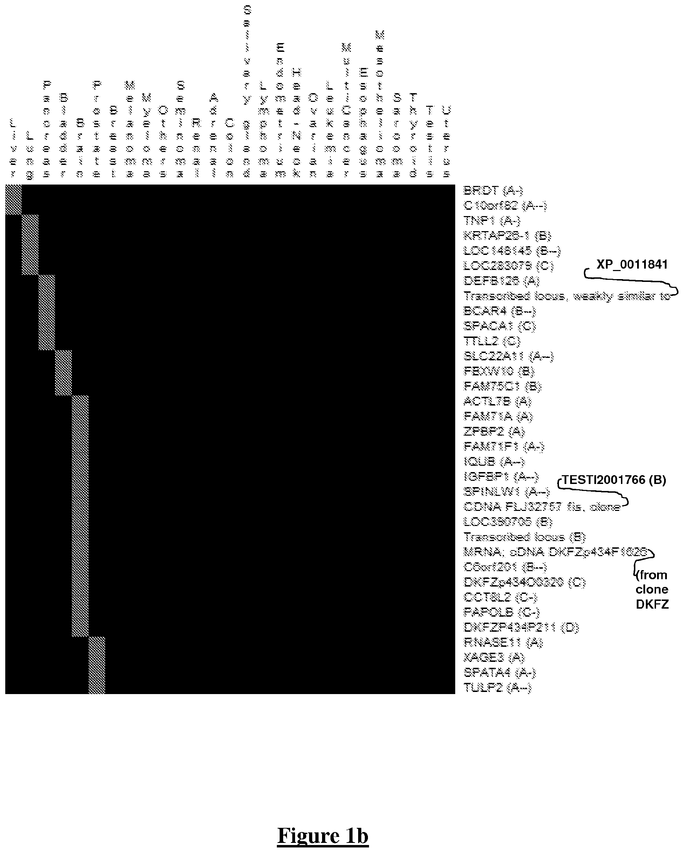

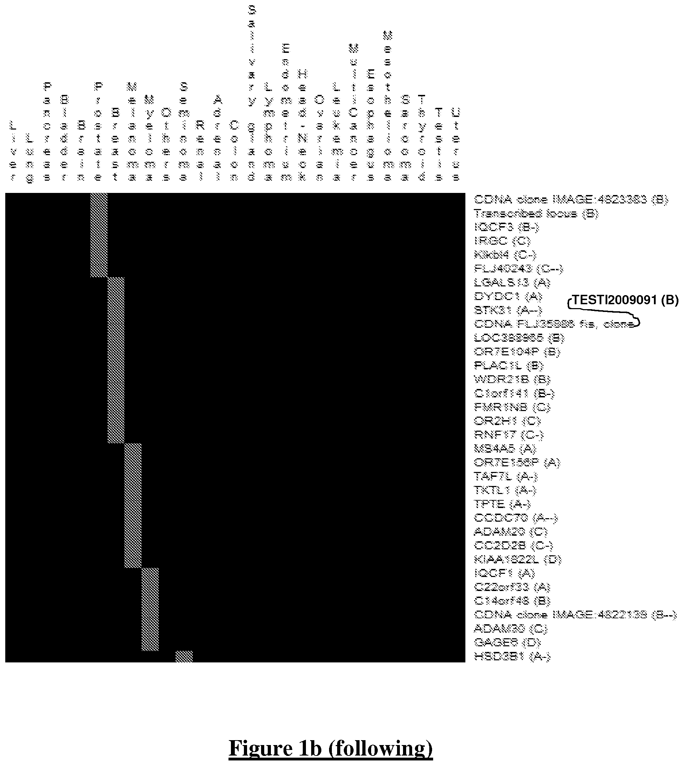

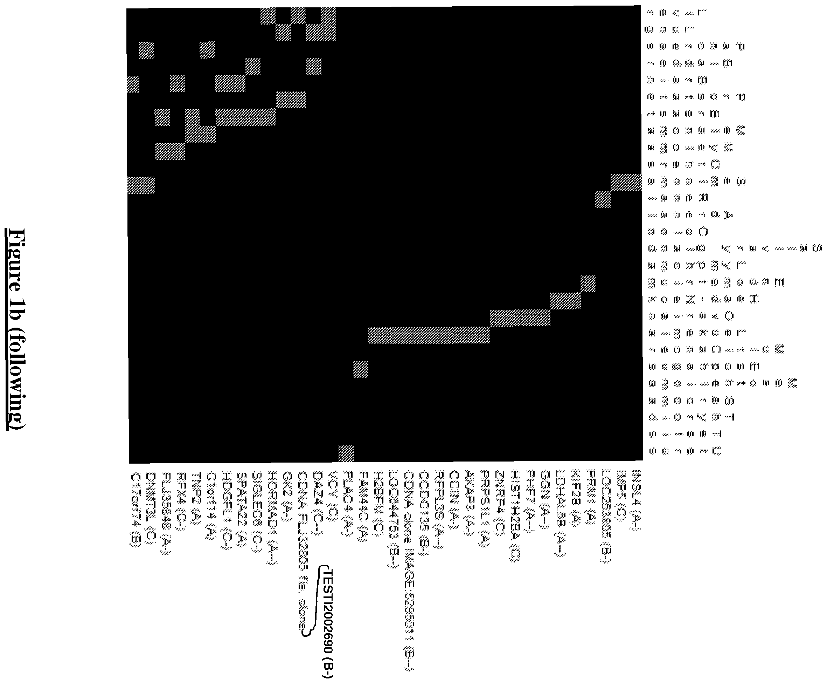

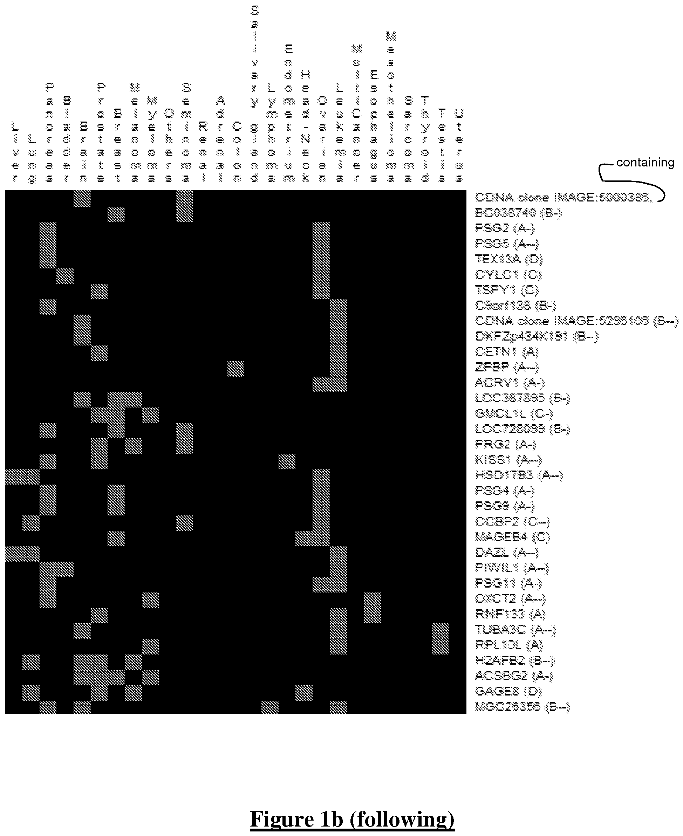

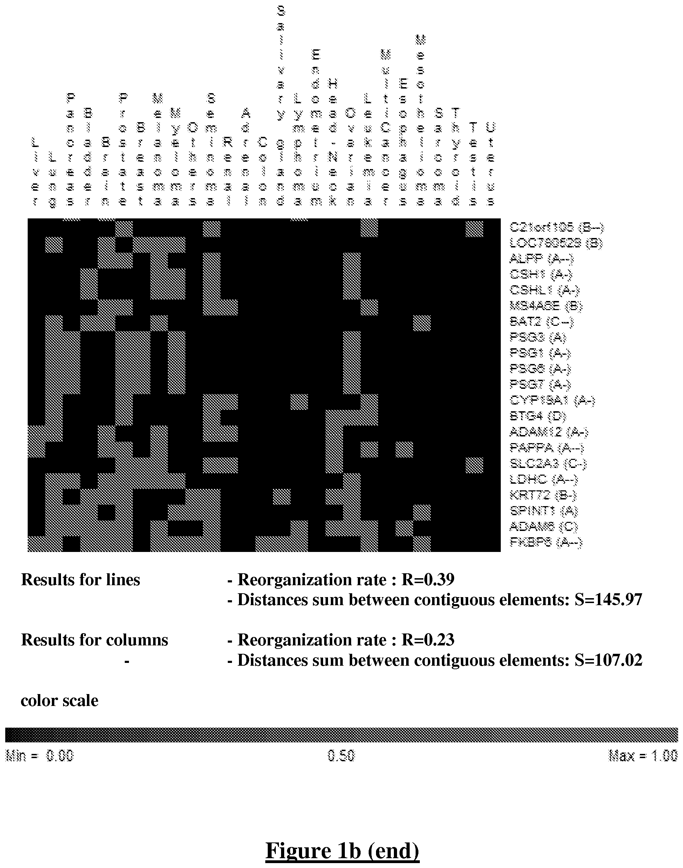

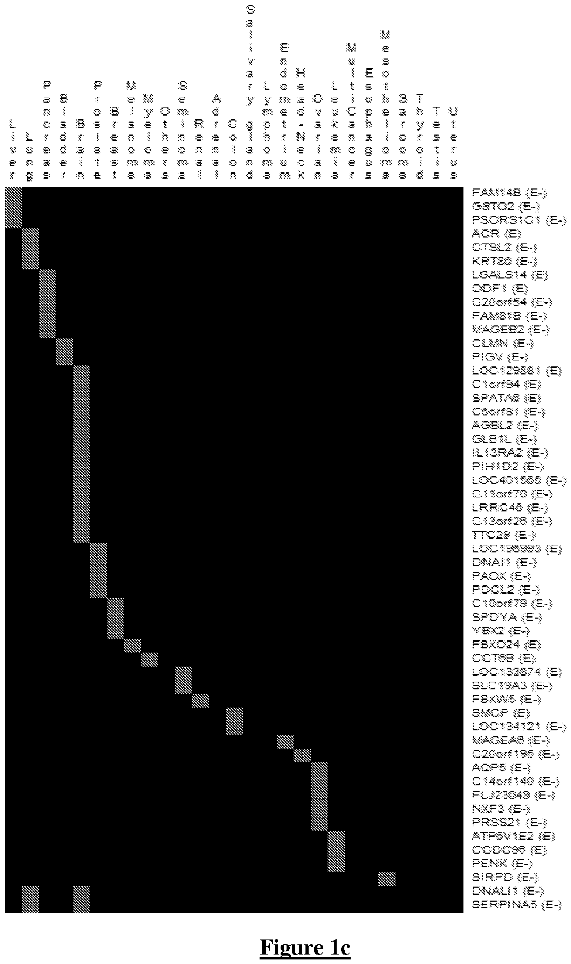

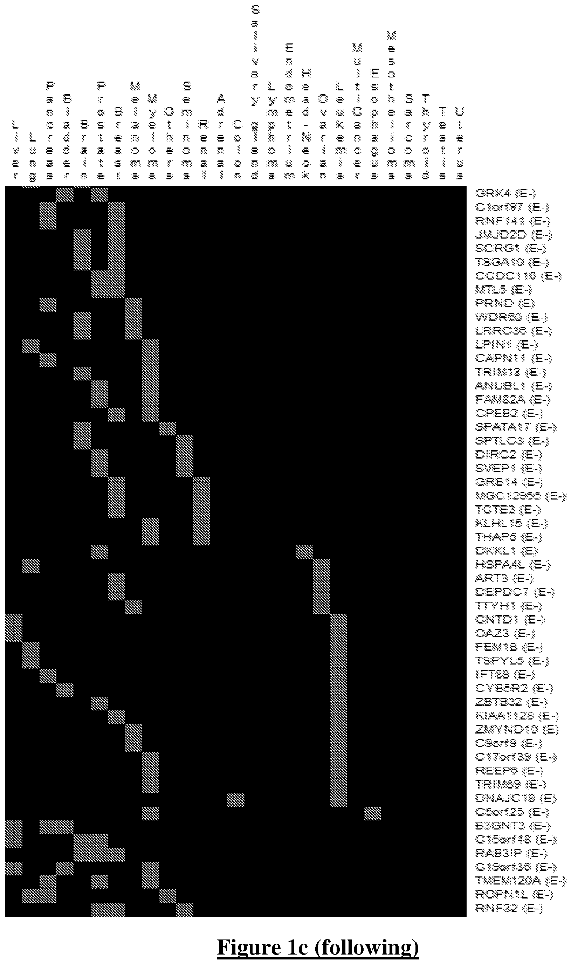

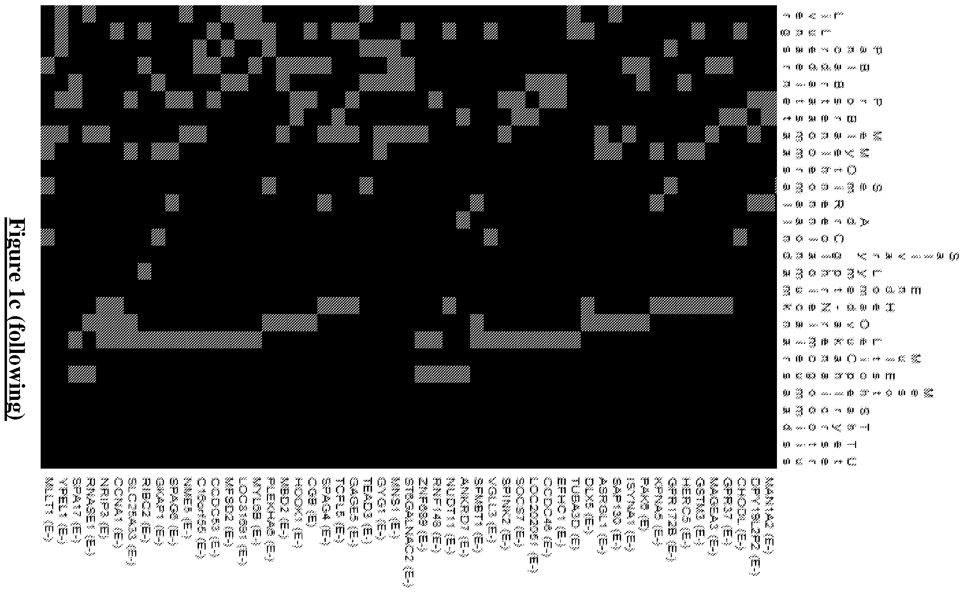

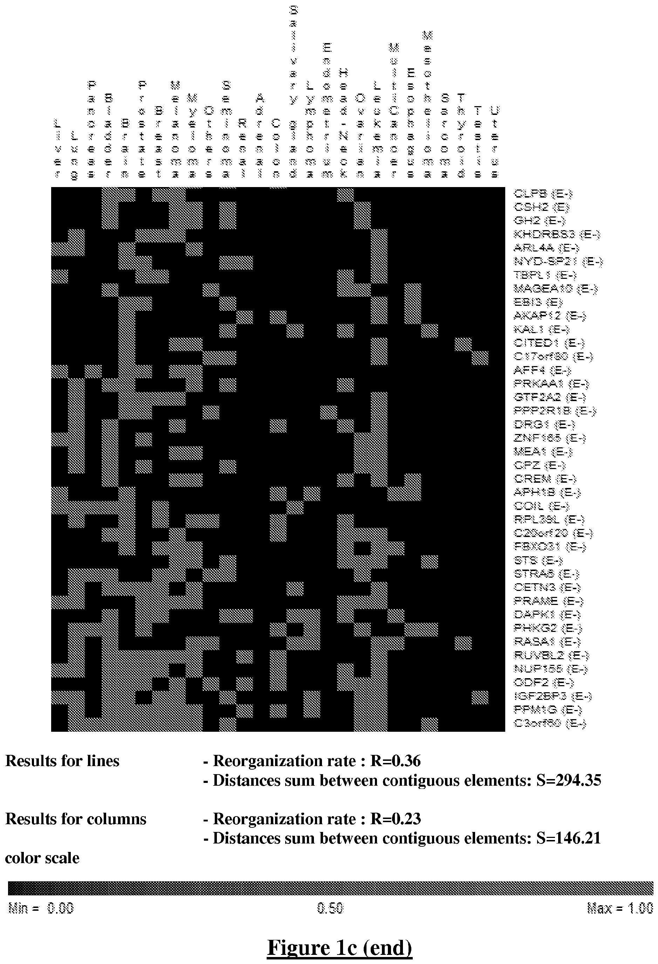

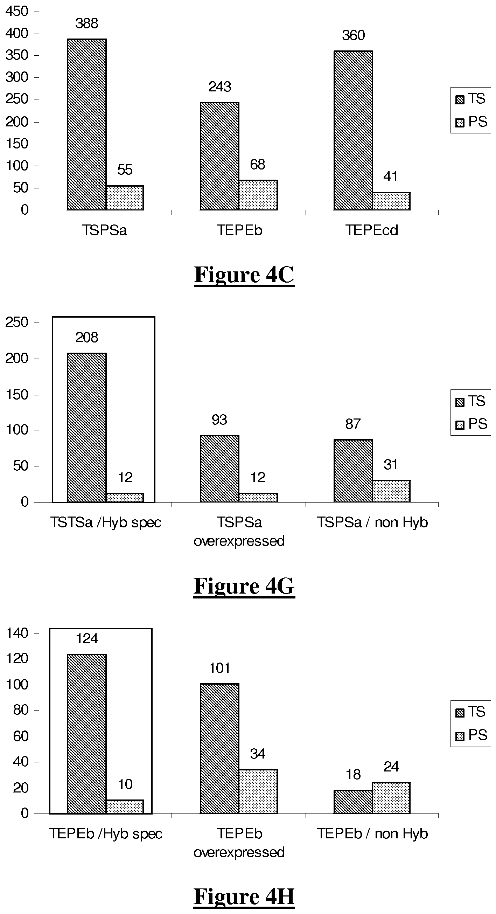

The invention is illustrated, but not limited to, by the following examples 1 to 3 and the following figures 1 to 7.
**Figure 1a** represents a meta-analysis of Oncomine data, showing the aberrant expression, in somatic or ovarian cancers, of testis- or placenta- specific genes of the list (classes A to D - includes list of genes described above) and of testis- or placenta-overexpressed genes (class E&E-). The genes are represented vertically, and the different tissue-specific tumthes horizontally. Each red square corresponds to a gene overexpressed in at least one study of each type of somatic cancer compared to the corresponding normal tissue (with p<0.001). Genes found expressed in only one type of somatic cancer are displayed at the top of the map, whereas genes found overexpressed in several somatic cancers are found at the bottom of the map.
**Figures 1b** **and** **1c** respectively represent a magnification of the map results of the genes belonging to class A to D (1b) and E to E- (1c).
**Figure 2a** summarizes the results of the first version of the CT chip represented as a hierarchical clustering of the genes belonging to the indicated categories. This was done using "permutmatrix" software (free online http://www.lirmm.fr/∼caraux/Permu tMatrix/).
**Figures 2b** represents a clustering magnification of testis and placenta specific genes as detected on the first version of the CT chip.
**Figure 2c** recapitulates CT chip (first version) global results with testis- and placenta-expressed genes. Number of testis- or placenta- specific (A-D) or testis- or placenta-overexpressed (E) genes showing no hybridization (No Hyb), no specific hybridization with one probe or one of many probes, or displaying a testis- or placenta- specific pattern of expression are represented.
   NA on chip: number of genes absent from this first version of the CT chip (not analysed here); No hyb : no expression detected in any of the analysed tissues, Non spe one probe: genes found expressed in at least one somatic tissue (with one probe), Non spe one of many probes: genes with different profiles of expression depending on the probe and found expressed in at least one somatic tissue ; Testis or placenta spe: genes with a restricted expression pattern in the testis and/or placenta according to the 1^{st} version of the chip.

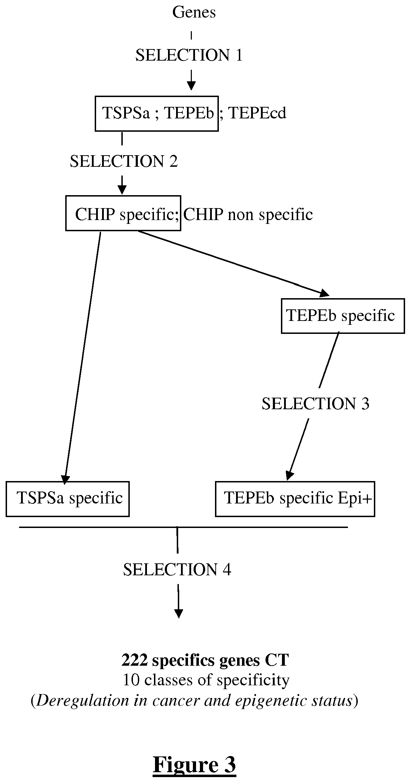

**Figure 3** represents the strategy for the determination of the 222 CT genes according to the invention and the 10 corresponding groups.
   Selection 1 corresponds to the analysis of the existing expression data in normal tissues, and classification of genes according to their specificity of expression in testis or placenta in 4 classes TSPSa, TEPEb, TEPEc and TEPEd.
   Selection 2 corresponds to the analysis of the expression of TSPSa and TEPEb genes in normal and non cancerous tissues on a dedicated microarray (version 2) comprising polynucleotide probes SEQ ID NO 415 to SEQ ID NO 2989, and selection of genes only expressed in testis or placenta (specific or non specific)
   Selection 3 corresponds to the analysis of the epigenetic status of TEPEb_spe genes (TEPEb genes expressed specifically in Testis or Placenta in the microarray) in fibroblasts and Embryonic Stem (ES) cells and selection of genes with a specific « germ-cell signature ».
   Selection 4 corresponds to the selection of genes significantly overexpressed in at least one study comparing cancer samples with normal samples of the corresponding tissue (p<0.05) or found expressed in at least one cancer sample of the CT chip v2 (Example 3) and the classification according to epigenetic status (promoter CpG content and methylation in somatic cells) and frequency of deregulation in cancer.
**Figures 4****:**

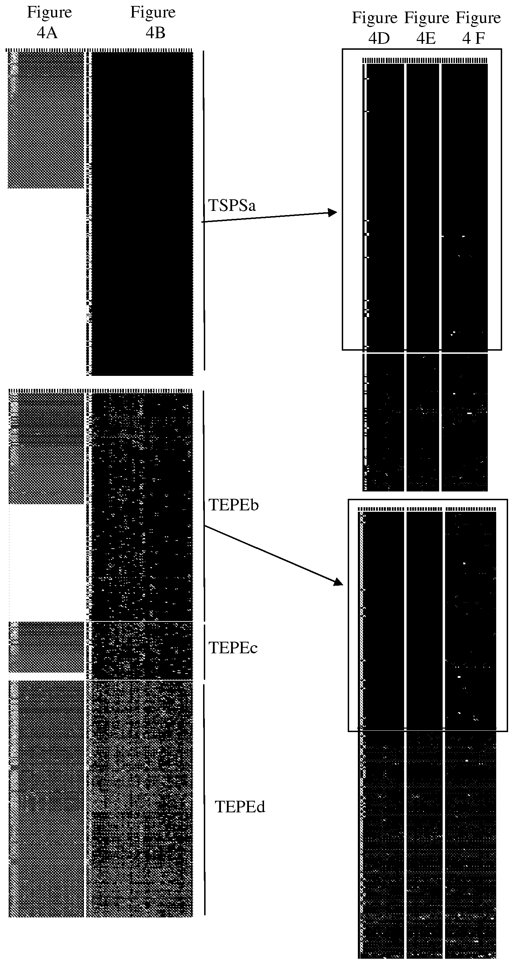

   **Figure 4A** represents the heatmap of the Symatlas online transcriptomic data.
   **Figure 4B** represents the heatmap of the EST online data.
      TSPSa, TEPEb, TEPEc and TEPEd genes are indicated
   **Figure 4C** represents the distribution of genes defined from the Symatlas and EST studies.
   **Figure 4D** represents the heatmap of the experimental transcriptomic data from the 2nd version of the dedicated microarray (CTChip_v2) (expression in normal somatic tissues)
   **Figure 4E** represents the heatmap of the experimental transcriptomic data from the 2nd version of the dedicated microarray (CTChip_v2) (expression in non-cancerous samples)
   **Figure 4F** represents the heatmap of the experimental transcriptomic data from the 2nd version of the dedicated microarray (CTChip_v2) (expression in cancerous samples)
      In all heatmap representations of figures 4A, 4B and 4D to 4F, the genes are classified according to their specificity in testis or placenta.
   **Figure 4G** represents the distribution of TSPSa genes defined experimentally (according to their expression on the second version of the CT chip), said TSPSa genes having an expression restricted to Testis and Placenta,.
   **Figure 4H** represents the distribution of TEPEb genes defined experimentally (according to their expression on the 2nd version of the dedicated microarray (CTChip_v2), said TEPEb genes having an expression restricted to Testis and Placenta, and sporadically expressed in some somatic tissues in Symatlas or EST data.

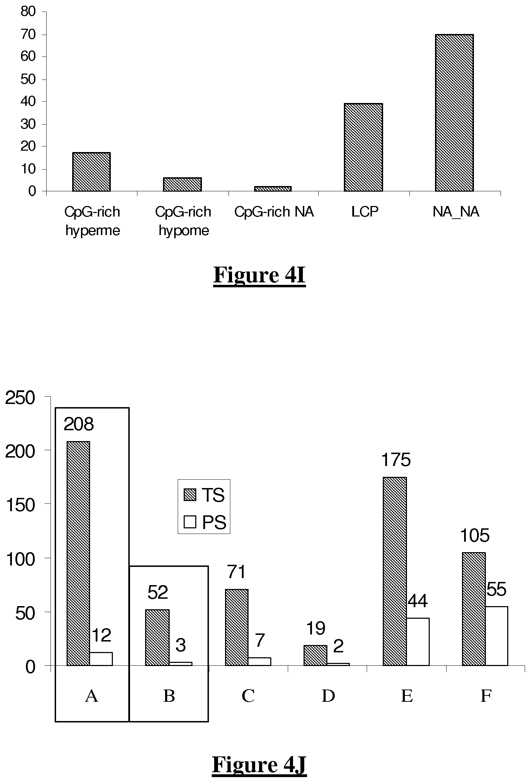

   **Figure 4I** represents the epigenetic status of the TEPEb genes highly overexpressed in testis or placenta according to Symatlas and EST data, with less than 30% non testis or placenta ESTs, and which are specifically expressed on the microarray (CTchip version 2). Genes are classified according to the presence of CpG islands in the promoter region (CpG rich), the low level of CpG in the promoter (LCP) or the absence of available data on CpG content (NA_NA). CpG rich genes are subdivided according their methylation status: hypermethylated (hyperme), hypomethylated (hypome) or unmethylated (NA).
   **Figure 4J** represents the distribution of the testis and placenta expressed genes in the following categories A-F: A represents the TSPSa genes having a specific expression on the microarray, B represents the TEPEb genes having a specific expression on the microarray and being positive for epigenetic modifications (germ cell "signature"), C represents the TEPEb genes having a specific expression on the microarray and being negative for epigenetic modifications, D represents genes expressed in non cancerous cells, E represents genes expressed in somatic tissues, and F represents genes undetected in Testis or Placenta on the second version of the chip.

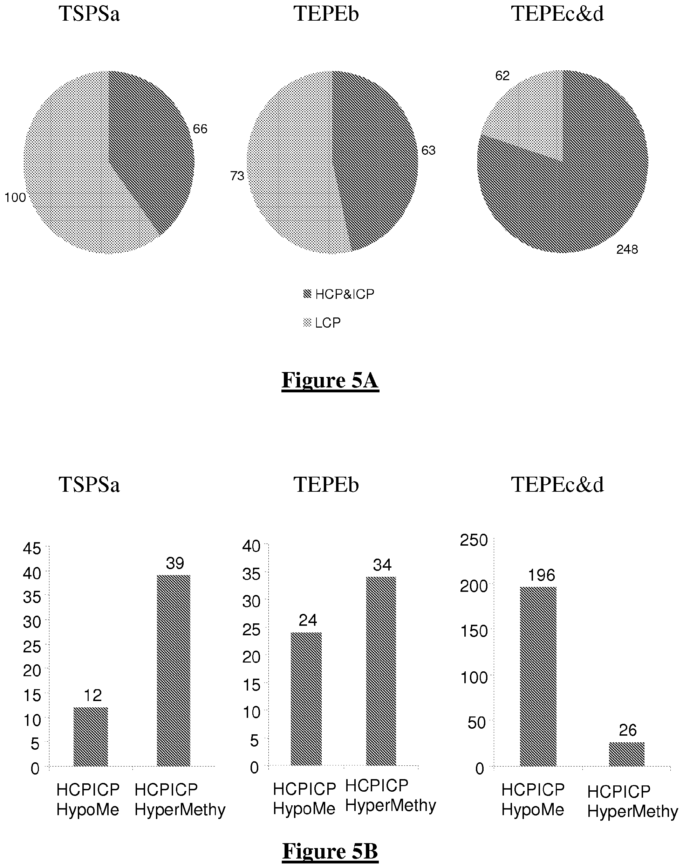

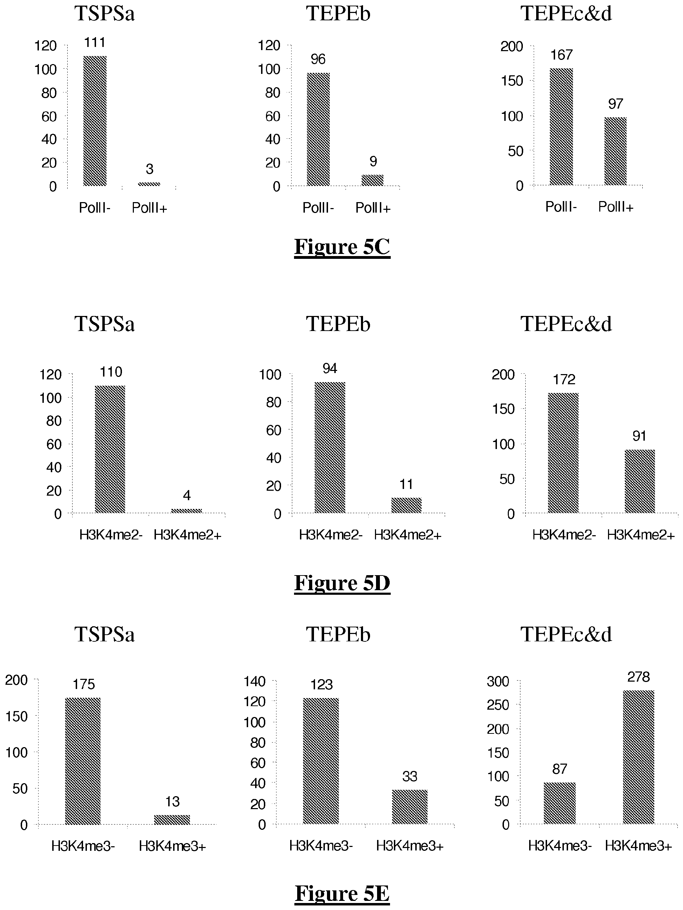

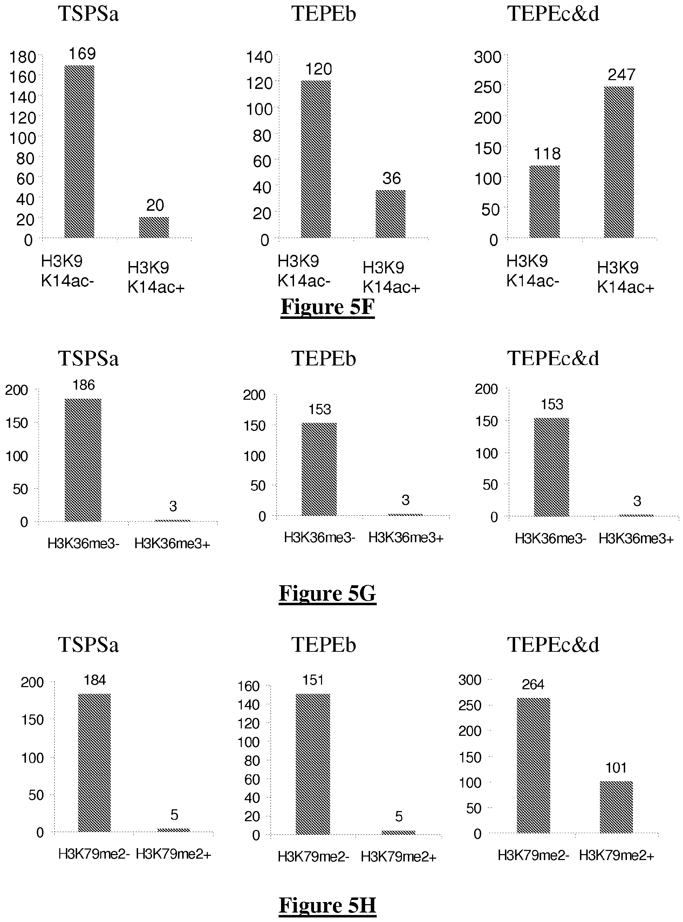

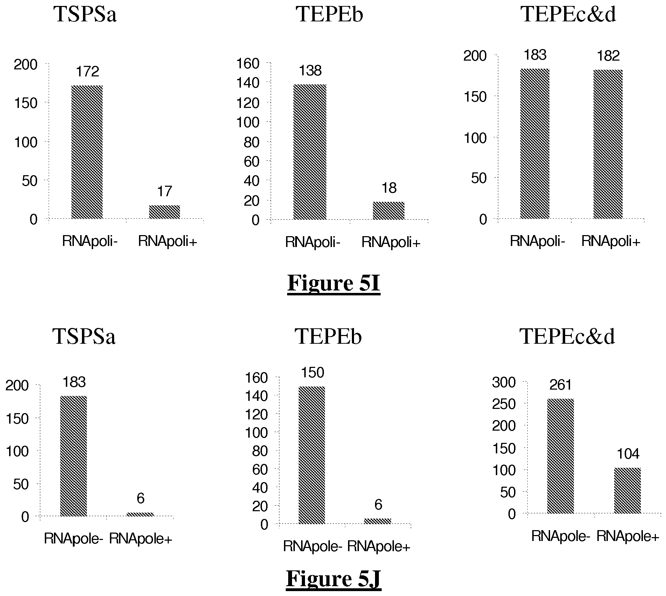

**Figures 5****:** The epigenetic characteristics of the promoter regions of the genes correlate with the specificity of their expression in testis or placenta.
Figure 5A represents the proportion of genes which belong to the promoter types HICP: CpG-rich promoters (or intermediate) or LCP: CpG-poor promoters, in the sub class of genes TSPSa, TEPEb or TEPEc&d.
Figure 5B represents the proportion of genes of each class TSPSa (left), TEPEb (middle) or TEPEc&d (right) according to the methylation of their CpG-rich promoters: HCPICP hypoMe = low methylation level; HCPICP hyperMe = high methylation level.
Figure 5C represents the proportion of genes for each class TSPSa (left), TEPEb (middle) or TEPEc&d (right) according to their enrichment in polymerase (PolII)
Figure 5D represents the proportion of genes for each class TSPSa (left), TEPEb (middle) or TEPEc&d (right) according to their enrichment in enrichment in histone H3 dimethylated lysine 4 (H3K4me2)
Figure 5E represents the proportion of genes for each class TSPSa (left), TEPEb (middle) or TEPEc&d (right) according to their enrichment in enrichment in histone H3 trimethylated lysine 4 (H3K4me3)
Figure 5F represents the proportion of genes for each class TSPSa (left), TEPEb (middle) or TEPEc&d (right) according to their enrichment in enrichment in histone H3 acetylated on lysines 9 and 14 (H3K9/14ac)
Figure 5G represents the proportion of genes for each class TSPSa (left), TEPEb (middle) or TEPEc&d (right) according to their enrichment in enrichment in histone H3 trimethylated on lysine 36 (H3K36me3)
Figure 5H represents the proportion of genes for each class TSPSa (left), TEPEb (middle) or TEPEc&d (right) according to their enrichment in enrichment in histone H3 dimethylated on lysine 79 (H3K79me2)
Figure 5I represents the proportion of genes for each class TSPSa (left), TEPEb (middle) or TEPEc&d (right) according to their enrichment in enrichment in initiation complex of polymerase II (RNApoli)
Figure 5J represents the proportion of genes for each class TSPSa (left), TEPEb (middle) or TEPEc&d (right) according to their enrichment in enrichment in elongation complex of polymerase II (RNApole)

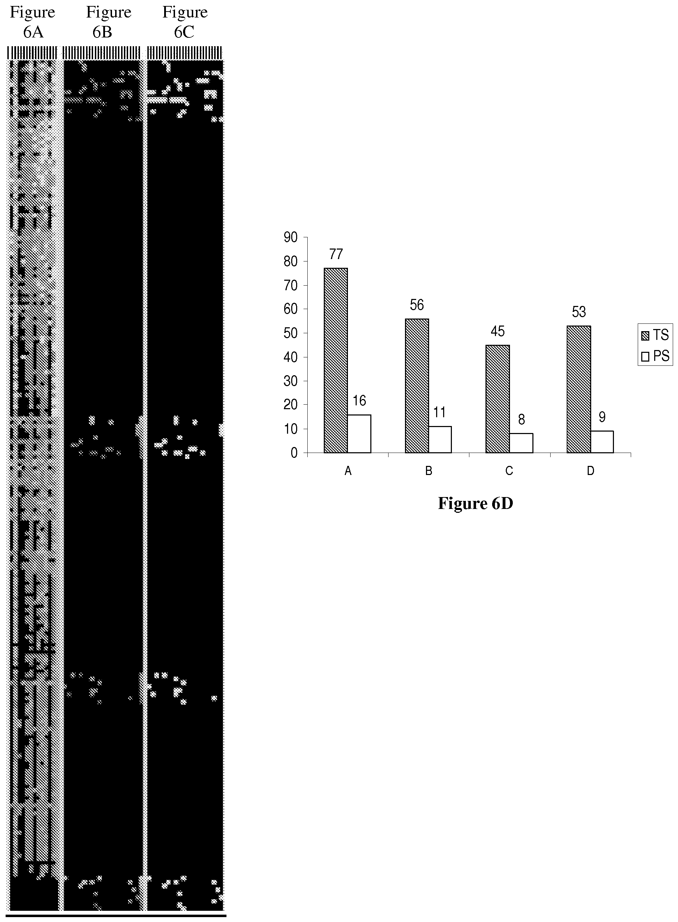

**Figures 6** **Aberrant expression of TSPS genes in somatic cancers**
**Figure 6A** represents the heatmap illustrating the aberrant expression of TSPS genes in somatic cancer according to Oncomine studies the intensity of the white was arbitrarily defined according to the p ranges, with bright white representing p<0.001 and black p>0.05 or unavailable results.
**Figure 6B** represents the heatmap illustrating the aberrant expression of TSPS genes in somatic cancer according the microarray wherein the expression values are normalized on the mean expression value for each gene on all normal tissues.
**Figure 6C** represents the heatmap illustrating the aberrant expression of TSPS genes in somatic cancer according the microarray (same as above) wherein white shows an expression and black an absence of expression.
**Figure 6D** recapitulates the data regarding the gene expression in somatic cancers (oncomine studies: results expressed as p values; and CT chip v2 (second version of the microarray): chip-= not expressed; chip+ = expressed). A corresponds to overexpressed genes in at least one OncoStudy (most significant p) p<0.001, B corresponds to overexpressed genes in at least one OncoStudy (most significant p) 0.001<p<0.01, C corresponds to overexpressed genes in at least one OncoStudy (most significant p) 0.01<p<0.05, D corresponds to genes not overexpressed in OncoStudy (Example 3).
**Figure 7****: Classification of CT genes according to their epigenetic status and deregulation in somatic cancer**
Figure 7A represents the distribution of TSPS genes according to their epigenetic status (class and methylation of promoter region ; see legend to fig 5A and %B) and their aberrant expression in somatic cancer according to oncomine studies (see legend of fig 6)
Figure 7B represents all genes aberrantly expressed in somatic cancers according to the CTchip v2 (second version of the microarray), 53 of which were already found expressed in somatic cancer(s) according to oncomine and 9 were either not studied in oncomine studies or not found overexpressed in cancer. Groups are defined above and in Example 3.

### EXAMPLES

### Example 1: In silico identification of testis-specific (TS) and placenta-specific (PS) genes

The inventors have undertaken a large-scale identification of "Cancer Testis" (CT) genes, which are normally specifically expressed in the male germinal cells (and/or the placenta) but illegitimately expressed in somatic cancer cells.
Combining transcriptomic and EST data has led to the identification of 467 human genes, specifically expressed in male germinal cells (TS genes) or placenta (PS genes).
In order to investigate the aberrant expression of these genes in somatic cancers, the inventors took advantage of cancer transcriptomic data and search engine available on the oncomine website (http://www.oncomine.org/main/index.jsp), and found that 250 of the testis- or placenta- specific genes were illegitimately expressed in somatic cancers recorded in Oncomine and each cancer type expressed a variable number of these genes. This database mining approach has been very efficient in identifying new CT genes.

This list of CT genes will provide the basis to develop new tools for the diagnosis, follow-up and treatment of cancers. In addition, the 196 placenta or testis-specific genes which have not been found over-expressed in any somatic cancer in Oncomine, could however be deregulated in other cancer types, for which the expression data have not been studied yet.

### 1-Identification of testis-specific (TS) and placenta-specific (PS) genes

### Identification of human testis-specific (TS) and placenta-specific (PS) sequences using available EST data

In order to search for testis-specific EST sequences, the following query was made in unigene (ncbi website: http://www.ncbi.nlm.nih.gov/entrez/query.fcgi?CMD=search&DB=unigene): " testis"[restricted] AND HOMO SAPIENS.

A similar query was made for the identification of placenta-specific transcripts: " placenta"[restricted] AND HOMO SAPIENS).

### Identification of human testis-specific (TS) and placenta-specific (PS) genes using available transcriptomic data

The Genomics Institute of the Novartis Research Foundation ("GNF") Gene Expression Database SymAtlas displays transcriptomic data obtained from designed custom arrays, which interrogate the expression of most protein-encoding human and mouse genes in a panel of 79 human and 61 mouse tissues (http://symatlas.gnf.org/SymAtlas/) (ref (Su et al. 2004)).
Two query strategies have been undertaken to interrogate the expression of human genes. The first approach searched for testis-overexpressed genes, which expression in testis germinal cells and/or testis seminiferous tubules was ten fold over the median of expression in all tissues, in at least one probe set, using GNF1H gcRMA as well as MAS5 condensed datasets. The expression profile images were then downloaded and the testis-specific expression checked upon.
In the second approach a similar query was made, but searching for testis-overexpressed genes, which expression in germinal cells and/or seminiferous tubules was three folds over the median of expression in all tissues, and the expression in all the other tissues was less than 2 folds over the median. The expression data in the following tissues or cell lines were excluded from this search: 721 B lymphoblasts, colorectal adenocarcinoma, leukaemia chronic myelogenous k562, leukaemia lymphoblastic molt4, leukaemia promyelocytic hl60, lymphoma Burkitt Daudi, lymphoma Burkitt Raji, Ovary, Placenta, and other Testis tissues.
Transcriptomic studies are based on the hybridization of labelled cDNA from the different tissue/cells with oligonucleotides attached to a solid surface (oligoprobes). The sequence specificity of the oligonucleotide sequences defines the hybridization specificity. Depending on its sequence, an oligoprobe can be specific for a single gene product or for a family of genes. Moreover, some oligoprobes are only hybridizing with spliced transcripts. In transcriptomic arrays, each gene is represented by one or more oligonucleotide(s), some of them being more specific than others. In the present approach, a gene was considered overexpressed in male germinal cells when hybridization data obtained with all its oligoprobes showed a «testis specific » profile.
A similar approach was undertaken to identify genes specifically over-expressed in the placenta.

These combined approaches allowed the identification of 733 genes, of which 655 were testis-expressed according to the EST and/or transcriptomic data, and 78 were identified as placenta-expressed. A selection procedure was then undertaken in order to select for genes whose pattern of expression was restricted to the testis and/or the placenta.

### Classification of the testis-expressed and placenta-expressed genes according to their tissue-specificity data

The 733 genes identified above were then classified according to their tissue specificity.

For this purpose, it was assumed that the specificity of the EST sequences prevailed for the following reasons. The ESTs are the result of the systematic sequencing of expressed sequences in specific tissues, whereas transcriptomic data highly depend on the specificity of the oligonucleotide(s) present on the array. Depending on its sequence, the latter could be not entirely specific to a particular gene, and could hybridize with the transcripts of the whole gene family, some of them being non testis- or placenta-specific. This would result in a non-specific hybridization profile of a testis- or placenta-specific gene. Conversely, the chosen oligoprobe(s) could represent a testis- or placenta-specific splicing variant of a particular gene, which expresses other somatic-expressed variants. The resulting hybridization profile with one or several probes would be testis- or placenta- specific, but the gene itself would be expressed in several somatic tissues. For the above reasons, the classification of testis and placenta expressed genes was based on the specificity of the corresponding EST sequences.
For each gene, when more than 20 ESTs were available, a ratio "R0" was defined by the number of ESTs found in tissues other than testis or placenta over the total number of ESTs. The ratio R0 represents the proportion of EST sequences, which were not found in testis or placenta.
Another special mention concerns the testis or placenta expressed genes, which are also expressed in the brain. Indeed, many testis- or placenta-expressed genes are also expressed in the brain. One important characteristic of the testis-specific genes is that they encode products, which are normally kept separated from the immune system by the blood-testis barrier. Placenta-specific genes are in a similar situation. Their illegitimate expression in somatic tissues can induce an immune response, and most of the applications of the CTs are based on their immunogenic properties. Since a similar barrier exists in the brain, the genes, which are expressed in the brain, are also protected from the immune system under normal circumstances, and potentially immunogenic if illegitimately expressed in other tissues. Therefore, those of the testis- and placenta- expressed genes, which are also expressed in the brain, but not in other tissues, were here included in the list of testis- or placenta-specific genes.

For each gene for which more than 20 ESTs were available, another ratio, **"R1",** was calculated defined by the number of ESTs found in tissues other than testis or placenta or brain, over the total number of ESTs. Hence, R1 defines the proportion of EST sequences, which were not found in testis or placenta or brain.

The specificity classes of the genes were based on this ratio R1, and defined as follow.

**Class A** was defined by testis- or placenta-expressed genes, which displayed a specific expression profile according to the Symatlas data, as well as a specificity of their ESTs with R1 = 0. Genes of classes A- and A-- also displayed a specific Symatlas expression profile, but showed a small proportion of non-specific ESTs: up to 20% (A- genes) or 30% (A-- genes) of the corresponding ESTs were found in somatic tissues (other than testis or placenta or brain) (A- genes: R1 ratio between 0 and 0.2; A-- genes: R1 ratio between 0.2 and 0.3). For these genes, the arguments for a testis or placenta specificity of expression are strong.

**Class B** was defined by the genes with testis or placenta specific ESTs (R1=0), but for which no transcriptomic data were available in Symatlas. Genes of classes B- and B-- showed small proportions of non-specific ESTs : up to 20% (B- genes) or 30% (B-- genes) of the corresponding ESTs were found in somatic tissues (other than testis or placenta or brain) (B-genes: R1 ratio between 0 and 0.2 ; B-- genes: R1 ratio between 0.2 and 0.3). Since EST specificity does not depend on probe choices or hybridization conditions, this group of genes could be considered as testis- or placenta- specific with high reliability.

The genes of **class C** also showed testis or placenta specificity of their ESTs (R1=0), but their transcriptomic profiles in Symatlas were not. The most likely explanation for this apparent discrepancy is a lack of specificity of the chosen oligoprobes, which could hybridize with somatic expressed genes of the same family sharing similar sequences. Classes C- and Cincluded genes with a small proportion of non-specific ESTs : up to 20% (C- genes) or 30% (C-- genes) of the corresponding ESTs were found in somatic tissues (other than testis or placenta or brain) (C- genes: R1 ratio between 0 and 0.2 ; C-- genes: R1 ratio between 0.2 and 0.3). Although the transcriptomic data available in symatlas did not convincingly show specific patterns of expression, the large predominance of the corresponding ESTs in testis or placenta suggested that they could be considered as testis- or placenta- specific genes with reasonable reliability.

Genes grouped in **class D** displayed a testis- or placenta- specific expression profile according to symatlas transcriptomic data, but too few EST sequences were available in total. Indeed less than 20 recorded transcripts per Million was considered insufficient for tissue-specificity studies.

Genes of **class E and E-** displayed a testis- or placenta- specific expression profile according to symatlas transcriptomic data, but more than 30% (E) or 50% (E-) of non-specific ESTs were found in somatic tissues (other than testis or placenta or brain). The presence of ESTs in some somatic tissues suggested that, although the genes of E and E- classes were overexpressed in testis or placenta, they are not entirely testis-or placenta-specific. For the genes of the E class, the number of testis and/or placenta transcripts exceeded the number of somatic transcripts, whereas for the E- genes, the somatic transcripts outnumbered the testis and placenta transcripts. These genes were excluded from the final list of TS and PS genes.

From this confrontation between EST specificities and SymAtlas transcriptomic data, a classification of the list of TS and PS genes was established according to their specificity.

### Quality control using other available published data

Data available from other published studies were used to check that the list of testis-specific genes was exhaustive.
In the first study (Fox et al. 2003), the authors have identified 800 sequences differentially expressed between human adult normal testes and Sertoli Cell Only testes (testes with no germinal cells). By using Symatlas and EST data (as above), among these sequences, the inventors found **57** testis-specifically expressed genes. All of these genes were redundant with those identified above.
In the second study (Schultz et al. 2003), 385 mouse testis-specific genes were identified and clustered in five groups, according to the time of their expression in pre-pubertal mice. Using a nucleotide blast search (http://www.ncbi.nlm.nih.gov/BLAST/) the inventors have systematically looked for a human homolog for each of the testis-specific mouse gene, which has led to the identification of 233 human genes. By selecting those that presented a testis-specific expression (according to SymAtlas and/or EST data) the inventors were able to identify **29** human testis-specific genes, all of which were also redundant with those identified with the approaches described above.

Finally, the human homologs of rodents genes, with were described as meiotically or post-meiotically differentially expressed in the male germinal cells (Chalmel et al. 2007), were searched for, and their testis-specificity recorded. Of the **244** human homologs, **64** were found testis-specific according to transcriptomic symatlas data, of which **13** also showed testis-specific ESTs, and one gene was specific of the placenta in the human. All these **65** genes were present on the list of testis- and placenta-expressed genes. The other genes, absent from the list, did not show any clear evidence of testis-overexpression.
Finally, a recent study, recording several tissue-specific genes on the basis of transcriptomic data, have listed 242 genes as being "testis-specific" (Bock-Axelsen et al. 2007). However only 51 of them are redundant with the list of TS genes (classes A to D). Another 74 genes were also found in the testis-overexpressed classes of genes (classes E and E-) but could not be considered as "testis-specific" because of the absence of specificity of their ESTs. The other 117 genes either showed a testis-specific profile with only some of the symatlas probes and a non-specific expression with other probes suggesting that they correspond to differentially spliced genes (43 genes), or displayed a non-specific expression profile with all symatlas probes, as well as non-specific ESTs (74 genes).

Hence, all the above sets of data support the evidence that, the list of germline specific genes includes all genes exclusively expressed in the testis, for which expression data are presently available.

The status of DNA methylation of the promoter of a gene could also provide information on its specificity. Indeed Schubeler and collaborators have recently systematically characterized the DNA methylation status of the promoter regions of the whole human genome in primary fibroblasts (representative of normal somatic cells) and in sperm cells (Weber et al. 2007). They have observed that CpG rich promoters were generally hypomethylated in somatic cells, apart from the germline specific genes promoters, which were generally hypermethylated in fibroblasts and hypomethylated in sperm. Checking the DNA methylation data obtained from their study (http://www.fmi.ch/members/dirk.schubeler/supplemental.htm) on the promoters of the genes, which the inventors have listed as testis-specific (TS, classes A to D), the inventors have indeed found i/ that approximately half of them had CpG-rich or intermediate promoters, and ii/ that 70% of these CpG-rich/intermediate promoters were hypermethylated. In contrast, the testis-expressed genes, which had been found overexpressed but not specifically expressed in the testis (classes E and E-), although showing a higher proportion of CpG-rich/intermediate promoters (77%), displayed a much lower percentage of hypermethylated CpG-rich/intermediate promoters (13%). Hence, the testis specificity of the genes of the TS- list is not only confirmed by ESTs and transcriptomic data, but also by epigenetic marks.

### 2- Classification of TS and PS genes according to their expression in cancer

### Known and new CT genes

A review of the literature and the data available online (http://www.cancerimmunity.org/CTdatabase/) show that 72 genes have been so far recorded as CT genes. Twelve of these genes (10 TS and 2 PS) are redundant with the present list of TS-PS genes deregulated in cancer. Therefore 12 of the testis- and placenta- specific genes identified by us had already been described as "Cancer Testis" genes, deregulated in several somatic cancers.

However, 60 known CTs were not identified as CTs by the approach. The main reason is that these genes did not meet the testis or placenta specificity criteria, which were used to establish the list. Indeed, nine of the known CT genes (7 testis-expressed and 2 placenta-expressed) were found among the testis- or placenta overexpressed genes (classes E and E-). The other 51 did not show specific patterns of expression according to symatlas transcriptomic data and/or EST sequences specificity. They therefore did not qualify as CTs on the basis of the criteria.

In addition, Scanlan and collaborators have described the following genes as CTs, in a published work as well as in WO 2006/029176. Nine of the genes they describe are redundant with the TS genes and have been removed.

Altogether, an exhaustive survey of the literature on the subject demonstrates that, although the discovery and study of CTs have raised a lot of interest because of their potential use in cancer diagnosis and/or treatment, these medical applications have so far been hampered by the fact that all known CTs are sporadically expressed in cancers. No "perfect" CT or group of CTs had been found, which could allow a reliable and highly specific detection and/or targeting of all cancer types.

The present list records the first large-scale identification of genes with "Cancer Testis" specific restricted patterns of expression. As a whole, this list provides a basis for the development of reliable tests and therapy approaches available for all cancer types. These approaches are based on the known properties of "Cancer Testis" genes.

### Expression of testis- or placenta- specific genes in somatic cancers

In order to extend this observation to other somatic cancers, the inventors then took advantage of the cancer transcriptomic data and search engine available on the oncomine website.
The cancer profiling database Oncomine (http://www.oncomine.org/main/index.jsp) (Rhodes et al. 2007; Rhodes et al. 2004) combines data from more than 20,000 cancer transcriptome profiles with an analysis engine and web application for data mining and visualization.

For each of the 467 testis-specific and placenta-specific genes listed above, the inventors searched the Oncomine database for an overexpression in tumthe versus normal tissue, with a p<0.001.

Thirty five of the original 467 testis- or placenta- specific genes were absent from the Oncomine database. Of the remaining 432 TS-PS- genes, this analysis revealed that 250 (58%) were found aberrantly expressed in tumthes in at least one of the transcriptomic studies recorded in Oncomine. Some of these genes were aberrantly expressed in studies comparing samples of a somatic cancer versus samples of its normal tissue counterpart (ECN: 157 genes), whereas others were overexpressed in studies comparing cancer samples with other cancer samples (CC: 93 genes). Moreover, every single cancer type recorded in Oncomine expressed a sub-set of these genes. A total of 182 TS-PS- genes were not found expressed in any of the recorded cancer studies recorded in oncomine despite being tested (NEC).

The transcriptomic data in Oncomine therefore shows that more than half of the testis specific and placenta specific genes are illegitimately expressed in at least one somatic cancer type, and that each cancer type is associated with the deregulation of a subset of TS-PS genes.
Expression of TS and PS genes in cancer, database comparition with transcriptomic data approach
This database comparition with transcriptomic data approach has enabled the Inventors to identify 250 CT genes (TS and PS genes deregulated in cancers), most of which had not been previously described as CTs.

The following table 4 illustrates the result data. Table 4 describes the expression of the testis- and placenta- specific genes of the list (the numbers of the corresponding sequences SEQ ID are displayed in the left column) in a meta analysis of the studies recorded in Oncomine comparing transcriptomic data of somatic or ovarian tumthes with the corresponding normal somatic tissue samples (the 27 columns are labelled according to the type of cancerous tissue - the values indicate the number of studies where each gene was found significantly overexpressed in the tumor samples compared to the normal corresponding tissue with p<0.001.
Oncomime column represents gene expression in studies recorded in Oncomine : illegitimate expression in studies comparing cancer samples versus their somatic counterpart (ECN) (also illustrated in figures 1a, 1b and 1c), or comparing cancer samples with other cancer samples (CC), or not found expressed in any of the Oncomine studies described above (NEC), or not recorded in Oncomine (NA).

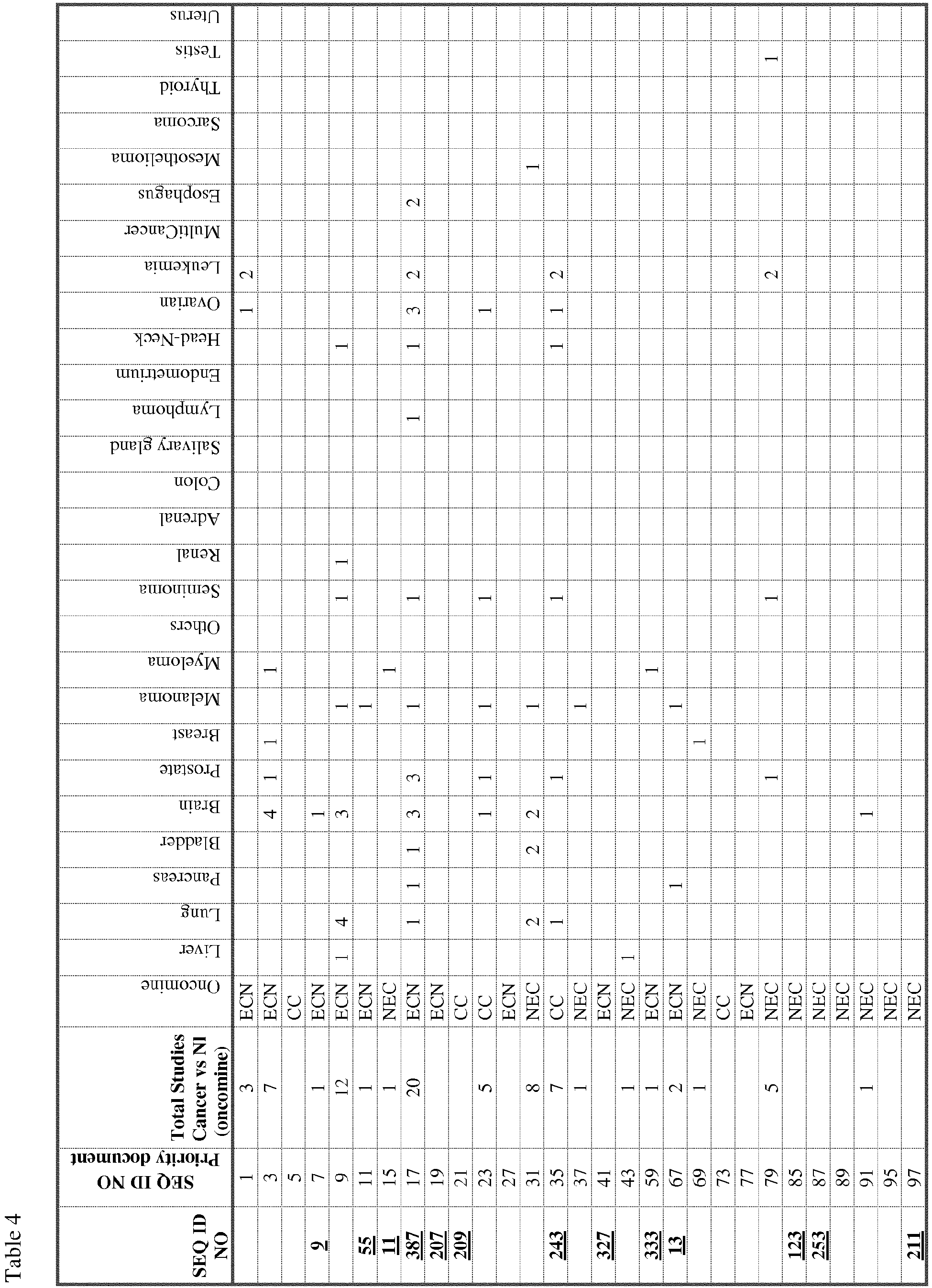

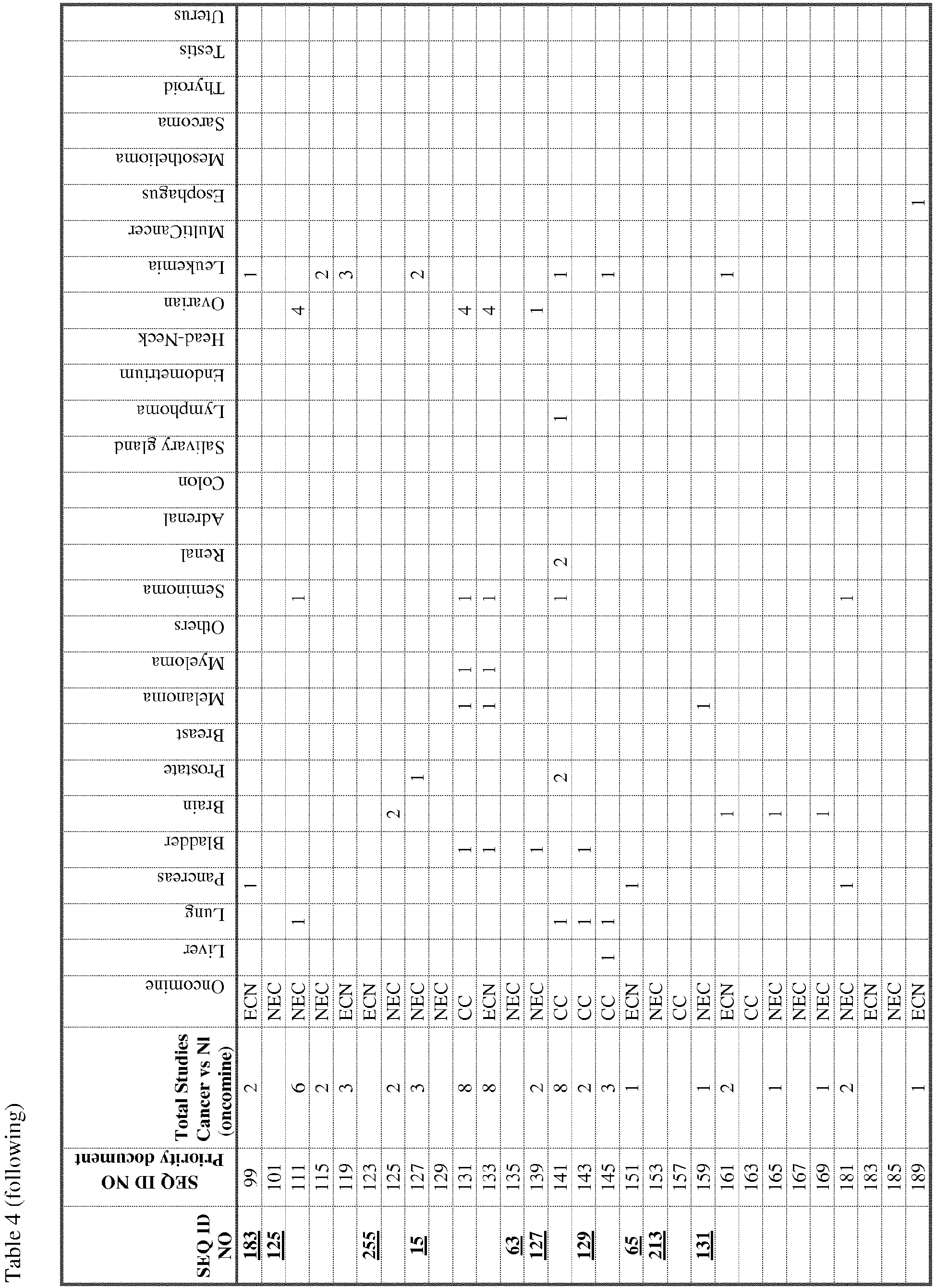

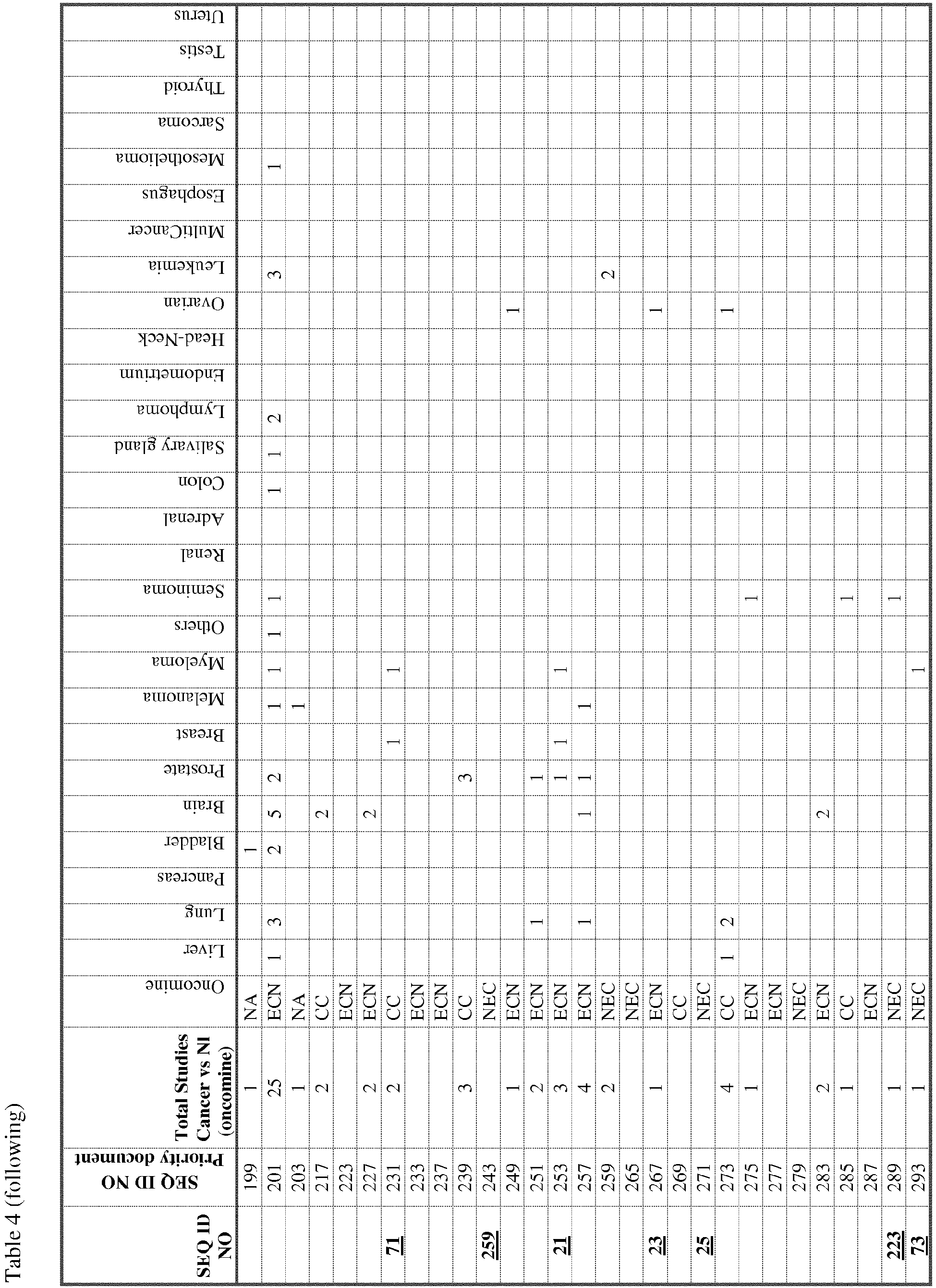

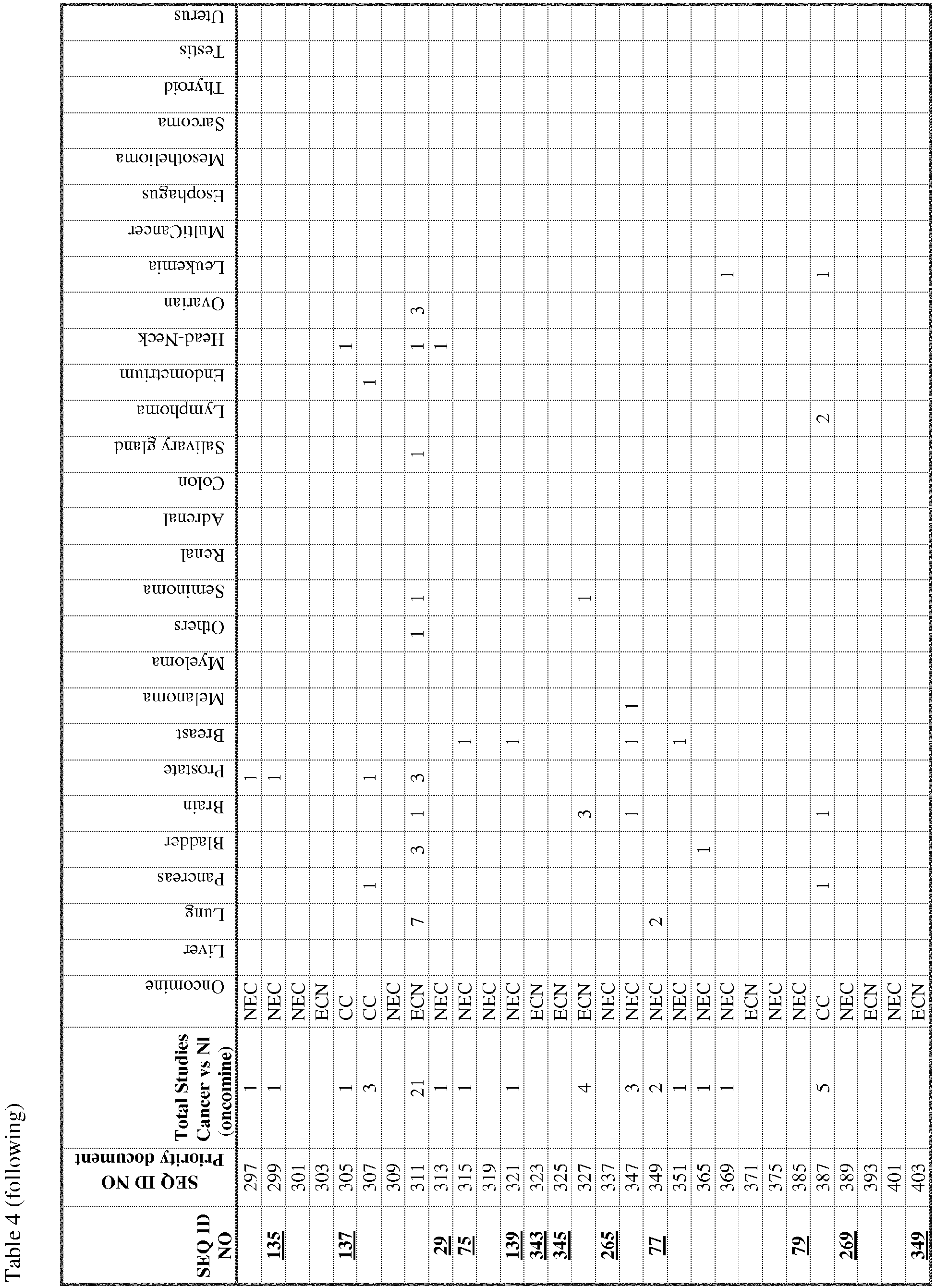

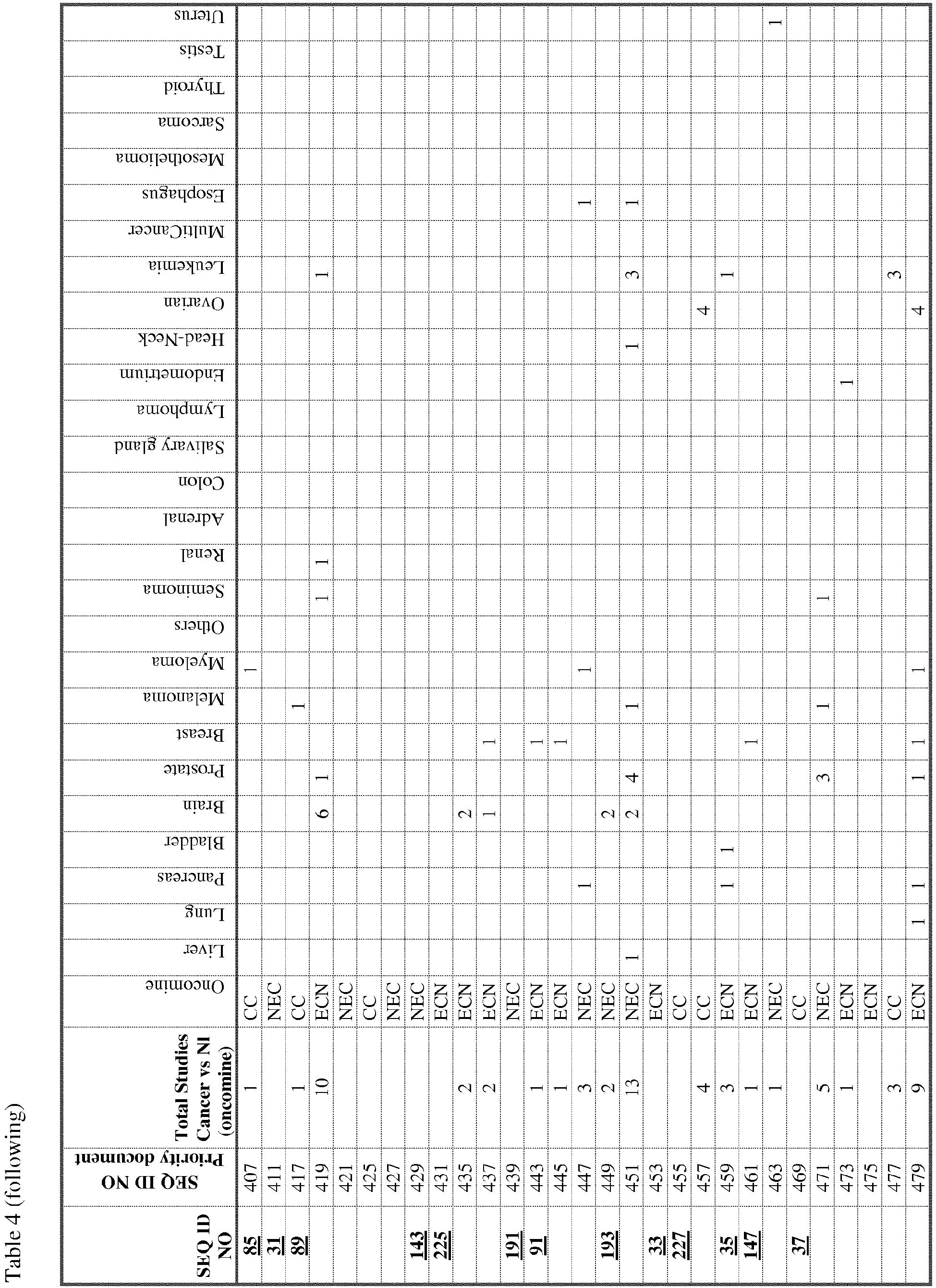

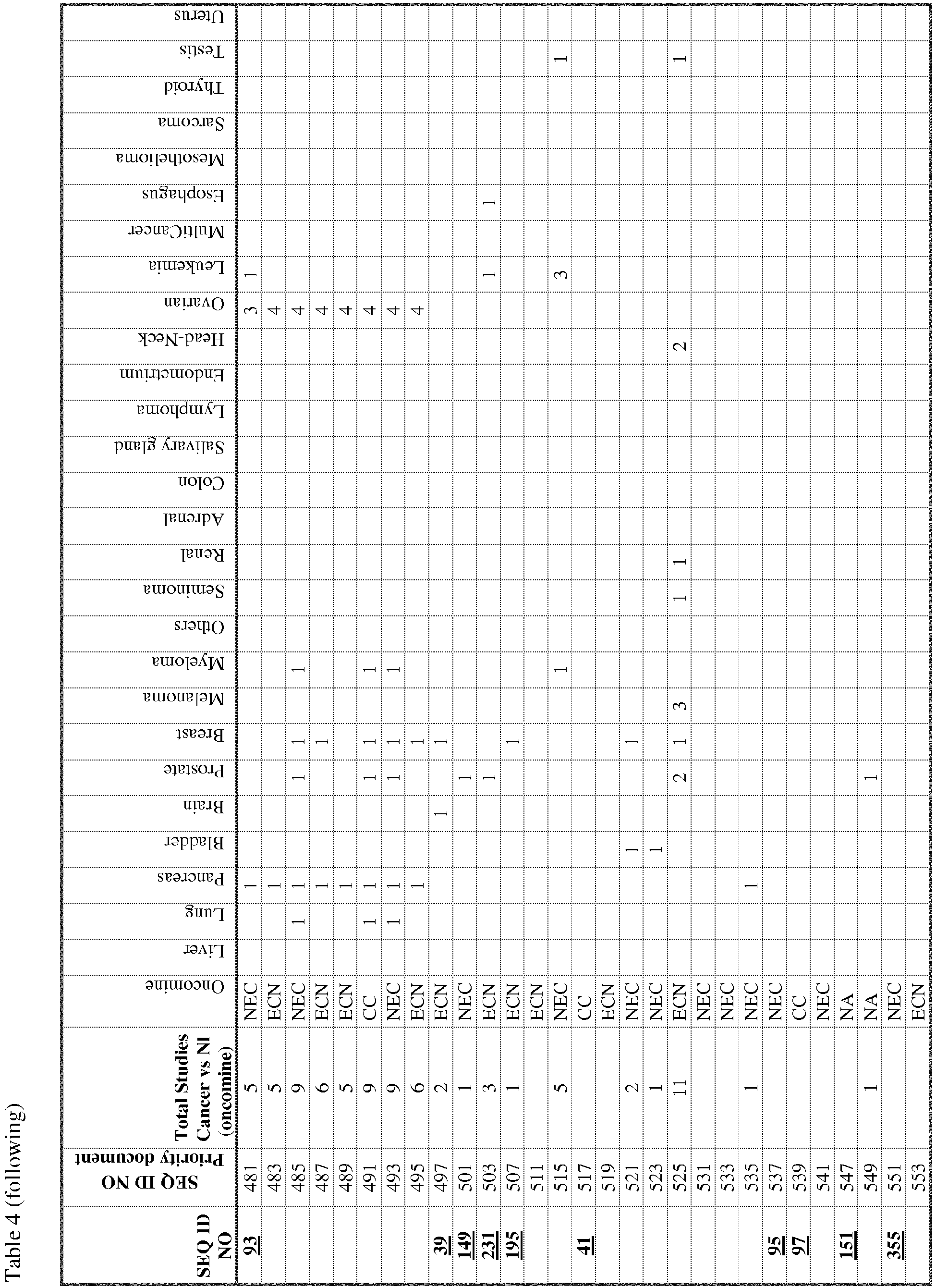

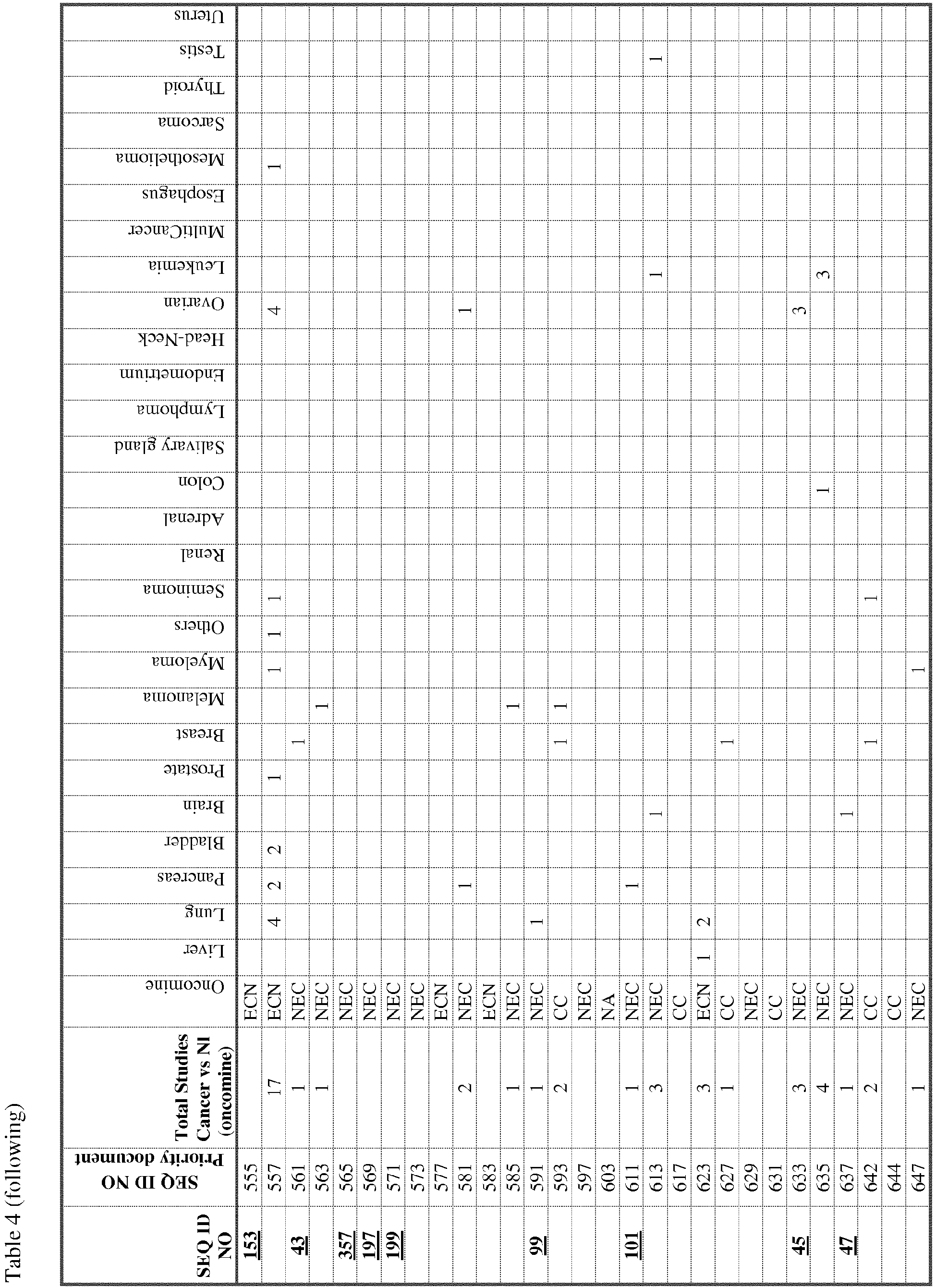

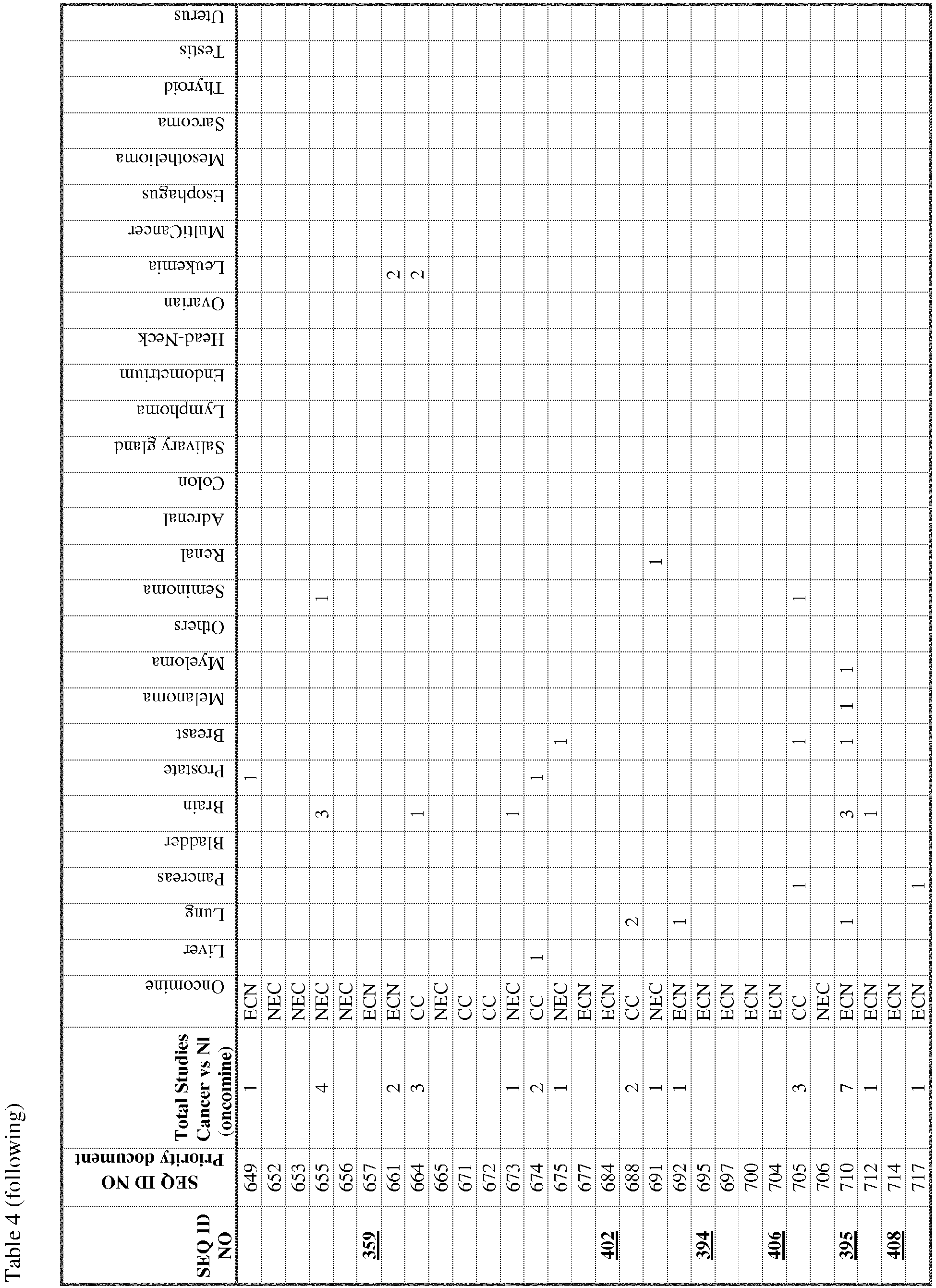

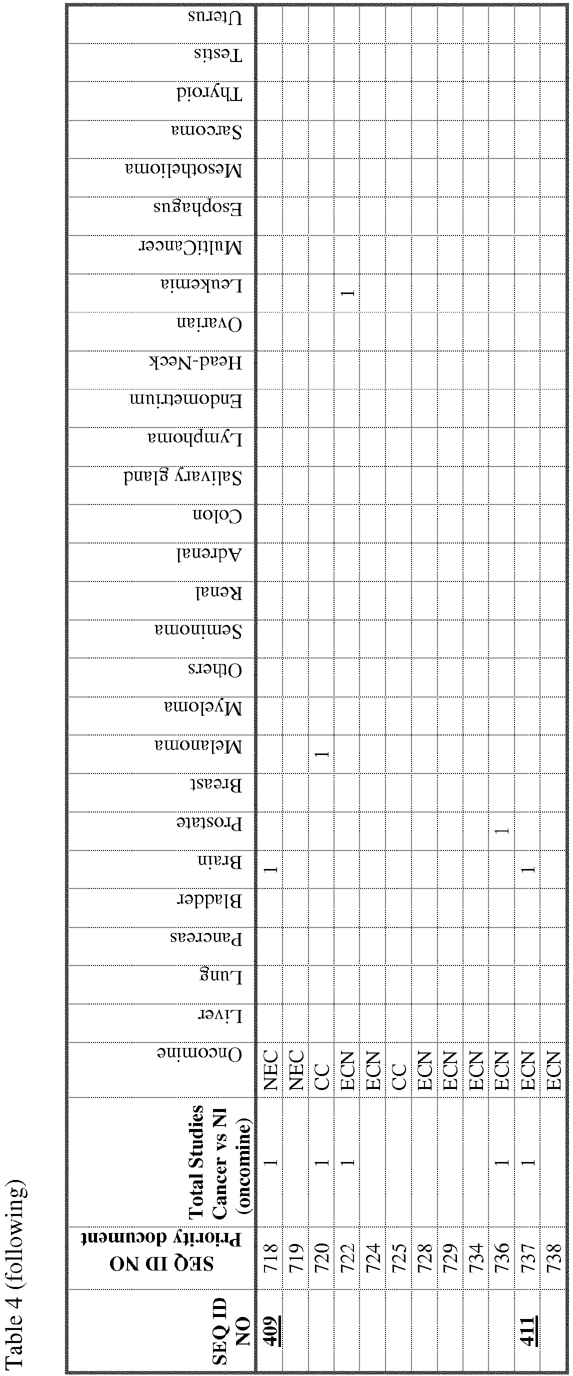

### Example 2: Development and validation of a macro-array, named "CT-chip" dedicated to analyze the expression of TS and PS genes in normal and tumoral tissues

### Objectives

The aim of this work is to design a macroarray (CTchip), based on the in silico data, which enables the detection and quantification of TS and PS genes in normal human tissues and somatic tumthes. A first macroarray was evaluated by studying the expression profile of these genes in eight samples of human tissues, including six normal tissues (placenta, testis, bladder, colon, liver, normal lung) a cancer cell line (Hela 53) and a tumthe (lung tumthe).

### Strategy and method

### 1- Identification of the genes and probes to include in the CTchip

The following categories of genes were included
- TS or PS genes (classes A to D,), n=318
- Testis- or placenta overexpressed genes (Classes E and E-, as defined above), n=241
- Genes with several splice variants including one at least overexpressed in testis or placenta and at least another one not specifically expressed in testis or placenta (Class F), or genes with no specificity or clear overexpression in testis or placenta (Classes G and H), n= 247
- Tissue-specific genes (selected according to symatlas transcriptomic data) expressed in one of the following tissues: bladder (Bl), brain (Bra), breast (Bre), colon (Co), kidney (K), liver (Li), lung (Lu), Ovary (O), prostate (Pros), skin (Sk), Thyroid (T), Uterus (U), n= 647
- Control genes expressed in all tissues, n= 334, used as hybridization controls
For each gene of the above lists, at least one specific probe was designed, corresponding to a 60 base paires sequence specific to the open reading frame or transcribed sequence of the gene.
In particular, a micro array comprising probes SEQ ID NO 421, SEQ ID NO 423, SEQ ID NO 424, SEQ ID NO 425, SEQ ID NO 426, SEQ ID NO , SEQ ID NO 427, SEQ ID NO 429, SEQ ID NO 430, SEQ ID NO 431, SEQ ID NO 432, SEQ ID NO 434, SEQ ID NO 435, SEQ ID NO 436, SEQ ID NO 437, SEQ ID NO , SEQ ID NO 444, SEQ ID NO 448, SEQ ID NO 449, SEQ ID NO 450, SEQ ID NO 451, SEQ ID NO 452, SEQ ID NO 455, SEQ ID NO 457, SEQ ID NO 458, SEQ ID NO 460, SEQ ID NO 461, SEQ ID NO 462, SEQ ID NO 463, SEQ ID NO 464, SEQ ID NO , SEQ ID NO 465, SEQ ID NO 466, SEQ ID NO 470, SEQ ID NO 471, SEQ ID NO , SEQ ID NO 472, SEQ ID NO 473, SEQ ID NO 474, SEQ ID NO 475, SEQ ID NO 476, SEQ ID NO 489, SEQ ID NO 492, SEQ ID NO 496, SEQ ID NO 497, SEQ ID NO , SEQ ID NO 499, SEQ ID NO 500, SEQ ID NO 501, SEQ ID NO 502, SEQ ID NO 503, SEQ ID NO 504, SEQ ID NO 505, SEQ ID NO 506, SEQ ID NO 507, SEQ ID NO 508, SEQ ID NO 509, SEQ ID NO 528, SEQ ID NO 530, SEQ ID NO 531, SEQ ID NO 532, SEQ ID NO , SEQ ID NO 533, SEQ ID NO 534, SEQ ID NO 535, SEQ ID NO , SEQ ID NO 536, SEQ ID NO 537, SEQ ID NO 538, SEQ ID NO , SEQ ID NO 540, SEQ ID NO 541, SEQ ID NO 545, SEQ ID NO 546, SEQ ID NO 547, SEQ ID NO 548, SEQ ID NO 549, SEQ ID NO 550, SEQ ID NO 551, SEQ ID NO , SEQ ID NO 552, SEQ ID NO 554, SEQ ID NO 556, SEQ ID NO 557, SEQ ID NO 558, SEQ ID NO 559, SEQ ID NO 560, SEQ ID NO 561, SEQ ID NO 562, SEQ ID NO 569, SEQ ID NO 571, SEQ ID NO 573, SEQ ID NO 576, SEQ ID NO 577, SEQ ID NO 578, SEQ ID NO , SEQ ID NO 581, SEQ ID NO 584, SEQ ID NO 588, SEQ ID NO 589, SEQ ID NO 591, SEQ ID NO 593, SEQ ID NO 616, SEQ ID NO 619, SEQ ID NO 621, SEQ ID NO 622, SEQ ID NO , SEQ ID NO 623, SEQ ID NO 624, SEQ ID NO 625, SEQ ID NO 626, SEQ ID NO 627, SEQ ID NO 628, SEQ ID NO 629, SEQ ID NO , SEQ ID NO 630, SEQ ID NO 631, SEQ ID NO 635, SEQ ID NO 636, SEQ ID NO 637, SEQ ID NO 638, SEQ ID NO 639, SEQ ID NO , SEQ ID NO 640, SEQ ID NO 641, SEQ ID NO 642, SEQ ID NO 643, SEQ ID NO 645, SEQ ID NO 646, SEQ ID NO 647, SEQ ID NO 649, SEQ ID NO 650, SEQ ID NO 652, SEQ ID NO 653, SEQ ID NO 567, SEQ ID NO 605, SEQ ID NO 666, SEQ ID NO 667, SEQ ID NO 668, SEQ ID NO 671 and SEQ ID NO 682, contained in the old group of the priority document (SEQ ID NO 755 to SEQ ID NO 1088) were used.

When available the existing *Agilent Technologies* probes were used. For some genes (within the first three categories above) comprising several transcripts, with or without restricted expression to testis or placenta, several probes were designed in order to detect all known variants.

### 2- CTchip design

The online software eArray (Agilent Technologies) was used to design this first version of the CTchip. The 8x15K was chosen. In order to optimize the data extraction by Feature Extraction, it was necessary to have at least 10 000 spots per array. Therefore, all the above lists of genes, apart from the control genes were replicated six times.

### 3- The following RNA samples were purchased from Applied Biosystems (France)

- Bladder
- Hela 53
- Normal lung
- Tumor lung
- Colon
- Liver
- Testis
- Placenta

### 4- Hybridization

Approach: RNA samples were hybridized on the CT chip in a one-colthe approach. This allows a direct comparison of fluorescent intensities between slides, without requirement for a common reference. Each RNA sample was hybridized three times, so that a total of 24 expression profiles were obtained.

RNA samples were first evaluated quantitatively (Nanodrop ND-1000) and qualitatively (analysis of ribosomal RNA electrophoretic profile by the Agilent BioAnalyser 2100), and then 400 ng of each sample was labelled in triplicates by incorporation of a CTP coupled with Cy3 using the Low RNA input linear amplification kit (Agilent Technologies). The concentrations of labelled cDNA were checked, adjusted at 200ng/ml, and their electrophoretic profiles were analysed as above. The labelled cDNAs were then fragmented (by denaturation at 60C for 30 min in a specific buffer) in order to obtain 50 to 200 bp fragments, which were necessary for an optimal hybridization with the 60-mers probes.
600ng of Cy3-cDNA were then hybridized at 65C during 17 hthes. The hybridized slides were read in green using an Agilent scanner device.

### 5- Data extraction

The data were extracted by the software "Feature Extraction 9.1", using a one-colthe protocol (GE1-v5_95_Feb97). Three files were generated, which include an image file (.jpg), a data file (.text) and a quality report (.pdf). The quality parameters were satisfactory, with a variation coefficient between signals obtained with the same probe of less than 10%, indicating a high reproducibility of the experiments.

In the data file, after background noise signal substraction, the mean signal obtained for each probe was normalized to give the value of the "processed signal". The value in "IsWellAboveBG" indicates if the mean signal is significantly superior to background, and whether this signal is 2.6 times superior to the standard deviation of the background noise for Cy3 (0= not significant; 1= significant).

### 6- Analysis of expression profiles

The aim was
- to identify transcripts expressed in each tissue
- to compare levels of expression in somatic tissues with those in testis or placenta and validate the tissue-specificity of the analysed genes
- to identify testis- or placenta-specific genes expressed in HeLA cells or in tumor lung.

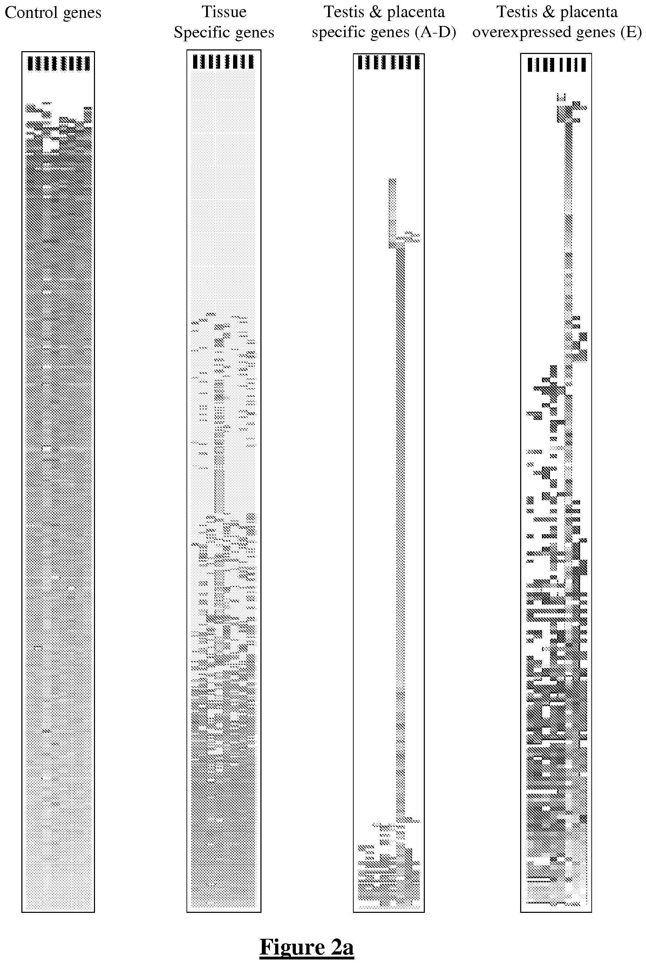

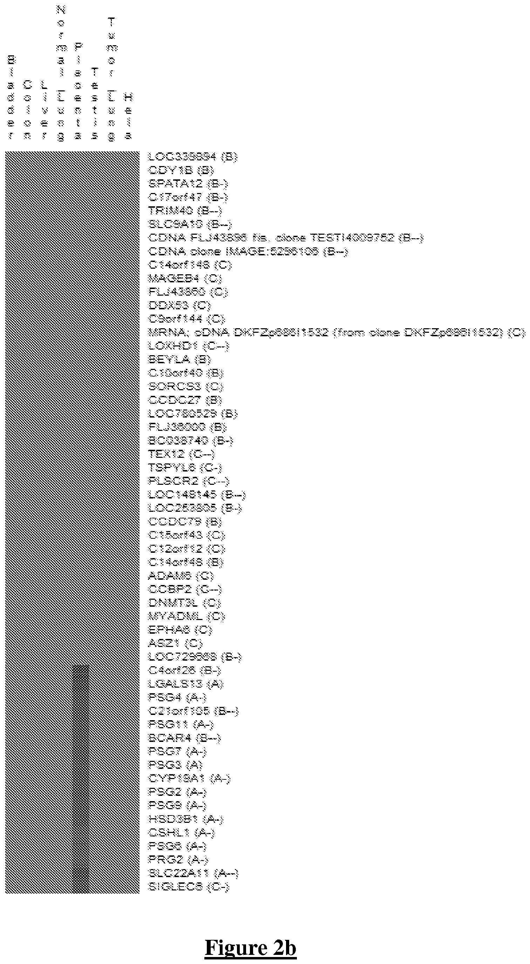

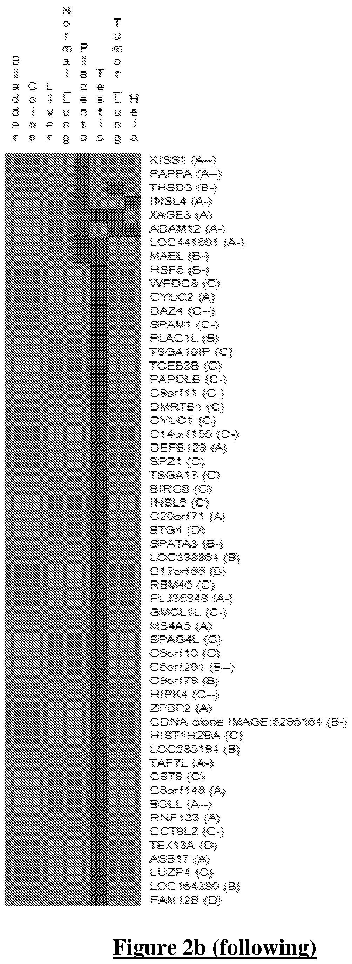

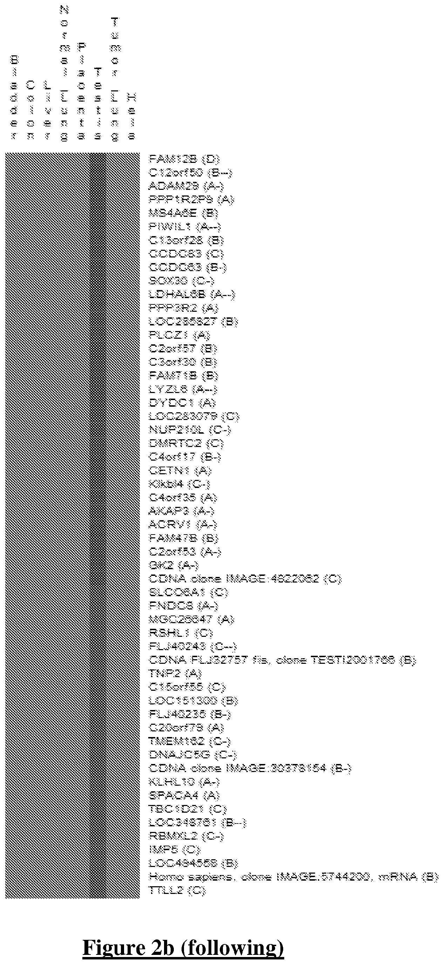

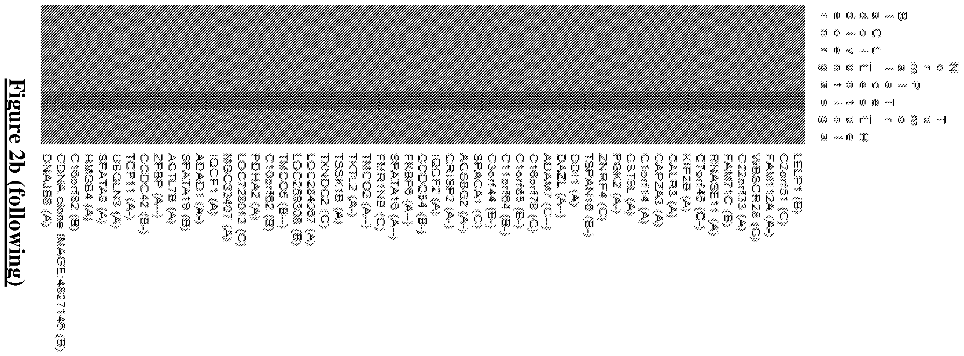

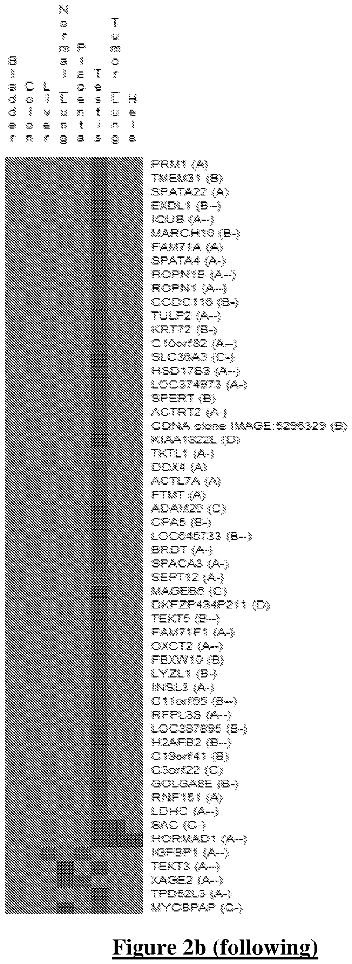

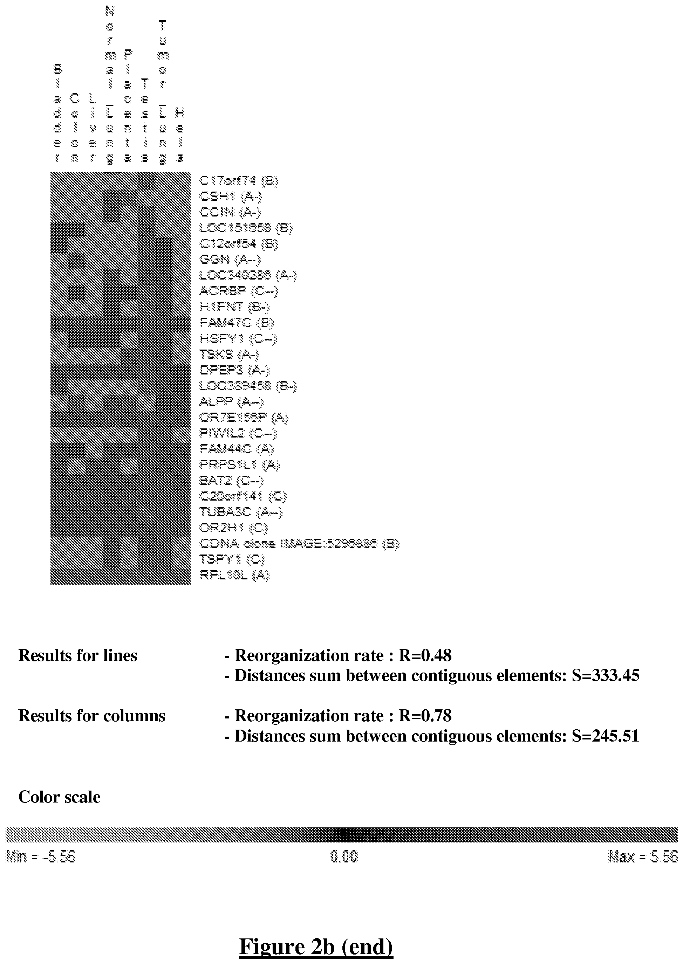

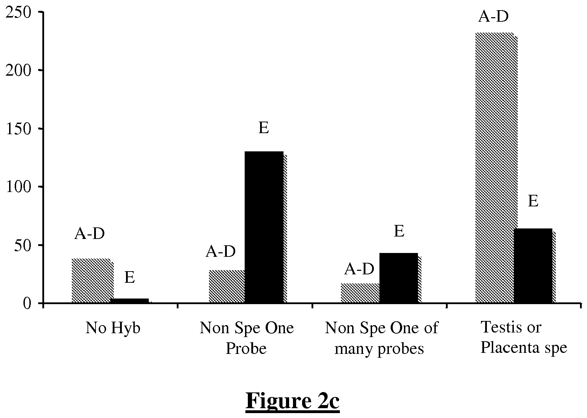

The expression profiles of the genes of the categories described above are summarized in the following Figures 2a and 2b show a hierarchical clustering of the genes belonging to the different categories. This was done using "permutmatrix" software (free online http://www.lirmm.fr/∼caraux/PermutMatrix/).

The following table 6 recapitulates the expression data of genes studies on the CTchip. The data of all the studied genes belonging to the A-D category, as well as some of the data of the genes belonging to the two other categories are indicated (28 genes E and 14 genes F-H).

CTchip_CT = testis or placenta specific genes, which are also found expressed in Hela cells and/or lung tumthe. Genes found as CT genes are indicated in bold text.
All the other columns have been described above.

The results obtained from the CTchip indicate that a large majority of the genes analyzed on this first version of the CT chip display the expected profile of expression in normal somatic tissues. These results therefore validate the in silico approach to identify a large number of new testis- or placenta- specific genes (see figure 2b).
These results also validate the specificity of the above described approach, which relies on the concept that the use of this large number of genes enables the detection of a cancer-related gene deregulation in any case of somatic cancer (fig 1a, also refer to fig 2a describing the Oncomine data).
To enforce the data, it is to be noticed that most of the control genes with an expected expression in all tissues were positive in all tissues in the experiment.

A large proportion of the tissue-specific genes showed a rather tissue-restricted pattern of expression, although some of them did not have this restricted pattern of expression. It should be reminded that these genes were selected for a restricted expression pattern according to symatlas transcriptomic data, but were not classified according to the specificities of their ESTs. Therefore these non-specific expression profiles were to be expected.
In particular, a high proportion of the testis-specific and placenta-specific genes (as defined previously, belonging to specificity classes A to D) clearly show a testis- or placenta-restricted pattern of expression in the normal tissues (Figure 2a, third column, and figure 2b).

Among the testis- or placenta-specific genes, nine were found expressed in Hela cells and/or the lung tumor sample analyzed in this series of experiments (see table 6), showing that they can be considered as "CT" genes, which could be deregulated in somatic cancers. The present results demonstrate that this CT-chip approach can indeed detect the deregulation of testis- or placenta-specific genes in somatic cancers and identify a set of deregulated CT genes in somatic cancers.

Altogether the data obtained with this first CT-chip enable to validate the in silico approaches for the identification of testis- or placenta- specific genes and confirm most of the initial data, and in particular the expression specificity of the genes.
These results also demonstrate that this tool can be extremely powerful to systematically identify genes aberrantly expressed in all tumthes, and be used to define any cancer type and stage of evolution (see Oncomine data).

These results therefore validate the concept that at least one of testis and placenta specific genes can be found abnormally expressed in cancer cells of at least one type of the somatic or ovarian cancers, and that each type of somatic or ovarian cancer cells can abnormally express at least one of the testis and placenta specific genes.

### References

Bock-Axelsen J, Lotem J, Sachs L, Domany E (2007) Genes overexpressed in different human solid cancers exhibit different tissue-specific expression profiles. Proc Natl Acad Sci U S A
Chalmel F, Rolland AD, Niederhauser-Wiederkehr C, Chung SS, Demougin P, Gattiker A, Moore J, Patard JJ, Wolgemuth DJ, Jegou B, Primig M (2007) The conserved transcriptome in human and rodent male gametogenesis. Proc Natl Acad Sci USA
Chen YT, Scanlan MJ, Venditti CA, Chua R, Theiler G, Stevenson BJ, Iseli C, Gure AO, Vasicek T, Strausberg RL, Jongeneel CV, Old LJ, Simpson AJ (2005) Identification of cancer/testis-antigen genes by massively parallel signature sequencing. Proc Natl Acad Sci U S A 102: 7940-5
Costa FF, Le Blanc K, Brodin B (2007) Concise review: cancer/testis antigens, stem cells, and cancer. Stem Cells 25: 707-11
Fox MS, Ares VX, Turek PJ, Haqq C, Reijo Pera RA (2003) Feasibility of global gene expression analysis in testicular biopsies from infertile men. Mol Reprod Dev 66: 403-21
Kalejs M, Erenpreisa J (2005) Cancer/testis antigens and gametogenesis: a review and "brain-storming" session. Cancer Cell Int 5: 4
Meklat F, Li Z, Wang Z, Zhang Y, Zhang J, Jewell A, Lim SH (2007) Cancer-testis antigens in haematological malignancies. Br J Haematol 136: 769-76
Rhodes DR, Kalyana-Sundaram S, Mahavisno V, Varambally R, Yu J, Briggs BB, Barrette TR, Anstet MJ, Kincead-Beal C, Kulkarni P, Varambally S, Ghosh D, Chinnaiyan AM (2007) Oncomine 3.0: genes, pathways, and networks in a collection of 18,000 cancer gene expression profiles. Neoplasia 9: 166-80
Rhodes DR, Yu J, Shanker K, Deshpande N, Varambally R, Ghosh D, Barrette T, Pandey A, Chinnaiyan AM (2004) ONCOMINE: a cancer microarray database and integrated data-mining platform. Neoplasia 6: 1-6
Scanlan MJ, Gordon CM, Williamson B, Lee SY, Chen YT, Stockert E, Jungbluth A, Ritter G, Jager D, Jager E, Knuth A, Old LJ (2002) Identification of cancer/testis genes by database mining and mRNA expression analysis. Int J Cancer 98: 485-92
Scanlan MJ, Simpson AJ, Old LJ (2004) The cancer/testis genes: review, standardization, and commentary. Cancer Immun 4: 1
Schultz N, Hamra FK, Garbers DL (2003) A multitude of genes expressed solely in meiotic or postmeiotic spermatogenic cells offers a myriad of contraceptive targets. Proc Natl Acad Sci U S A 100: 12201-6
Simpson AJ, Caballero OL, Jungbluth A, Chen YT, Old LJ (2005) Cancer/testis antigens, gametogenesis and cancer. Nat Rev Cancer 5: 615-25
Su AI, Wiltshire T, Batalov S, Lapp H, Ching KA, Block D, Zhang J, Soden R, Hayakawa M, Kreiman G, Cooke MP, Walker JR, Hogenesch JB (2004) A gene atlas of the mouse and human protein-encoding transcriptomes. Proc Natl Acad Sci USA
Weber M, Hellmann I, Stadler MB, Ramos L, Paabo S, Rebhan M, Schubeler D (2007) Distribution, silencing potential and evolutionary impact of promoter DNA methylation in the human genome. Nat Genet

### Example 3

### Second and more stringent analysis of tissue specificity, update of the literature, epigenetic data of TSPS genes and results of the second version of the CT chip.

The strategy for the identification of CT genes is outlined in **figure 3****.**

The inventors undertook a work to investigate the question of the impact of epigenetic regulation in gene expression and the occurrence of systematic epigenetic mis-regulation in cancers, and made the hypothesis that a reliable global identification of genes, whose expression is strictly restricted to testis or placenta, is the unique condition for a large-scale identification of "Cancer Testis" (CT) genes, and would give us the power to systematically detect any somatic cancer.

The first attempt to find a list of testis and placenta specific genes, published or unpublished but publicly available, showed that they largely include genes with non-restricted patterns of expression or even ubiquitous genes.

According to the hypothesis, this non-restrictive expression disqualifies these genes as cancer markers for somatic cancers.

This non-restrictive tissue-specificity of the known CT genes, has also very recently been confirmed and the possibility that some CT genes could be expressed in somatic tissues in non-cancer cells under particular physiological or pathological circumstances, has not been investigated.

The Inventors have therefore decided to establish their own list of testis- and placenta-specific genes and defined criteria for the selection of strict specificity of their expression

Combining large-scale online and home made transcriptomic approaches (See previous exemples), the inventors identified genes, whose expression is strictly restricted to testis or placenta. The inventors then confronted these expression data with several sets of pangenomic epigenetic data as additional criteria for the selection of the genes of interest.

Strikingly, these analyses consistently demonstrated the presence of specific epigenetic marks associated with the silencing of the selected genes in somatic cell, therefore increasing the reliability of the selection.

They designed a dedicated microarray containing sequences representative of an exhaustive list of strictly specific testis (TS) and placenta (PS) genes. This microarray not only allowed to finely tune the list of genes and propose a final list of genes for which the strict specificity of expression for testis and placenta was confirmed, but also detected the illegitimate expression of at least one of these genes in all 20 samples representative of a variety of somatic and ovarian cancer types.
In order to test for the aberrant expression of the list of TS/PS genes in a wide range of somatic cancer types and subtypes, the Inventors then analysed cancer transcriptomic data available online (using the Oncomine website). This approach demonstrated that most testis-or placenta- specific genes of the list are sporadically expressed in one or more somatic cancer. Moreover, it demonstrated that most, if not all, cases of cancer are associated with the aberrant expression of at least one of these testis- or placenta- specific genes.

The strategy for the identification of CT genes is outlined in **figure 3****.**

### 1- Identification of genes with an expression pattern restricted to testis or placenta

Although lists of testis-specific genes have been established for several species, including mouse (http://www.germonline.org/Multi/martview), until recently none was yet available for human genes. The prior art methods previously used did not allow sorting the genes according to their strict expression in testis.

### Large-scale screening for testis-specific (TS) and placenta-specific (PS) genes from expression data available online (Figure 4)

In order to establish a list of genes with specific patterns of expression in normal tissues the inventors took advantage of :
i/ transcriptomic data (SymAtlas transcriptomic data published by the Genomics Institute of the Novartis Research Foundation ("GNF"), obtained from designed custom arrays, which interrogate the expression of most protein-encoding human and mouse genes in a panel of 79 human and 61 mouse tissues (http://symatlas.gnf.org/SymAtlas/) and
ii/ EST data (ncbi website: http://www.ncbi.nlm.nih.gov/entrez/query.fcgi?CMD=search&DB= unigene) available online.

Using Symatlas transcriptomic data, the inventors selected genes with a mean expression in male germinal cells (samples "testis seminiferous tubules" or "testis germ cells") or in placenta, which was at least five times the mean expression in non-germinal normal tissues. In parallel, in an independent approach, the inventors considered the frequency of EST found in each tissue library. In this case, a ratio, R, was calculated with the number of EST found in tissues other than testis or placenta divided by the total number of EST, representing the proportion of non-specific EST.

During the initial screening, the inventors selected all genes for which more than half of the total number of EST were found in placenta or testis (ratio R<0.5). A ratio R=0 indicated a gene with strict specificity of expression restricted to testis or placenta (TSPSa genes).

Combining transcriptomic and EST data led to the identification of **1154** human genes or sequences over-expressed in testis (n= **990**) or placenta (n=**164**). A close analysis of the EST data allowed the classification of these genes according to their specificity, as follow.
When ESTs were found exclusively in placenta or testis (R=0, not considering the EST found in brain or nervous system) the gene was classified as testis- or placenta- specific (in silico specificity class: TSPSa). Of the initial selection of overexpressed genes, **443** had a pattern of expression, which was restricted to testis (TS genes, n=**388**) or placenta (PS genes, n=**55**), whereas 711 genes were classified as highly expressed, but not exclusively, in testis (TE genes, n=602) or placenta (PE genes, n=109).
The overexpressed genes were sub-classified according to their pattern of expression according to the EST and symatlas data and the following class are defined:
TSPSa = genes with a pattern of expression restricted to testis or placenta (ESTs exclusively found in placenta or testis, R=0)
TSPSb = genes highly overexpressed in testis or placenta (less than 30% of ESTs found in tissues other than testis or placenta or brain)
TSPSc = genes overexpressed in testis or placenta (between 30% and 50% of ESTs found in tissues other than testis or placenta or brain)
TSPSd = genes overexpressed in testis or placenta but also expressed in other tissues (more than 50% of ESTs were found in tissues other than testis or placenta or brain; symatlas data showed that the expression in testis or placenta is five times or more the mean expression in all tissues).

### Epigenetic status of TSPS genes in somatic cells (figure 5)

In order to include additional criteria for the selection of the genes, the Inventors decided to take into account the gene "epigenetic signatures" which could be "unearthed" from now available genome-wide epigenetic data in several normal somatic cell types was undertaken. The inventors took advantage of recently published large-scale epigenetic studies of the human genome. In the first study from Schubeler's team (Weber et al. 2007 Apr;39(4):457-66. ), DNA methylation, RNA polymerase occupancy and histone modifications were measured at 16,000 promoters in primary somatic cells and spermatozoa. In a second work, Young and collaborators (Guenther et al. 2007 Cell 130: 77-88) published a genome-wide analysis of three types human cells, Embryonic Stem (ES) cells, liver cells and reticulocytes, looking for several histone modifications, including H3K4me3 and H3K9,14Ac as well as H3K36me3 and RNA polymerase II occupancy.

The data corresponding to the lists of testis and placenta expressed genes were extracted from these studies. The characteristics of the genes with a restricted expression pattern (TSPSa) were compared with those of the genes overexpressed in testis and placenta (TEPEb = high overexpression in testis or placenta, with sporadic expression in other tissues, and TEPEcd = overexpressed in testis or placenta, but also found expressed in many other tissues), as well as with the patterns observed for other human genes, found widely expressed according to the study (not shown), or described above (Fig. 5).

A meta-analysis of the promoter classes and DNA methylation data obtained from the study of Schubeler's team (Weber et al. 2007 Apr;39(4):457-66.) (http://www.fmi.ch/members/dirk .schubeler/supplemental.htm) corresponding to the genes, which the inventors had listed as testis- or placenta- specific, shows here that the genes bear epigenetic characteristics of germline specific genes. Indeed, compared to the promoters of the majority of human genes, the inventors observe that TSPSa genes promoters are more often CpG poor (half are CpG poor (LCP), and half are CpG intermediate or CpG rich promoters (HICP)). Moreover, in fibroblasts, in contrast to most other human genes promoters, TSPSa genes promoters are hypermethylated and not enriched in H3K4me2 or polymerase II. Most TSPS genes were consistently depleted in histone H3K4me3, acH3K14K9, and in DNA pole, in these different somatic cell types, whereas the authors describe that most human genes, independent of their expression status, are enriched in H3K4me3 and in polIIe, and many are also associated with acH3K9K14.

The same analysis performed with the genes, which the Inventors found overexpressed in testis or placenta but not strictly restricted to these tissues, show that their types of promoters, patterns of DNA methylation and histone modifications could be correlated with the levels of specificity defined according to the EST data (fig 5). Indeed, the "germline gene specific" epigenetic characteristics (CpG poor promoters, depleted in polymerase and histone modifications, and hypermethylated CpG-rich promoters) were also found in a high proportion of TEPEb genes (overexpressed with less than 30% of EST in tissues other than testis or placenta), whereas TEPEcd genes showed a distribution close to that described for most human genes (a majority of hypomethylated CpG-rich promoters).

Altogether, this analysis of pangenomic epigenetic data reveals that a large proportion of PS and TS genes bear "germline gene specific" epigenetic marks in their promoter region, different from those characterizing most human genes, in several undifferentiated (ES cells) or differentiated somatic cells (fibroblasts, reticulocytes, T lymphocytes). Moreover the study demonstrates that this specific epigenetic configuration can be directly correlated with the strict expression specificity of these genes, indicating an active and strong repressive state in all lineages of normal somatic cells.

Following this analysis, among TEPEb genes (genes highly overexpressed in testis or placenta according to the in silico data, with more than 70% testis or placenta ESTs), the genes selected for their strict specificity of expression on the macroarray, and associated with "germline specific" epigenetic marks, according to these data (see below, and fig 4I).

### Design of a second dedicated "CT" macroarray and expression analysis of testis and placenta genes in normal and no- cancerous tissues (figure 4)

These genes and sequences identified in silico were then used to design a dedicated microarray in order to assess their expression in a wide range of normal human tissues, including testis (2 samples), placenta, breast (2 samples), bladder, colon (2 samples), liver, lung, prostate, pancreas, ovary, lymph nodes, resting B lymphocytes from blood and spleen.

Moreover, in order to assess the potential deregulation of these TS and PS genes in non-cancerous situations, The inventors also assessed their expression during physiological processes such as lymphocyte activation or inflammatory lymph nodes, or non-cancerous pathological conditions, including Crohn's disease (2 samples), liver cirrhosis (2 samples), lung with chronic bronchitis, pancreatitis, hyperplasic or inflammatory prostate.
The microarray contains the polynucleotides probes SEQ ID NO 415 to 2989.

The methodological approaches for the macroarray design and hybridization, as well as the signal analysis and statistics, are described above (Example 2).

The expression analysis in normal tissue showed a strict specificity of expression in testis or placenta for approximately half of the TSPSa genes identified from existing expression data. (n=**220**, SPE-spe genes) including 208 testis- and 12 placenta- specific genes. These experimental data therefore confirmed that these genes are strictly expressed in testis or placenta and repressed in somatic tissues under normal and non-cancerous conditions (Fig4 d and e).
Among the other TSPSa genes, 105 genes showed positive signal(s) in one or more of the somatic tissues analysed, suggesting that either their expression is not strictly testis- or placenta- specific, or that the oligonucleotide(s) selected for the transcriptomic analysis produced a non-specific hybridization signal. Another 118 genes or sequences did not show any signal in testis or placenta, either because they were not expressed in testis or placenta, or because the probes were not chosen appropriately.

Among the TEPEb genes, 135 showed non-specific expression in normal or non-cancerous somatic tissues and 42 did not show any hybridization signal in testis or placenta.However, 134 displayed a restricted pattern of expression in testis (n=124) or placenta (n=10) (Fig **4** d and e). The inventors had a close look at the promoter CpG content and methylation in fibroblasts (using data from Weber et al. 2007), as well as the histone modifications in ES cells (data from Guenther et al. 2007). For **55** of these genes, the inventors had clear evidence for "germline gene specific" epigenetic marks (fig **4**I), including 17 genes with a CpG-rich promoter hypermethylated in fibroblasts and 38 genes which, in ES cells, liver cells and reticulocytes, consistently combined a lack of histone modifications H3K4 me3 and H3K9/14ac, with the absence of polymerase (initiation and elongation complex).

Altogether, taking into all the above analyses of normal and non-cancerous tissues, as well as epigenetic features, the inventors identified a total of **275** genes with strong evidence for a pattern of expression strictly restricted to testis or placenta, in the absence of cancer, which were therefore good CT candidates.

### 2- Expression of CT gene candidates in somatic and ovarian cancers

The **275** genes identified as strictly testis- or placenta-specific (respectively TS and PS genes) were examined in search for their illegitimate expression in somatic cancer cells.

The data obtained from the small series of various cancerous samples analysed on the dedicated macroarray suggested that some of the genes of the list were sporadically de-repressed in somatic cancers. Indeed, the analysis of 13 solid tumthe samples (including bladder, breast, colon, lung, ovary, pancreas, prostate tumthes) and 7 haematological cancer samples (lymphoma and leukaemia samples) on the dedicated macroarray showed that at least one gene of the list is expressed in each sample. Fthety fthe of the genes were found illegitimately expressed in at least one cancer sample (Fig 4f, Fig 6b, c, d).

In order to have a large overview of the expression of TSPS genes in a wide range of somatic cancers, the inventors took advantage of the cancer profiling database Oncomine (http://www.oncomine.org/main/index.jsp) (Rhodes et al. 2007; Rhodes et al. 2004), which combines data from more than 20,000 cancer transcriptome profiles with an analysis engine and web application for data mining and visualization. In oncomine, the expression profile of each gene is compared between two groups of samples and box plots and a p value are calculated from this comparison. For each of the 275 testis-specific and placenta-specific genes listed above, the inventors searched the Oncomine database for an overexpression in studies comparing tumthe versus normal tissue samples. The inventors selected the analyses recorded in Oncomine, which compared normal samples with somatic cancer samples of various origins and selected those where at least 30 genes of the list were analysed (as well as the few studies where less than 30 of the genes were analysed but in which one gene at least was overexpressed in the tumor samples with a highly significant p value, p<0.001). This approach led to the selection of **68 studies.** The p value corresponding to each of the genes in each study was recorded.

Nb of genes overexpressed in at least one oncomine study (with most significant p values) and/or in at least one cancer sample of the second microarray:

From this meta-analysis, **93** of the testis- or placenta- specific genes were found over-expressed in at least one oncomine study with p<0.001, and another **120** genes were over-expressed in at least one oncomine study with 0.001<p< 0.05. Twenty-two genes were never found overexpressed in any of the selected oncomine studies, and 40 were never tested in any of these studies.
Among the 62 genes, which had not been analysed (or found overexpressed) in any of the oncomine studies, **9** were found expressed in at least one of the cancer samples analysed on the macroarray.
These data are described in figure **7****.**

Hence, this analysis of available transcriptomic data (for the genes of the list for which data were available) showed that most genes of the list (**n= 222**) are potentially illegitimately expressed in one or several somatic - or ovarian - cancers, and therefore qualify for being "Cancer Testis" (CT) genes. A detailed analysis of several individual cancer samples shows that at least one of the testis- or placenta- specific genes is aberrantly expressed in each given case of cancer (not shown). This is also confirmed by the results of the dedicated microarray, since in each of the 20 cancer samples analysed, at least one gene of the list was found illegitimately expressed.

### 3- Epigenetic mechanisms involved in TSPS gene repression in normal somatic cells and potential deregulation in cancer

The results show that a large proportion of genes with a restricted expression profile, and of TS genes in particular, displays a unique pattern of epigenetic features, observed in all somatic cell types, undifferentiated as well as differentiated, which are very rarely observed for other human genes.

In many cancers, aberrant DNA methylation patterns have been demonstrated to contribute to cell transformation and cancer progression. For instance, aberrant methylation of the CpG rich promoter of tumthe suppressor genes has led to the aberrant repression of these genes, suggesting that this epigenetic aberration could be a direct contribution to the cancerous phenotype. Conversely an abnormal demethylation of repetitive non-coding regions of the genome has been shown in several cancers, including colon cancer.

The inventors have thus clearly defined a list of 222 genes that are deregulated in somatic and ovarian cancer.
They also have proposed 10 groups, based on the epigenetic status of the promoters of the genes belonging to said groups.
This list of 222 genes, and the groups allows to specifically and efficiently to detect each type of cancer, with a characteristic which is that at least one of theses genes of the groups is deregulated in at least one tumors, and each tumor present a deregulation of at least one gene of the groups.

## Claims

1. Use of at least one set of nucleic acid molecules chosen among:
• a set comprising at least 26 nucleic acid molecules, said 26 nucleic acid molecules being represented by the nucleic acid sequences SEQ ID NO : 1, 3, 5, 7, 9, 11, 13, 15, 17, 19, 21, 23, 25, 27, 29, 31, 33, 35, 37, 39, 41, 43, 45, 47, 49 and 51,
• a set comprising at least 26 complementary nucleic acid molecules of said at least 26 nucleic acid molecules,
for the *in vitro* or *ex vivo* diagnosis of any type of somatic or ovarian cancers, said somatic cancers being solid tumors or hematological neoplasms,
wherein:
- cancer cells of each type of somatic or ovarian cancers abnormally express at least one nucleic acid molecule of the above sets of nucleic acid molecules, and
- at least one of nucleic acid molecule of the above sets of nucleic acid molecules is abnormally expressed in cancer cells of at least one type of somatic or ovarian cancers.

2. Use of at least one set of nucleic acid molecules according to claim 1,
wherein said set of nucleic acid molecules comprises at least 59 nucleic acid molecules, said at least 59 nucleic acid molecules being represented by the nucleic acid sequences SEQ ID NO : 1, 3, 5, 7, 9, 11, 13, 15, 17, 19, 21, 23, 25, 27, 29, 31, 33, 35, 37, 39, 41, 43, 45, 47, 49, 51, 53, 55, 57, 59, 61, 63, 65, 67, 69, 71, 73, 75, 77, 79, 81, 83, 85, 87, 89, 91, 93, 95, 97, 99, 101, 103, 105, 107, 109, 111, 113, 385 and 386,
optionally, wherein said set of nucleic acid molecules comprises at least 93 nucleic acid molecules, said at least 93 nucleic acid molecules being represented by the nucleic acid sequences SEQ ID NO : 1, 3, 5, 7, 9, 11, 13, 15, 17, 19, 21, 23, 25, 27, 29, 31, 33, 35, 37, 39, 41, 43, 45, 47, 49, 51, 53, 55, 57, 59, 61, 63, 65, 67, 69, 71, 73, 75, 77, 79, 81, 83, 85, 87, 89, 91, 93, 95, 97, 99, 101, 103, 105, 107, 109, 111, 113, 385, 386, 115, 117, 119, 121, 123, 125, 127, 129, 131, 133, 135, 137, 139, 141, 143, 145, 147, 149, 151, 153, 155, 157, 159, 161, 163, 165, 167, 169, 171, 173, 175, 387, 388 and 389,
optionally, wherein said set of nucleic acid molecules comprises at least 108 nucleic acid molecules, said at least 108 nucleic acid molecules being represented by the nucleic acid sequences SEQ ID NO : 1, 3, 5, 7, 9, 11, 13, 15, 17, 19, 21, 23, 25, 27, 29, 31, 33, 35, 37, 39, 41, 43, 45, 47, 49, 51, 53, 55, 57, 59, 61, 63, 65, 67, 69, 71, 73, 75, 77, 79, 81, 83, 85, 87, 89, 91, 93, 95, 97, 99, 101, 103, 105, 107, 109, 111, 113, 385, 386, 115, 117, 119, 121, 123, 125, 127, 129, 131, 133, 135, 137, 139, 141, 143, 145, 147, 149, 151, 153, 155, 157, 159, 161, 163, 165, 167, 169, 171, 173, 175, 387, 388, 389, 177, 179, 181, 183, 185, 187, 189, 191, 193, 195, 197, 199, 201, 203 and 205,
optionally, wherein said set of nucleic acid molecules comprises at least 128 nucleic acid molecules, said at least 128 nucleic acid molecules being represented by the nucleic acid sequences SEQ ID NO : 1, 3, 5, 7, 9, 11, 13, 15, 17, 19, 21, 23, 25, 27, 29, 31, 33, 35, 37, 39, 41, 43, 45, 47, 49, 51, 53, 55, 57, 59, 61, 63, 65, 67, 69, 71, 73, 75, 77, 79, 81, 83, 85, 87, 89, 91, 93, 95, 97, 99, 101, 103, 105, 107, 109, 111, 113, 385, 386, 115, 117, 119, 121, 123, 125, 127, 129, 131, 133, 135, 137, 139, 141, 143, 145, 147, 149, 151, 153, 155, 157, 159, 161, 163, 165, 167, 169, 171, 173, 175, 387, 388, 389, 177, 179, 181, 183, 185, 187, 189, 191, 193, 195, 197, 199, 201, 203, 205, 207, 209, 211, 213, 215, 217, 219, 221, 223, 225, 227, 229, 231, 233, 235, 237, 239, 241, 390 and 391,
optionally, wherein said set of nucleic acid molecules comprises at least 160 nucleic acid molecules, said at least 160 nucleic acid molecules being represented by the nucleic acid sequences SEQ ID NO : 1, 3, 5, 7, 9, 11, 13, 15, 17, 19, 21, 23, 25, 27, 29, 31, 33, 35, 37, 39, 41, 43, 45, 47, 49, 51, 53, 55, 57, 59, 61, 63, 65, 67, 69, 71, 73, 75, 77, 79, 81, 83, 85, 87, 89, 91, 93, 95, 97, 99, 101, 103, 105, 107, 109, 111, 113, 385, 386, 115, 117, 119, 121, 123, 125, 127, 129, 131, 133, 135, 137, 139, 141, 143, 145, 147, 149, 151, 153, 155, 157, 159, 161, 163, 165, 167, 169, 171, 173, 175, 387, 388, 389, 177, 179, 181, 183, 185, 187, 189, 191, 193, 195, 197, 199, 201, 203, 205, 207, 209, 211, 213, 215, 217, 219, 221, 223, 225, 227, 229, 231, 233, 235, 237, 239, 241, 390, 391, 243, 245, 247, 249, 251, 253, 255, 257, 259, 261, 263, 265, 267, 269, 271, 273, 275, 277, 279, 281, 283, 285, 287, 392, 393, 394, 395, 396, 397, 398, 399 and 400,
optionally, wherein said set of nucleic acid molecules comprises at least 166 nucleic acid molecules being represented by the nucleic acid sequences SEQ ID NO : 1, 3, 5, 7, 9, 11, 13, 15, 17, 19, 21, 23, 25, 27, 29, 31, 33, 35, 37, 39, 41, 43, 45, 47, 49, 51, 53, 55, 57, 59, 61, 63, 65, 67, 69, 71, 73, 75, 77, 79, 81, 83, 85, 87, 89, 91, 93, 95, 97, 99, 101, 103, 105, 107, 109, 111, 113, 385, 386, 115, 117, 119, 121, 123, 125, 127, 129, 131, 133, 135, 137, 139, 141, 143, 145, 147, 149, 151, 153, 155, 157, 159, 161, 163, 165, 167, 169, 171, 173, 175, 387, 388, 389, 177, 179, 181, 183, 185, 187, 189, 191, 193, 195, 197, 199, 201, 203, 205, 207, 209, 211, 213, 215, 217, 219, 221, 223, 225, 227, 229, 231, 233, 235, 237, 239, 241, 390, 391, 243, 245, 247, 249, 251, 253, 255, 257, 259, 261, 263, 265, 267, 269, 271, 273, 275, 277, 279, 281, 283, 285, 287, 392, 393, 394, 395, 396, 397, 398, 399, 400, 289, 291, 293, 295, 297 and 299,
optionally, wherein said set of nucleic acid molecules comprises at least 179 nucleic acid molecules, said at least 179 nucleic acid molecules being represented by the nucleic acid sequences SEQ ID NO : 1, 3, 5, 7, 9, 11, 13, 15, 17, 19, 21, 23, 25, 27, 29, 31, 33, 35, 37, 39, 41, 43, 45, 47, 49, 51, 53, 55, 57, 59, 61, 63, 65, 67, 69, 71, 73, 75, 77, 79, 81, 83, 85, 87, 89, 91, 93, 95, 97, 99, 101, 103, 105, 107, 109, 111, 113, 385, 386, 115, 117, 119, 121, 123, 125, 127, 129, 131, 133, 135, 137, 139, 141, 143, 145, 147, 149, 151, 153, 155, 157, 159, 161, 163, 165, 167, 169, 171, 173, 175, 387, 388, 389, 177, 179, 181, 183, 185, 187, 189, 191, 193, 195, 197, 199, 201, 203, 205, 207, 209, 211, 213, 215, 217, 219, 221, 223, 225, 227, 229, 231, 233, 235, 237, 239, 241, 390, 391, 243, 245, 247, 249, 251, 253, 255, 257, 259, 261, 263, 265, 267, 269, 271, 273, 275, 277, 279, 281, 283, 285, 287, 392, 393, 394, 395, 396, 397, 398, 399, 400, 289, 291, 293, 295, 297, 299, 301, 303, 305, 307, 309, 311, 313, 315, 317, 319, 321, 323 and 325,
optionally, wherein said set of nucleic acid molecules comprises at least 213 nucleic acid molecules, said at least 213 nucleic acid molecules being represented by the nucleic acid sequences SEQ ID NO : 1, 3, 5, 7, 9, 11, 13, 15, 17, 19, 21, 23, 25, 27, 29, 31, 33, 35, 37, 39, 41, 43, 45, 47, 49, 51, 53, 55, 57, 59, 61, 63, 65, 67, 69, 71, 73, 75, 77, 79, 81, 83, 85, 87, 89, 91, 93, 95, 97, 99, 101, 103, 105, 107, 109, 111, 113, 385, 386, 115, 117, 119, 121, 123, 125, 127, 129, 131, 133, 135, 137, 139, 141, 143, 145, 147, 149, 151, 153, 155, 157, 159, 161, 163, 165, 167, 169, 171, 173, 175, 387, 388, 389, 177, 179, 181, 183, 185, 187, 189, 191, 193, 195, 197, 199, 201, 203, 205, 207, 209, 211, 213, 215, 217, 219, 221, 223, 225, 227, 229, 231, 233, 235, 237, 239, 241, 390, 391, 243, 245, 247, 249, 251, 253, 255, 257, 259, 261, 263, 265, 267, 269, 271, 273, 275, 277, 279, 281, 283, 285, 287, 392, 393, 394, 395, 396, 397, 398, 399, 400, 289, 291, 293, 295, 297, 299, 301, 303, 305, 307, 309, 311, 313, 315, 317, 319, 321, 323, 325, 327, 329, 331, 333, 335, 337, 339, 341, 343, 345, 347, 349, 351, 353, 355, 357, 359, 361, 363, 365, 367, 369, 371, 401, 402, 403, 404, 405, 406, 407, 408, 409, 410 and 411,
optionally, wherein said set of nucleic acid molecules comprises 222 nucleic acid molecules, said at least 222 nucleic acid molecules being represented by the nucleic acid sequences SEQ ID NO : 1, 3, 5, 7, 9, 11, 13, 15, 17, 19, 21, 23, 25, 27, 29, 31, 33, 35, 37, 39, 41, 43, 45, 47, 49, 51, 53, 55, 57, 59, 61, 63, 65, 67, 69, 71, 73, 75, 77, 79, 81, 83, 85, 87, 89, 91, 93, 95, 97, 99, 101, 103, 105, 107, 109, 111, 113, 385, 386, 115, 117, 119, 121, 123, 125, 127, 129, 131, 133, 135, 137, 139, 141, 143, 145, 147, 149, 151, 153, 155, 157, 159, 161, 163, 165, 167, 169, 171, 173, 175, 387, 388, 389, 177, 179, 181, 183, 185, 187, 189, 191, 193, 195, 197, 199, 201, 203, 205, 207, 209, 211, 213, 215, 217, 219, 221, 223, 225, 227, 229, 231, 233, 235, 237, 239, 241, 390, 391, 243, 245, 247, 249, 251, 253, 255, 257, 259, 261, 263, 265, 267, 269, 271, 273, 275, 277, 279, 281, 283, 285, 287, 392, 393, 394, 395, 396, 397, 398, 399, 400, 289, 291, 293, 295, 297, 299, 301, 303, 305, 307, 309, 311, 313, 315, 317, 319, 321, 323, 325, 327, 329, 331, 333, 335, 337, 339, 341, 343, 345, 347, 349, 351, 353, 355, 357, 359, 361, 363, 365, 367, 369, 371, 401, 402, 403, 404, 405, 406, 407, 408, 409, 410, 411, 373, 375, 377, 379, 381, 383, 412, 413 and 414.

3. Use of at least one set of amino acid molecules chosen among:
o a set comprising at least 26 proteins,
o a set comprising at least one fragment of each of
said at least 26 proteins, said fragment being able to be recognized by an antibody specifically directed against a protein from which said fragment derives,
said at least 26 proteins being coded by at least 26 nucleic acid molecules according to claim 1, and said at least 26 proteins being represented by the amino acid sequences SEQ ID NO : 2, 4, 6, 8, 10, 12, 14, 16, 18, 20, 22, 24, 26, 28, 30, 32, 34, 36, 38, 40, 42, 44, 46, 48, 50 and 52,
each amino acid molecule contained in a given set above-defined being specifically recognized by at least one specific antibody,
for the *in vitro* or *ex vivo* diagnosis of any type of somatic or ovarian cancers, said somatic cancers being solid tumors or hematological neoplasms,
wherein:
- a biological sample of a patient afflicted by any type of somatic or ovarian cancer presents an abnormal amount of at least one antibody that specifically recognizes an amino acid molecule of the above set of amino acid molecules, and
- at least one antibody that specifically recognizes an amino acid molecule of the above sets of amino acid molecules is present in an abnormal amount in a biological sample of a patient afflicted by at least one type of somatic or ovarian cancer.

4. Use of at least a set of amino acid molecules according to claim 3,
wherein said set of proteins comprises at least 57 proteins, said at least 57 proteins being represented by the amino acid sequences SEQ ID NO : 2, 4, 6, 8, 10, 12, 14, 16, 18, 20, 22, 24, 26, 28, 30, 32, 34, 36, 38, 40, 42, 44, 46, 48, 50, 52, 54, 56, 58, 60, 62, 64, 66, 68, 70, 72, 74, 76, 78, 80, 82, 84, 86, 88, 90, 92, 94, 96, 98, 100, 102, 104, 106, 108, 110, 112 and 114,
optionally, wherein said set of proteins comprises at least 88 proteins, said at least 88 proteins being represented by the amino acid sequences SEQ ID NO : 2, 4, 6, 8, 10, 12, 14, 16, 18, 20, 22, 24, 26, 28, 30, 32, 34, 36, 38, 40, 42, 44, 46, 48, 50, 52, 54, 56, 58, 60, 62, 64, 66, 68, 70, 72, 74, 76, 78, 80, 82, 84, 86, 88, 90, 92, 94, 96, 98, 100, 102, 104, 106, 108, 110, 112, 114, 116, 118, 120, 122, 124, 126, 128, 130, 132, 134, 136, 138, 140, 142, 144, 146, 148, 150, 152, 154, 156, 158, 160, 162, 164, 166, 168, 170, 172, 174 and 176,
optionally, wherein said set of proteins comprises at least 103 proteins, said at least 103 proteins being represented by the amino acid sequences SEQ ID NO : 2, 4, 6, 8, 10, 12, 14, 16, 18, 20, 22, 24, 26, 28, 30, 32, 34, 36, 38, 40, 42, 44, 46, 48, 50, 52, 54, 56, 58, 60, 62, 64, 66, 68, 70, 72, 74, 76, 78, 80, 82, 84, 86, 88, 90, 92, 94, 96, 98, 100, 102, 104, 106, 108, 110, 112, 114, 116, 118, 120, 122, 124, 126, 128, 130, 132, 134, 136, 138, 140, 142, 144, 146, 148, 150, 152, 154, 156, 158, 160, 162, 164, 166, 168, 170, 172, 174, 176, 178, 180, 182, 184, 186, 188, 190, 192, 194, 196, 198, 200, 202, 204 and 206,
optionally, wherein said set of proteins comprises at least 121 proteins, said at least 121 proteins being represented by the amino acid sequences SEQ ID NO : 2, 4, 6, 8, 10, 12, 14, 16, 18, 20, 22, 24, 26, 28, 30, 32, 34, 36, 38, 40, 42, 44, 46, 48, 50, 52, 54, 56, 58, 60, 62, 64, 66, 68, 70, 72, 74, 76, 78, 80, 82, 84, 86, 88, 90, 92, 94, 96, 98, 100, 102, 104, 106, 108, 110, 112, 114, 116, 118, 120, 122, 124, 126, 128, 130, 132, 134, 136, 138, 140, 142, 144, 146, 148, 150, 152, 154, 156, 158, 160, 162, 164, 166, 168, 170, 172, 174, 176, 178, 180, 182, 184, 186, 188, 190, 192, 194, 196, 198, 200, 202, 204, 206, 208, 210, 212, 214, 216, 218, 220, 222, 224, 226, 228, 230, 232, 234, 236, 238, 240 and 242,
optionally, wherein said set of proteins comprises at least 144 proteins, said at least 144 proteins being represented by the amino acid sequences SEQ ID NO : 2, 4, 6, 8, 10, 12, 14, 16, 18, 20, 22, 24, 26, 28, 30, 32, 34, 36, 38, 40, 42, 44, 46, 48, 50, 52, 54, 56, 58, 60, 62, 64, 66, 68, 70, 72, 74, 76, 78, 80, 82, 84, 86, 88, 90, 92, 94, 96, 98, 100, 102, 104, 106, 108, 110, 112, 114, 116, 118, 120, 122, 124, 126, 128, 130, 132, 134, 136, 138, 140, 142, 144, 146, 148, 150, 152, 154, 156, 158, 160, 162, 164, 166, 168, 170, 172, 174, 176, 178, 180, 182, 184, 186, 188, 190, 192, 194, 196, 198, 200, 202, 204, 206, 208, 210, 212, 214, 216, 218, 220, 222, 224, 226, 228, 230, 232, 234, 236, 238, 240, 242, 244, 246, 248, 250, 252, 254, 256, 258, 260, 262, 264, 266, 268, 270, 272, 274, 276, 278, 280, 282, 284, 286 and 288,
optionally, wherein said set of proteins comprises at least 150 proteins, said at least 150 proteins being represented by the amino acid sequences SEQ ID NO : 2, 4, 6, 8, 10, 12, 14, 16, 18, 20, 22, 24, 26, 28, 30, 32, 34, 36, 38, 40, 42, 44, 46, 48, 50, 52, 54, 56, 58, 60, 62, 64, 66, 68, 70, 72, 74, 76, 78, 80, 82, 84, 86, 88, 90, 92, 94, 96, 98, 100, 102, 104, 106, 108, 110, 112, 114, 116, 118, 120, 122, 124, 126, 128, 130, 132, 134, 136, 138, 140, 142, 144, 146, 148, 150, 152, 154, 156, 158, 160, 162, 164, 166, 168, 170, 172, 174, 176, 178, 180, 182, 184, 186, 188, 190, 192, 194, 196, 198, 200, 202, 204, 206, 208, 210, 212, 214, 216, 218, 220, 222, 224, 226, 228, 230, 232, 234, 236, 238, 240, 242, 244, 246, 248, 250, 252, 254, 256, 258, 260, 262, 264, 266, 268, 270, 272, 274, 276, 278, 280, 282, 284, 286, 288, 290, 292, 294, 296, 298 and 300,
optionally, wherein said set of proteins comprises at least 163 proteins, said at least 163 proteins being represented by the amino acid sequences SEQ ID NO : 2, 4, 6, 8, 10, 12, 14, 16, 18, 20, 22, 24, 26, 28, 30, 32, 34, 36, 38, 40, 42, 44, 46, 48, 50, 52, 54, 56, 58, 60, 62, 64, 66, 68, 70, 72, 74, 76, 78, 80, 82, 84, 86, 88, 90, 92, 94, 96, 98, 100, 102, 104, 106, 108, 110, 112, 114, 116, 118, 120, 122, 124, 126, 128, 130, 132, 134, 136, 138, 140, 142, 144, 146, 148, 150, 152, 154, 156, 158, 160, 162, 164, 166, 168, 170, 172, 174, 176, 178, 180, 182, 184, 186, 188, 190, 192, 194, 196, 198, 200, 202, 204, 206, 208, 210, 212, 214, 216, 218, 220, 222, 224, 226, 228, 230, 232, 234, 236, 238, 240, 242, 244, 246, 248, 250, 252, 254, 256, 258, 260, 262, 264, 266, 268, 270, 272, 274, 276, 278, 280, 282, 284, 286, 288, 290, 292, 294, 296, 298, 300, 302, 304, 306, 308, 310, 312, 314, 316, 318, 320, 322, 324 and 326,
optionally, wherein said set of proteins comprises at least 186 proteins, said at least 186 proteins being represented by the amino acid sequences SEQ ID NO : 2, 4, 6, 8, 10, 12, 14, 16, 18, 20, 22, 24, 26, 28, 30, 32, 34, 36, 38, 40, 42, 44, 46, 48, 50, 52, 54, 56, 58, 60, 62, 64, 66, 68, 70, 72, 74, 76, 78, 80, 82, 84, 86, 88, 90, 92, 94, 96, 98, 100, 102, 104, 106, 108, 110, 112, 114, 116, 118, 120, 122, 124, 126, 128, 130, 132, 134, 136, 138, 140, 142, 144, 146, 148, 150, 152, 154, 156, 158, 160, 162, 164, 166, 168, 170, 172, 174, 176, 178, 180, 182, 184, 186, 188, 190, 192, 194, 196, 198, 200, 202, 204, 206, 208, 210, 212, 214, 216, 218, 220, 222, 224, 226, 228, 230, 232, 234, 236, 238, 240, 242, 244, 246, 248, 250, 252, 254, 256, 258, 260, 262, 264, 266, 268, 270, 272, 274, 276, 278, 280, 282, 284, 286, 288, 290, 292, 294, 296, 298, 300, 302, 304, 306, 308, 310, 312, 314, 316, 318, 320, 322, 324, 326, 328, 330, 332, 334, 336, 338, 340, 342, 344, 346, 348, 350, 352, 354, 356, 358, 360, 362, 364, 366, 368, 370 and 372,
optionally, wherein said set of proteins comprises 192 proteins, said proteins being represented by the amino acid sequences SEQ ID NO : 2, 4, 6, 8, 10, 12, 14, 16, 18, 20, 22, 24, 26, 28, 30, 32, 34, 36, 38, 40, 42, 44, 46, 48, 50, 52, 54, 56, 58, 60, 62, 64, 66, 68, 70, 72, 74, 76, 78, 80, 82, 84, 86, 88, 90, 92, 94, 96, 98, 100, 102, 104, 106, 108, 110, 112, 114, 116, 118, 120, 122, 124, 126, 128, 130, 132, 134, 136, 138, 140, 142, 144, 146, 148, 150, 152, 154, 156, 158, 160, 162, 164, 166, 168, 170, 172, 174, 176, 178, 180, 182, 184, 186, 188, 190, 192, 194, 196, 198, 200, 202, 204, 206, 208, 210, 212, 214, 216, 218, 220, 222, 224, 226, 228, 230, 232, 234, 236, 238, 240, 242, 244, 246, 248, 250, 252, 254, 256, 258, 260, 262, 264, 266, 268, 270, 272, 274, 276, 278, 280, 282, 284, 286, 288, 290, 292, 294, 296, 298, 300, 302, 304, 306, 308, 310, 312, 314, 316, 318, 320, 322, 324, 326, 328, 330, 332, 334, 336, 338, 340, 342, 344, 346, 348, 350, 352, 354, 356, 358, 360, 362, 364, 366, 368, 370, 372, 374, 376, 378, 380, 382 and 384.

5. Use of a set of at least 26 antibodies, optionally a set of 57 antibodies, optionally a set of 88 antibodies, optionally a set of 103 antibodies, optionally a set of 121 antibodies, optionally a set of 150 antibodies, optionally a set of 163 antibodies, optionally a set of 186 antibodies, optionally a set of 192 antibodies **characterized in that** each antibody of a given mentioned set of antibodies specifically recognizes an amino acid molecule of a set of amino acid molecules as defined in claim 3 or 4, and each amino acid molecules of a given set of amino acid molecules as defined in claims 3 or 4 is specifically recognized by an antibody of said given set of antibodies,
for the *in vitro* or *ex vivo* diagnosis of any type of somatic or ovarian cancers,
wherein:
- cancer cells of each type of somatic or ovarian cancer abnormally express at least one amino acid molecule recognized by an antibody of the above sets of antibodies, and
- at least one of amino acid molecule recognized by an antibody of the above sets of antibodies is abnormally expressed in cancer cells of at least one type of somatic or ovarian cancers.

6. Microarray comprising at least 32 oligonucleotide probes represented by the oligonucleotide sequences SEQ ID NO 415 to 446, each of said at least 32 oligonucleotide probes specifically hybridizing with one nucleic acid molecule of at least a set of nucleic acid molecules according to claim 1, the correspondence between oligonucleotide probes and their corresponding nucleic acid sequence being represented in Table 3a.

7. Microarray according to claim 6, comprising at least 70 oligonucleotide probes represented by the oligonucleotide sequences SEQ ID NO 415 to 484, each of said at least 70 oligonucleotide probes specifically hybridizing with one nucleic acid molecule of at least 59 nucleic acid molecules as defined in claim 2,
optionally, comprising at least 110 oligonucleotide probes represented by the oligonucleotide sequences SEQ ID NO 415 to 524, each of said at least 110 oligonucleotide probes specifically hybridizing with one nucleic acid molecule of at least 93 nucleic acid molecules as defined in claim 2,
optionally, comprising at least 130 oligonucleotide probes represented by the oligonucleotide sequences SEQ ID NO 415 to 544, each of said at least 130 oligonucleotide probes specifically hybridizing with one nucleic acid molecule of at least 108 nucleic acid molecules as defined in claim 2,
optionally, comprising at least 154 oligonucleotide probes represented by the oligonucleotide sequences SEQ ID NO 415 to 568, each of said at least 154 oligonucleotide probes specifically hybridizing with one nucleic acid molecule of at least 128 nucleic acid molecules as defined in claim 2,
optionally, comprising at least 197 oligonucleotide probes represented by the oligonucleotide sequences SEQ ID NO 415 to 611, each of said at least 197 oligonucleotide probes specifically hybridizing with one nucleic acid molecule of at least 160 nucleic acid molecules as defined in claim 2,
optionally, comprising at least 204 oligonucleotide probes represented by the oligonucleotide sequences SEQ ID NO 415 to 618, each of said at least 204 oligonucleotide probes specifically hybridizing with one nucleic acid molecule of at least 166 nucleic acid molecules as defined in claim 2,
optionally, comprising at least 220 oligonucleotide probes represented by the oligonucleotide sequences SEQ ID NO 415 to 634, each of said at least 220 oligonucleotide probes specifically hybridizing with one nucleic acid molecule of at least 179 nucleic acid molecules as defined in claim 2,
optionally, comprising at least 261 oligonucleotide probes represented by the oligonucleotide sequences SEQ ID NO 415 to 675, each of said at least 261 oligonucleotide probes specifically hybridizing with one nucleic acid molecule of at least 213 nucleic acid molecules as defined in claim 2,
optionally, comprising at least 270 oligonucleotide probes represented by the oligonucleotide sequences SEQ ID NO 415 to 684, each of said at least 270 oligonucleotide probes specifically hybridizing with one nucleic acid molecule of the 222 nucleic acid molecules of the collection of 222 nucleic acid molecules as defined in claim 2,
the correspondence between oligonucleotide probes and their corresponding gene being represented in Table 3b,
said microarray possibly comprising positive and negative oligonucleotide probes specifically hybridizing with positive and negative control nucleic acid molecules.

8. Microarray according to claim 6 or 7, comprising the oligonucleotide probes represented by the oligonucleotide sequences SEQ ID NO 415 to SEQ ID NO 684, optionally comprising the oligonucleotide probes represented by the oligonucleotide sequences SEQ ID NO 415 to SEQ ID NO 1617, optionally comprising or consisting in the oligonucleotide probes represented by the oligonucleotide sequences SEQ ID NO 415 to SEQ ID NO 2989.

9. Method for the *in vitro* and/or *ex vivo* somatic or ovarian cancer diagnosis in a subject, by determining, among nucleic acids from a biological sample from the subject, the presence or the variation of the amount of at least one nucleic acid molecule of a group of at least 26 nucleic acid molecules as defined in claim 1,
said presence or variation of the amount of said nucleic acid molecule being assessed with respect to the absence or the given amount of said nucleic acid molecule from a sample isolated from a healthy subject, comprising:
- contacting nucleic acids from the biological sample with an agent to allow the formation of at least one nucleic acid complex between said agent and said at least one nucleic acid molecule from the biological sample of the subject,
• said agent comprising at least:
∘ said nucleic acid molecule,
∘ a complementary molecule of said nucleic acid molecule,
∘ a fragment of said nucleic acid molecule, or
∘ a fragment of said complementary molecule,
of each of said at least 26 nucleic acid molecules, and
• the nucleic acid molecule, the complementary molecule of said nucleic acid molecule, or the fragments thereof, contained in said agent being able to selectively hybridize with said at least 26 nucleic acid molecules,
- determining the presence or the variation of amount of at least one nucleic acid complex indicating the fact that the subject is afflicted with cancer.

10. Method according to claim 9, wherein said agent contains nucleic acid sequences that allow a PCR amplification of a fragment of at least one nucleic acid molecule of said at least 26 nucleic acid molecules,
said PCR amplification being preferably reverse transcription-quantitative PCR, or PCR array.

11. Method according to claim 9, comprising
- contacting nucleic acids from the biological sample with an agent, said agent being a microarray such as defined in any of claims 6 to 8, to allow the formation of at least one nucleic acid complex, between said agent and at least one nucleic acid from a sample of a subject,
- determining the presence or the variation of the amount of at least one nucleic acid complex indicating the fact that the subject is afflicted with cancer.

12. Method for the *in vitro* and/or *ex vivo* cancer diagnosis in a subject, by determining the presence or the variation of amount of at least one protein, or a fragment thereof, of a group of at least 26 proteins as defined in claim 3, in the biological sample from the subject,
said presence or variation of amount of said protein being assessed with respect to the absence or the given amount of said protein in a sample isolated from a healthy subject, comprising:
- contacting proteins from the biological sample with an agent to allow the formation of at least one immune complex between said agent and said at least one protein from the biological sample of the subject,
said agent consisting of at least one antibody specifically hybridizing with one protein of each of said at least 26 proteins,
- determining the presence or the variation of amount of said at least one immune complex indicating the fact that the subject is afflicted by cancer, said immune complex being determined preferably by immunohistochemistry, immunocytochemistry, immunofluorescence, western blotting and immunoprecipitation.

13. Method for the *in vitro* and/or *ex vivo* cancer diagnosis in a subject, by determining the presence or the variation of amount of at least one antibody among a group of at least 26 antibodies as defined in claim 5 that specifically recognizes at least 26 proteins as defined in claim 3, in a biological sample from the subject,
said presence or variation of amount of said antibody being assessed with respect to the absence or the given amount of said antibody in a sample isolated from a healthy subject, comprising:
- contacting the biological sample from the subject with an agent, to allow the formation of at least one immune complex between said agent and at least one antibody from the biological sample of the subject,
said agent consisting of at least 26 proteins that are able to specifically hybridize with said at least 26 antibodies,
- determining the presence or the variation of amount of said at least one immune complex indicating the fact that the subject is afflicted by cancer, said immune complex being determined preferably by immunohistochemistry, immunocytochemistry, immunofluorescence, western blotting and immunoprecipitation.

14. Kit for the *in vitro* and/or *ex vivo* cancer diagnosis comprising:
- a microarray such as defined in anyone of claims 6,
- optionally material for preparation of nucleic acids of the biological sample from a patient suspected to be afflicted by cancer, in particular the preparation of cDNAs,
- optionally labelled molecules for labelling said nucleic acids,
- optionally a negative control corresponding to nucleic acids from a biological sample from a healthy subject.

15. Kit for the *in vitro* and/or *ex vivo* cancer diagnosis comprising:
- ELISA support consisting of at least 26 proteins as defined in claim 3, or fragments thereof,
- optionally labelled antibodies directed against antibodies that recognizes specifically said proteins, said protein being liable to be present in a sample from a patient suspected to be afflicted by cancer,
- optionally a negative control corresponding to antibodies from a sample from a healthy subject.

16. Kit for the *in vitro* and/or *ex vivo* cancer diagnosis comprising:
- ELISA support consisting of at least 26 antibodies that specifically recognize at least 26 proteins as defined in claim 3, or fragments thereof,
- optionally labelled antibody directed against a protein specifically recognized by said antibody, said antibody being liable to be present in a sample from a patient suspected to be afflicted by cancer,
- optionally a negative control corresponding to polypeptides from a sample from a healthy subject.

## Patentansprüche

1. Verwendung mindestens eines Satzes Nukleinsäuremoleküle, ausgewählt aus
• einem Satz, umfassend mindestens 26 Nukleinsäuremoleküle, wobei die 26 Nukleinsäuremoleküle dargestellt sind durch die Nukleinsäuresequenzen SEQ ID NR. : 1, 3, 5, 7, 9, 11, 13, 15, 17, 19, 21, 23, 25, 27, 29, 31, 33, 35, 37, 39, 41, 43, 45, 47, 49 und 51,
• einem Satz, umfassend mindestens 26 komplementäre Nukleinsäuremoleküle der mindestens 26 Nukleinsäuremoleküle
für die In-vitro- oder Ex-vivo-Diagnose jeder Art somatischer Krebserkrankung oder Eierstockkrebs, wobei die somatischen Krebserkrankungen solide Tumoren oder hämatologische Neoplasmen sind,
wobei:
- Krebszellen von jeder Art somatischer Krebserkrankung oder von Eierstockkrebs mindestens ein Nukleinsäuremolekül der obigen Sätze Nukleinsäuremoleküle abnormal exprimieren und
- mindestens eines der Nukleinsäuremoleküle der obigen Sätze Nukleinsäuremoleküle in Krebszellen von mindestens einer Art somatischer Krebserkrankung oder Eierstockkrebs abnormal exprimiert wird.

2. Verwendung mindestens eines Satzes Nukleinsäuremoleküle nach Anspruch 1,
wobei der Satz Nukleinsäuremoleküle mindestens 59 Nukleinsäuremoleküle umfasst, wobei die mindestens 59 Nukleinsäuremoleküle dargestellt sind durch die Nukleinsäuresequenzen SEQ ID NR.: 1, 3, 5, 7, 9, 11, 13, 15, 17, 19, 21, 23, 25, 27, 29, 31, 33, 35, 37, 39, 41, 43, 45, 47, 49, 51, 53, 55, 57, 59, 61, 63, 65, 67, 69, 71, 73, 75, 77, 79, 81, 83, 85, 87, 89, 91, 93, 95, 97, 99, 101, 103, 105, 107, 109, 111, 113, 385 und 386,
wobei der Satz Nukleinsäuremoleküle fakultativ mindestens 93 Nukleinsäuremoleküle umfasst, wobei die mindestens 93 Nukleinsäuremoleküle dargestellt sind durch die Nukleinsäuresequenzen SEQ ID NR.: 1, 3, 5, 7, 9, 11, 13, 15, 17, 19, 21, 23, 25, 27, 29, 31, 33, 35, 37, 39, 41, 43, 45, 47, 49, 51, 53, 55, 57, 59, 61, 63, 65, 67, 69, 71, 73, 75, 77, 79, 81, 83, 85, 87, 89, 91, 93, 95, 97, 99, 101, 103, 105, 107, 109, 111, 113, 385, 386, 115, 117, 119, 121, 123, 125, 127, 129, 131, 133, 135, 137, 139, 141, 143, 145, 147, 149, 151, 153, 155, 157, 159, 161, 163, 165, 167, 169, 171, 173, 175, 387, 388 und 389,
wobei der Satz Nukleinsäuremoleküle fakultativ mindestens 128 Nukleinsäuremoleküle umfasst, wobei die mindestens 128 Nukleinsäuremoleküle dargestellt sind durch die Nukleinsäuresequenzen SEQ ID NR.: 1, 3, 5, 7, 9, 11, 13, 15, 17, 19, 21, 23, 25, 27, 29, 31, 33, 35, 37, 39, 41, 43, 45, 47, 49, 51, 53, 55, 57, 59, 61, 63, 65, 67, 69, 71, 73, 75, 77, 79, 81, 83, 85, 87, 89, 91, 93, 95, 97, 99, 101, 103, 105, 107, 109, 111, 113, 385, 386, 115, 117, 119, 121, 123, 125, 127, 129, 131, 133, 135, 137, 139, 141, 143, 145, 147, 149, 151, 153, 155, 157, 159, 161, 163, 165, 167, 169, 171, 173, 175, 387, 388, 389, 177, 179, 181, 183, 185, 187, 189, 191, 193, 195, 197, 199, 201, 203 und 205,
wobei der Satz Nukleinsäuremoleküle fakultativ mindestens 128 Nukleinsäuremoleküle umfasst, wobei die mindestens 128 Nukleinsäuremoleküle dargestellt sind durch die Nukleinsäuresequenzen SEQ ID NR.: 1, 3, 5, 7, 9, 11, 13, 15, 17, 19, 21, 23, 25, 27, 29, 31, 33, 35, 37, 39, 41, 43, 45, 47, 49, 51, 53, 55, 57, 59, 61, 63, 65, 67, 69, 71, 73, 75, 77, 79, 81, 83, 85, 87, 89, 91, 93, 95, 97, 99, 101, 103, 105, 107, 109, 111, 113, 385, 386, 115, 117, 119, 121, 123, 125, 127, 129, 131, 133, 135, 137, 139, 141, 143, 145, 147, 149, 151, 153, 155, 157, 159, 161, 163, 165, 167, 169, 171, 173, 175, 387, 388, 389, 177, 179, 181, 183, 185, 187, 189, 191, 193, 195, 197, 199, 201, 203, 205, 207, 209, 211, 213, 215, 217, 219, 221, 223, 225, 227, 229, 231, 233, 235, 237, 239, 241, 390 und 391,
wobei der Satz Nukleinsäuremoleküle fakultativ mindestens 160 Nukleinsäuremoleküle umfasst, wobei die mindestens 160 Nukleinsäuremoleküle dargestellt sind durch die Nukleinsäuresequenzen SEQ ID NR.: 1, 3, 5, 7, 9, 11, 13, 15, 17, 19, 21, 23, 25, 27, 29, 31, 33, 35, 37, 39, 41, 43, 45, 47, 49, 51, 53, 55, 57, 59, 61, 63, 65, 67, 69, 71, 73, 75, 77, 79, 81, 83, 85, 87, 89, 91, 93, 95, 97, 99, 101, 103, 105, 107, 109, 111, 113, 385, 386, 115, 117, 119, 121, 123, 125, 127, 129, 131, 133, 135, 137, 139, 141, 143, 145, 147, 149, 151, 153, 155, 157, 159, 161, 163, 165, 167, 169, 171, 173, 175, 387, 388, 389, 177, 179, 181, 183, 185, 187, 189, 191, 193, 195, 197, 199, 201, 203, 205, 207, 209, 211, 213, 215, 217, 219, 221, 223, 225, 227, 229, 231, 233, 235, 237, 239, 241, 390, 391, 243, 245, 247, 249, 251, 253, 255, 257, 259, 261, 263, 265, 267, 269, 271, 273, 275, 277, 279, 281, 283, 285, 287, 392, 393, 394, 395, 396, 397, 398, 399 und 400,
wobei der Satz Nukleinsäuremoleküle fakultativ mindestens 166 Nukleinsäuremoleküle umfasst, die dargestellt sind durch die Nukleinsäuresequenzen SEQ ID NR.: 1, 3, 5, 7, 9, 11, 13, 15, 17, 19, 21, 23, 25, 27, 29, 31, 33, 35, 37, 39, 41, 43, 45, 47, 49, 51, 53, 55, 57, 59, 61, 63, 65, 67, 69, 71, 73, 75, 77, 79, 81, 83, 85, 87, 89, 91, 93, 95, 97, 99, 101, 103, 105, 107, 109, 111, 113, 385, 386, 115, 117, 119, 121, 123, 125, 127, 129, 131, 133, 135, 137, 139, 141, 143, 145, 147, 149, 151, 153, 155, 157, 159, 161, 163, 165, 167, 169, 171, 173, 175, 387, 388, 389, 177, 179, 181, 183, 185, 187, 189, 191, 193, 195, 197, 199, 201, 203, 205, 207, 209, 211, 213, 215, 217, 219, 221, 223, 225, 227, 229, 231, 233, 235, 237, 239, 241, 390, 391, 243, 245, 247, 249, 251, 253, 255, 257, 259, 261, 263, 265, 267, 269, 271, 273, 275, 277, 279, 281, 283, 285, 287, 392, 393, 394, 395, 396, 397, 398, 399, 400, 289, 291, 293, 295, 297 und 299,
wobei der Satz Nukleinsäuremoleküle fakultativ mindestens 179 Nukleinsäuremoleküle umfasst, wobei die mindestens 179 Nukleinsäuremoleküle dargestellt sind durch die Nukleinsäuresequenzen SEQ ID NR.: 1, 3, 5, 7, 9, 11, 13, 15, 17, 19, 21, 23, 25, 27, 29, 31, 33, 35, 37, 39, 41, 43, 45, 47, 49, 51, 53, 55, 57, 59, 61, 63, 65, 67, 69, 71, 73, 75, 77, 79, 81, 83, 85, 87, 89, 91, 93, 95, 97, 99, 101, 103, 105, 107, 109, 111, 113, 385, 386, 115, 117, 119, 121, 123, 125, 127, 129, 131, 133, 135, 137, 139, 141, 143, 145, 147, 149, 151, 153, 155, 157, 159, 161, 163, 165, 167, 169, 171, 173, 175, 387, 388, 389, 177, 179, 181, 183, 185, 187, 189, 191, 193, 195, 197, 199, 201, 203, 205, 207, 209, 211, 213, 215, 217, 219, 221, 223, 225, 227; 229, 231, 233, 235, 237, 239, 241, 390, 391, 243, 245, 247, 249, 251, 253, 255, 257, 259, 261, 263, 265, 267, 269, 271, 273, 275, 277, 279, 281, 283, 285, 287, 392, 393, 394, 395, 396, 397, 398, 399, 400, 289, 291, 293, 295, 297, 299, 301, 303, 305, 307, 309, 311, 313, 315, 317, 319, 321, 323 und 325,
wobei der Satz Nukleinsäuremoleküle fakultativ mindestens 213 Nukleinsäuremoleküle umfasst, wobei die mindestens 213 Nukleinsäuremoleküle dargestellt sind durch die Nukleinsäuresequenzen SEQ ID NR.: 1, 3, 5, 7, 9, 11, 13, 15, 17, 19, 21, 23, 25, 27, 29, 31, 33, 35, 37, 39, 41, 43, 45, 47, 49, 51, 53, 55, 57, 59, 61, 63, 65, 67, 69, 71, 73, 75, 77, 79, 81, 83, 85, 87, 89, 91, 93, 95, 97, 99, 101, 103, 105, 107, 109, 111, 113, 385, 386, 115, 117, 119, 121, 123, 125, 127, 129, 131, 133, 135, 137, 139, 141, 143, 145, 147, 149, 151, 153, 155, 157, 159, 161, 163, 165, 167, 169, 171, 173, 175, 387, 388, 389, 177, 179, 181, 183, 185, 187, 189, 191, 193, 195, 197, 199, 201, 203, 205, 207, 209, 211, 213, 215, 217, 219, 221, 223, 225, 227, 229, 231, 233, 235, 237, 239, 241, 390, 391, 243, 245, 247, 249, 251, 253, 255, 257, 259, 261, 263, 265, 267, 269, 271, 273, 275, 277, 279, 281, 283, 285, 287, 392, 393, 394, 395, 396, 397, 398, 399, 400, 289, 291, 293, 295, 297, 299, 301, 303, 305, 307, 309, 311, 313, 315, 317, 319, 321, 323, 325, 327, 329, 331, 333, 335, 337, 339, 341, 343, 345, 347, 349, 351, 353, 355, 357, 359, 361, 363, 365, 367, 369, 371, 401, 402, 403, 404, 405, 406, 407, 408, 409, 410 und 411,
wobei der Satz Nukleinsäuremoleküle fakultativ 222 Nukleinsäuremoleküle umfasst, wobei die mindestens 222 Nukleinsäuremoleküle dargestellt sind durch die Nukleinsäuresequenzen SEQ ID NR.: 1, 3, 5, 7, 9, 11, 13, 15, 17, 19, 21, 23, 25, 27, 29, 31, 33, 35, 37, 39, 41, 43, 45, 47, 49, 51, 53, 55, 57, 59, 61, 63, 65, 67, 69, 71, 73, 75, 77, 79, 81, 83, 85, 87, 89, 91, 93, 95, 97, 99, 101, 103, 105, 107, 109, 111, 113, 385, 386, 115, 117, 119, 121, 123, 125, 127, 129, 131, 133, 135, 137, 139, 141, 143, 145, 147, 149, 151, 153, 155, 157, 159, 161, 163, 165, 167, 169, 171, 173, 175, 387, 388, 389, 177, 179, 181, 183, 185, 187, 189, 191, 193, 195, 197, 199, 201, 203, 205, 207, 209, 211, 213, 215, 217, 219, 221, 223, 225, 227, 229, 231, 233, 235, 237, 239, 241, 390, 391, 243, 245, 247, 249, 251, 253, 255, 257, 259, 261, 263, 265, 267, 269, 271, 273, 275, 277, 279, 281, 283, 285, 287, 392, 393, 394, 395, 396, 397, 398, 399, 400, 289, 291, 293, 295, 297, 299, 301, 303, 305, 307, 309, 311, 313, 315, 317, 319, 321, 323, 325, 327, 329, 331, 333, 335, 337, 339, 341, 343, 345, 347, 349, 351, 353, 355, 357, 359, 361, 363, 365, 367, 369, 371, 401, 402, 403, 404, 405, 406, 407, 408, 409, 410, 411, 373, 375, 377, 379, 381, 383, 412, 413 und 414.

3. Verwendung mindestens eines Satzes Aminosäuremoleküle, ausgewählt aus
∘ einem Satz, umfassend mindestens 26 Proteine,
∘ einem Satz, umfassend mindestens ein Fragment jedes der mindestens 26 Proteine, wobei das Fragment einen Antikörper erkennen kann, der spezifisch gegen ein Protein gerichtet ist, von dem das Fragment abgeleitet ist,
wobei die mindestens 26 Proteine von mindestens 26 Nukleinsäuremolekülen nach Anspruch 1 codiert werden und wobei die mindestens 26 Proteine dargestellt sind durch die Aminosäuresequenzen SEQ ID NR.: 2, 4, 6, 8, 10, 12, 14, 16, 18, 20, 22, 24, 26, 28, 30, 32, 34, 36, 38, 40, 42, 44, 46, 48, 50 und 52,
wobei jedes in einem vorstehend definierten Satz enthaltene Aminosäuremolekül spezifisch von mindestens einem bestimmten Antikörper erkannt wird,
für die In-vitro- oder Ex-vivo-Diagnose jeder Art somatischer Krebserkrankung oder Eierstockkrebs, wobei die somatischen Krebserkrankungen solide Tumoren oder hämatologische Neoplasmen sind,
wobei:
- eine biologische Probe eines an jeder Art von somatischer Krebserkrankung oder Eierstockkrebs leidenden Patienten eine abnormale Menge mindestens eines Antikörpers präsentiert, der spezifisch ein Aminosäuremolekül des vorstehenden Satzes Aminosäuremoleküle erkennt, und
- mindestens ein Antikörper spezifisch das Vorhandensein eines Aminosäuremoleküls des vorstehenden Satzes Aminosäuremoleküle in einer abnormalen Menge in einer biologischen Probe eines an mindestens einer Art von somatischer Krebserkrankung oder Eierstockkrebs leidenden Patienten erkennt.

4. Verwendung mindestens eines Satzes Aminosäuremoleküle nach Anspruch 3,
wobei der Satz Proteine mindestens 57 Proteine umfasst, wobei die mindestens 57 Proteine dargestellt sind durch die Aminosäuresequenzen SEQ ID NR.: 2, 4, 6, 8, 10, 12, 14, 16, 18, 20, 22, 24, 26, 28, 30, 32, 34, 36, 38, 40, 42, 44, 46, 48, 50, 52, 54, 56, 58, 60, 62, 64, 66, 68, 70, 72, 74, 76, 78, 80, 82, 84, 86, 88, 90, 92, 94, 96, 98, 100, 102, 104, 106, 108, 110, 112 und 114,
wobei der Satz Proteine fakultativ mindestens 88 Proteine umfasst, wobei die mindestens 88 Proteine dargestellt sind durch die Aminosäuresequenzen SEQ ID NR.: 2, 4, 6, 8, 10, 12, 14, 16, 18, 20, 22, 24, 26, 28, 30, 32, 34, 36, 38, 40, 42, 44, 46, 48, 50, 52, 54, 56, 58, 60, 62, 64, 66, 68, 70, 72, 74, 76, 78, 80, 82, 84, 86, 88, 90, 92, 94, 96, 98, 100, 102, 104, 106, 108, 110, 112, 114, 116, 118, 120, 122, 124, 126, 128, 130, 132, 134, 136, 138, 140, 142, 144, 146, 148, 150, 152, 154, 156, 158, 160, 162, 164, 166, 168, 170, 172, 174 und 176,
wobei der Satz Proteine fakultativ mindestens 103 Proteine umfasst, wobei die mindestens 103 Proteine dargestellt sind durch die Aminosäuresequenzen SEQ ID NR.: 2, 4, 6, 8, 10, 12, 14, 16, 18, 20, 22, 24, 26, 28, 30, 32, 34, 36, 38, 40, 42, 44, 46, 48, 50, 52, 54, 56, 58, 60, 62, 64, 66, 68, 70, 72, 74, 76, 78, 80, 82, 84, 86, 88, 90, 92, 94, 96, 98, 100, 102, 104, 106, 108, 110, 112, 114, 116, 118, 120, 122, 124, 126, 128, 130, 132, 134, 136, 138, 140, 142, 144, 146, 148, 150, 152, 154, 156, 158, 160, 162, 164, 166, 168, 170, 172, 174, 176, 178, 180, 182, 184, 186, 188, 190, 192, 194, 196, 198, 200, 202, 204 und 206,
wobei der Satz Proteine fakultativ mindestens 121 Proteine umfasst, wobei die mindestens 121 Proteine dargestellt sind durch die Aminosäuresequenzen SEQ ID NR.: 2, 4, 6, 8, 10, 12, 14, 16, 18, 20, 22, 24, 26, 28, 30, 32, 34, 36, 38, 40, 42, 44, 46, 48, 50, 52, 54, 56, 58, 60, 62, 64, 66, 68, 70, 72, 74, 76, 78, 80, 82, 84, 86, 88, 90, 92, 94, 96, 98, 100, 102, 104, 106, 108, 110, 112, 114, 116, 118, 120, 122, 124, 126, 128, 130, 132, 134, 136, 138, 140, 142, 144, 146, 148, 150, 152, 154, 156, 158, 160, 162, 164, 166, 168, 170, 172, 174, 176, 178, 180, 182, 184, 186, 188, 190, 192, 194, 196, 198, 200, 202, 204, 206, 208, 210, 212, 214, 216, 218, 220, 222, 224, 226, 228, 230, 232, 234, 236, 238, 240 und 242,
wobei der Satz Proteine fakultativ mindestens 144 Proteine umfasst, wobei die mindestens 144 Proteine dargestellt sind durch die Aminosäuresequenzen SEQ ID NR.: 2, 4, 6, 8, 10, 12, 14, 16, 18, 20, 22, 24, 26, 28, 30, 32, 34, 36, 38, 40, 42, 44, 46, 48, 50, 52, 54, 56, 58, 60, 62, 64, 66, 68, 70, 72, 74, 76, 78, 80, 82, 84, 86, 88, 90, 92, 94, 96, 98, 100, 102, 104, 106, 108, 110, 112, 114, 116, 118, 120, 122, 124, 126, 128, 130, 132, 134, 136, 138, 140, 142, 144, 146, 148, 150, 152, 154, 156, 158, 160, 162, 164, 166, 168, 170, 172, 174, 176, 178, 180, 182, 184, 186, 188, 190, 192, 194, 196, 198, 200, 202, 204, 206, 208, 210, 212, 214, 216, 218, 220, 222, 224, 226, 228, 230, 232, 234, 236, 238, 240, 242, 244, 246, 248, 250, 252, 254, 256, 258, 260, 262, 264, 266, 268, 270, 272, 274, 276, 278, 280, 282, 284, 286 und 288,
wobei der Satz Proteine fakultativ mindestens 150 Proteine umfasst, wobei die mindestens 150 Proteine dargestellt sind durch die Aminosäuresequenzen SEQ ID NR.: 2, 4, 6, 8, 10, 12, 14, 16, 18, 20, 22, 24, 26, 28, 30, 32, 34, 36, 38, 40, 42, 44, 46, 48, 50, 52, 54, 56, 58, 60, 62, 64, 66, 68, 70, 72, 74, 76, 78, 80, 82, 84, 86, 88, 90, 92, 94, 96, 98, 100, 102, 104, 106, 108, 110, 112, 114, 116, 118, 120, 122, 124, 126, 128, 130, 132, 134, 136, 138, 140, 142, 144, 146, 148, 150, 152, 154, 156, 158, 160, 162, 164, 166, 168, 170, 172, 174, 176, 178, 180, 182, 184, 186, 188, 190, 192, 194, 196, 198, 200, 202, 204, 206, 208, 210, 212, 214, 216, 218, 220, 222, 224, 226, 228, 230, 232, 234, 236, 238, 240, 242, 244, 246, 248, 250, 252, 254, 256, 258, 260, 262, 264, 266, 268, 270, 272, 274, 276, 278, 280, 282, 284, 286, 288, 290, 292, 294, 296, 298 und 300, wobei der Satz Proteine fakultativ mindestens 163 Proteine umfasst, wobei die mindestens 163 Proteine dargestellt sind durch die Aminosäuresequenzen SEQ ID NR.: 2, 4, 6, 8, 10, 12, 14, 16, 18, 20, 22, 24, 26, 28, 30, 32, 34, 36, 38, 40, 42, 44, 46, 48, 50, 52, 54, 56, 58, 60, 62, 64, 66, 68, 70, 72, 74, 76, 78, 80, 82, 84, 86, 88, 90, 92, 94, 96, 98, 100, 102, 104, 106, 108, 110, 112, 114, 116, 118, 120, 122, 124, 126, 128, 130, 132, 134, 136, 138, 140, 142, 144, 146, 148, 150, 152, 154, 156, 158, 160, 162, 164, 166, 168, 170, 172, 174, 176, 178, 180, 182, 184, 186, 188, 190, 192, 194, 196, 198, 200, 202, 204, 206, 208, 210, 212, 214, 216, 218, 220, 222, 224, 226, 228, 230, 232, 234, 236, 238, 240, 242, 244, 246, 248, 250, 252, 254, 256, 258, 260, 262, 264, 266, 268, 270, 272, 274, 276, 278, 280, 282, 284, 286, 288, 290, 292, 294, 296, 298, 300, 302, 304, 306, 308, 310, 312, 314, 316, 318, 320, 322, 324 und 326,
wobei der Satz Proteine fakultativ mindestens 186 Proteine umfasst, wobei die mindestens 186 Proteine dargestellt sind durch die Aminosäuresequenzen SEQ ID NR.: 2, 4, 6, 8, 10, 12, 14, 16, 18, 20, 22, 24, 26, 28, 30, 32, 34, 36, 38, 40, 42, 44, 46, 48, 50, 52, 54, 56, 58, 60, 62, 64, 66, 68, 70, 72, 74, 76, 78, 80, 82, 84, 86, 88, 90, 92, 94, 96, 98, 100, 102, 104, 106, 108, 110, 112, 114, 116, 118, 120, 122, 124, 126, 128, 130, 132, 134, 136, 138, 140, 142, 144, 146, 148, 150, 152, 154, 156, 158, 160, 162, 164, 166, 168, 170, 172, 174, 176, 178, 180, 182, 184, 186, 188, 190, 192, 194, 196, 198, 200, 202, 204, 206, 208, 210, 212, 214, 216, 218, 220, 222, 224, 226, 228, 230, 232, 234, 236, 238, 240, 242, 244, 246, 248, 250, 252, 254, 256, 258, 260, 262, 264, 266, 268, 270, 272, 274, 276, 278, 280, 282, 284, 286, 288, 290, 292, 294, 296, 298, 300, 302, 304, 306, 308, 310, 312, 314, 316, 318, 320, 322, 324, 326, 328, 330, 332, 334, 336, 338, 340, 342, 344, 346, 348, 350, 352, 354, 356, 358, 360, 362, 364, 366, 368, 370 und 372,
wobei der Satz Proteine fakultativ 192 Proteine umfasst, wobei die Proteine dargestellt sind durch die Aminosäuresequenzen SEQ ID NR.: 2, 4, 6, 8, 10, 12, 14, 16, 18, 20, 22, 24, 26, 28, 30, 32, 34, 36, 38, 40, 42, 44, 46, 48, 50, 52, 54, 56, 58, 60, 62, 64, 66, 68, 70, 72, 74, 76, 78, 80, 82, 84, 86, 88, 90, 92, 94, 96, 98, 100, 102, 104, 106, 108, 110, 112, 114, 116, 118, 120, 122, 124, 126, 128, 130, 132, 134, 136, 138, 140, 142, 144, 146, 148, 150, 152, 154, 156, 158, 160, 162, 164, 166, 168, 170, 172, 174, 176, 178, 180, 182, 184, 186, 188, 190, 192, 194, 196, 198, 200, 202, 204, 206, 208, 210, 212, 214, 216, 218, 220, 222, 224, 226, 228, 230, 232, 234, 236, 238, 240, 242, 244, 246, 248, 250, 252, 254, 256, 258, 260, 262, 264, 266, 268, 270, 272, 274, 276, 278, 280, 282, 284, 286, 288, 290, 292, 294, 296, 298, 300, 302, 304, 306, 308, 310, 312, 314, 316, 318, 320, 322, 324, 326, 328, 330, 332, 334, 336, 338, 340, 342, 344, 346, 348, 350, 352, 354, 356, 358, 360, 362, 364, 366, 368, 370, 372, 374, 376, 378, 380, 382 und 384.

5. Verwendung eines Satzes mit mindestens 26 Antikörpern, fakultativ eines Satzes mit 57 Antikörpern, fakultativ eines Satzes mit 88 Antikörpern, fakultativ eines Satzes mit 103 Antikörpern, fakultativ eines Satzes mit 121 Antikörpern, fakultativ eines Satzes mit 150 Antikörpern, fakultativ eines Satzes mit 163 Antikörpern, fakultativ eines Satzes mit 186 Antikörpern, fakultativ eines Satzes mit 192 Antikörpern, **dadurch gekennzeichnet, dass** jeder Antikörper eines bestimmten genannten Satzes Antikörper spezifisch ein Aminosäuremolekül eines Satzes Aminosäuremoleküle nach Anspruch 3 oder 4 erkennt und jedes Aminosäuremolekül eines bestimmten Satzes Aminosäuremoleküle nach Anspruch 3 oder 4 von einem Antikörper des bestimmten Satzes Antikörper spezifisch erkannt wird,
für die In-vitro- oder Ex-vivo-Diagnose jeder Art somatischer Krebserkrankung oder Eierstockkrebs, wobei:
- Krebszellen von jeder Art somatischer Krebserkrankung oder von Eierstockkrebs mindestens ein Aminosäuremolekül, das von einem Antikörper der obigen Sätze Antikörper erkannt wird, abnormal exprimieren und
- mindestens eines der Aminosäuremoleküle, das von einem Antikörper der obigen Sätze Antikörper erkannt wird, in Krebszellen von mindestens einer Art somatischer Krebserkrankung oder Eierstockkrebs abnormal exprimiert wird.

6. Mikroarray, umfassend
mindestens 32 Oligonukleotidsonden, dargestellt durch die Oligonukleotidsequenzen SEQ ID NR. 415 bis 446, wobei jede der mindestens 32 Oligonukleotidsonden spezifisch mit einem Nukleinsäuremolekül mindestens eines Satzes Nukleinsäuremoleküle nach Anspruch 1 hybridisiert, wobei die Entsprechung zwischen Oligonukleotidsonden und ihrer entsprechenden Nukleinsäuresequenz in Tabelle 3a dargestellt ist.

7. Mikroarray nach Anspruch 6, umfassend mindestens 70 Oligonukleotidsonden, dargestellt durch die Oligonukleotidsequenzen SEQ ID NR. 415 bis 484, wobei jede der mindestens 70 Oligonukleotidsonden spezifisch mit einem Nukleinsäuremolekül von mindestens 59 Nukleinsäuremolekülen nach Anspruch 2 hybridisiert,
fakultativ umfassend mindestens 110 Oligonukleotidsonden, dargestellt durch die Oligonukleotidsequenzen SEQ ID NR. 415 bis 524, wobei jede der mindestens 110 Oligonukleotidsonden spezifisch mit einem Nukleinsäuremolekül von mindestens 93 Nukleinsäuremolekülen nach Anspruch 2 hybridisiert,
fakultativ umfassend mindestens 130 Oligonukleotidsonden, dargestellt durch die Oligonukleotidsequenzen SEQ ID NR. 415 bis 544, wobei jede der mindestens 130 Oligonukleotidsonden spezifisch mit einem Nukleinsäuremolekül von mindestens 108 Nukleinsäuremolekülen nach Anspruch 2 hybridisiert,
fakultativ umfassend mindestens 154 Oligonukleotidsonden, dargestellt durch die Oligonukleotidsequenzen SEQ ID NR. 415 bis 568, wobei jede der mindestens 154 Oligonukleotidsonden spezifisch mit einem Nukleinsäuremolekül von mindestens 128 Nukleinsäuremolekülen nach Anspruch 2 hybridisiert,
fakultativ umfassend mindestens 197 Oligonukleotidsonden, dargestellt durch die Oligonukleotidsequenzen SEQ ID NR. 415 bis 611, wobei jede der mindestens 197 Oligonukleotidsonden spezifisch mit einem Nukleinsäuremolekül von mindestens 160 Nukleinsäuremolekülen nach Anspruch 2 hybridisiert,
fakultativ umfassend mindestens 204 Oligonukleotidsonden, dargestellt durch die Oligonukleotidsequenzen SEQ ID NR. 415 bis 618, wobei jede der mindestens 204 Oligonukleotidsonden spezifisch mit einem Nukleinsäuremolekül von mindestens 166 Nukleinsäuremolekülen nach Anspruch 2 hybridisiert,
fakultativ umfassend mindestens 220 Oligonukleotidsonden, dargestellt durch die Oligonukleotidsequenzen SEQ ID NR. 415 bis 634, wobei jede der mindestens 220 Oligonukleotidsonden spezifisch mit einem Nukleinsäuremolekül von mindestens 179 Nukleinsäuremolekülen nach Anspruch 2 hybridisiert,
fakultativ umfassend mindestens 261 Oligonukleotidsonden, dargestellt durch die Oligonukleotidsequenzen SEQ ID NR. 415 bis 675, wobei jede der mindestens 261 Oligonukleotidsonden spezifisch mit einem Nukleinsäuremolekül von mindestens 213 Nukleinsäuremolekülen nach Anspruch 2 hybridisiert,
fakultativ umfassend mindestens 270 Oligonukleotidsonden, dargestellt durch die Oligonukleotidsequenzen SEQ ID NR. 415 bis 684, wobei jede der mindestens 270 Oligonukleotidsonden spezifisch mit einem Nukleinsäuremolekül der 222 Nukleinsäuremoleküle der Sammlung 222 Nukleinsäuremoleküle nach Anspruch 2 hybridisiert,
wobei die Entsprechung zwischen Oligonukleotidsonden und ihrer entsprechenden Nukleinsäuresequenz in Tabelle 3b dargestellt ist,
wobei das Mikroarray möglicherweise positive und negative Oligonukleotidsonden umfasst, die spezifisch mit positiven und negativen Kontroll-Nukleinsäuremolekülen hybridisieren.

8. Mikroarray nach Anspruch 6 oder 7, umfassend die Oligonukleotidsonden, dargestellt durch die Oligonukleotidsequenzen SEQ ID NR. 415 bis SEQ ID NR. 684, fakultativ umfassend die Oligonukleotidsonden, dargestellt durch die Oligonukleotidsequenzen SEQ ID NR. 415 bis SEQ ID NR. 1617, fakultativ umfassend die oder bestehend aus den Oligonukleotidsonden, dargestellt durch die Oligonukleotidsequenzen SEQ ID NR. 415 bis SEQ ID NR. 2989.

9. Verfahren zur In-vitro- und/oder Ex-vivo-Diagnose einer somatischen Krebserkrankung oder Eierstockkrebs bei einer
Person durch Bestimmen der Gegenwart oder der Schwankung der Menge mindestens eines Nukleinsäuremoleküls einer Gruppe von mindestens 26 Nukleinsäuremolekülen nach Anspruch 1 unter Nukleinsäuren aus einer biologischen Probe der Person, wobei die Gegenwart oder die Schwankungen der Menge des Nukleinsäurenmoleküls in Bezug auf das Fehlen oder die bestimmte Menge des Nukleinsäurenmoleküls in einer von einer gesunden Person isolierten Probe beurteilt wird, umfassend:
- Kontaktieren von Nukleinsäuren der biologischen Probe mit einem Mittel, um die Ausbildung mindestens eines Nukleinsäurekomplexes des Mittels und des mindestens einen Nukleinsäuremoleküls der biologischen Probe der Person zu ermöglichen,
• wobei das Mittel mindestens umfasst:
∘ das Nukleinsäuremolekül,
∘ ein komplementäres Molekül des Nukleinsäuremoleküls,
∘ ein Fragment des Nukleinsäuremoleküls oder
∘ ein Fragment des komplementären Moleküls,
jedes der mindestens 26 Nukleinsäuremoleküle und
• wobei das Nukleinsäuremolekül, das komplementäre Molekül des Nukleinsäuremoleküls oder die Fragmente davon, die in dem Mittel enthalten sind, selektiv mit den mindestens 26 Nukleinsäurenmolekülen hybridisieren können,
- Bestimmen der Gegenwart oder der Schwankung der Menge mindestens eines Nukleinsäurekomplexes als Anzeichen für die Tatsache, dass die Personen an Krebs leidet.

10. Verfahren nach Anspruch 9, wobei das Mittel Nukleinsäuresequenzen enthält, die eine PCR-Amplifikation eines Fragments mindestens eines Nukleinsäuremoleküls der mindestens 26 Nukleinsäuremoleküle ermöglicht,
wobei die PCR-Applikation vorzugsweise eine quantitative reverse Transkriptase-PCR oder ein PCR-Array ist.

11. Verfahren nach Anspruch 9, umfassend
- Kontaktieren von Nukleinsäuren der biologischen Probe mit einem Mittel, wobei das Mittel ein Mikroarray wie nach einem der Ansprüche 6 bis 8 ist, um die Ausbildung mindestens eines Nukleinsäurekomplexes des Mittels und der mindestens einen Nukleinsäure einer Probe einer Person zu ermöglichen,
- Bestimmen der Gegenwart oder der Schwankung der Menge mindestens eines Nukleinsäurekomplexes als Anzeichen für die Tatsache, dass die Personen an Krebs leidet.

12. Verfahren zur In-vitro- und/oder Ex-vivo-Krebsdiagnose bei einer Person durch Bestimmen der Gegenwart oder der Schwankung der Menge mindestens eines Proteins oder eines Fragments davon einer Gruppe von mindestens 26 Proteinen nach Anspruch 3 in der biologischen Probe der Person,
wobei die Gegenwart oder die Schwankungen der Menge des Proteins in Bezug auf das Fehlen oder die bestimmte Menge des Proteins in einer von einer gesunden Person isolierten Probe beurteilt wird, umfassend:
- Kontaktieren von Proteinen der biologischen Probe mit einem Mittel, um die Ausbildung mindestens eines Immunkomplexes des Mittels und des mindestens einen Proteins der biologischen Probe der Person zu ermöglichen,
wobei das Mittel aus mindestens einem Antikörper besteht, der spezifisch mit einem Protein jedes der mindestens 26 Proteine hybridisiert,
- Bestimmen der Gegenwart oder der Schwankung der Menge des mindestens einen Immunkomplexes als Anzeichen für die Tatsache, dass die Person an Krebs leidet, wobei der Immunkomplex vorzugsweise durch die Immunhistochemie, Immunzytochemie, Immunfluoreszenz, Western-Blotting und Immunpräzipitation bestimmt wird.

13. Verfahren zur In-vitro- und/oder Ex-vivo-Krebsdiagnose bei einer Person durch Bestimmen der Gegenwart oder der Schwankung der Menge mindestens eines Antikörpers einer Gruppe von mindestens 26 Antikörpern nach Anspruch 5, die spezifisch mindestens 26 Proteine nach Anspruch 3 erkennen, in der biologischen Probe der Person,
wobei die Gegenwart oder die Schwankungen der Menge des Antikörpers in Bezug auf das Fehlen oder die bestimmte Menge des Antikörpers in einer von einer gesunden Person isolierten Probe beurteilt wird, umfassend:
- Kontaktieren der biologischen Probe der Personen mit einem Mittel, um die Ausbildung mindestens eines Immunkomplexes des Mittels und des mindestens einen Antikörpers der biologischen Probe der Person zu ermöglichen,
wobei das Mittel aus mindestens 26 Proteinen besteht, die spezifisch mit den mindestens 26 Antikörpern hybridisieren können,
- Bestimmen der Gegenwart oder der Schwankung der Menge des mindestens einen Immunkomplexes als Anzeichen für die Tatsache, dass die Person an Krebs leidet, wobei der Immunkomplex vorzugsweise durch die Immunhistochemie, Immunzytochemie, Immunfluoreszenz, Western-Blotting und Immunpräzipitation bestimmt wird.

14. Kit zur In-vitro- und/oder Ex-vivo-Krebsdiagnose, umfassend:
- ein Mikroarray wie nach einem der Ansprüche 6,
- fakultativ Material zur Präparation von Nukleinsäuren der biologischen Probe eines Patienten, bei dem der Verdacht auf Krebs besteht, insbesondere der Präparation von cDNAs,
- fakultativ markierte Moleküle zum Markieren der Nukleinsäuren,
- fakultativ eine negative Kontrolle, die Nukleinsäuren einer biologischen Probe einer gesunden Person entspricht.

15. Kit zur In-vitro- und/oder Ex-vivo-Krebsdiagnose, umfassend:
- ELISA-Unterstützung, bestehend aus mindestens 26 Proteinen nach Anspruch 3 oder Fragmenten davon,
- fakultativ markierte Antikörper, die gegen Antikörper gerichtet sind, die spezifisch die Proteine erkennen, wobei das Protein wahrscheinlich in einer Probe von einem Patienten, bei dem der Verdacht auf Krebs besteht, vorhanden ist,
- fakultativ eine negative Kontrolle, die Antikörpern einer Probe einer gesunden Person entspricht.

16. Kit zur In-vitro- und/oder Ex-vivo-Krebsdiagnose, umfassend:
- ELISA-Unterstützung, bestehend aus mindestens 26 Antikörpern, die spezifisch mindestens 26 Proteine nach Anspruch 3 oder Fragmenten davon erkennen,
- fakultativ ein markierter Antikörper, der gegen ein Protein gerichtet ist, das spezifisch von dem Antikörper erkannt wird, wobei der Antikörper wahrscheinlich in einer Probe von einem Patienten, bei dem der Verdacht auf Krebs besteht, vorhanden ist,
- fakultativ eine negative Kontrolle, die Polypeptiden einer Probe einer gesunden Person entspricht.

## Revendications

1. Utilisation d'au moins un jeu d'acides nucléiques choisi parmi:
- Un jeu comprenant au moins 26 acides nucléiques, lesdits 26 acides nucléiques possédant les séquences d'acides nucléiques SEQ ID NO : 1, 3, 5, 7, 9, 11, 13, 15, 17, 19, 21, 23, 25, 27, 29, 31, 33, 35, 37, 39, 41, 43, 45, 47, 49 et 51,
- Un jeu comprenant au moins 26 acides nucléiques complémentaires desdits au moins 26 acides nucléiques
pour le diagnostic *in vitro* ou *ex vivo* de n'importe quels types de cancers somatiques ou ovariens, lesdits cancers somatiques étant des tumeurs solides ou des néoplasmes hématologiques,
où :
- les cellules cancéreuses de chaque type de cancer somatique ou ovarien expriment anormalement au moins un acide nucléique du jeu d'acides nucléiques ci-dessus, et
- au moins un acide nucléique du jeu d'acides nucléiques ci-dessus est anormalement exprimé dans les cellules cancéreuses d'au moins un type de cancers somatiques ou ovariens

2. Utilisation d'au moins un jeu d'acides nucléiques selon la revendication 1,
où ledit jeu d'acides nucléiques comprend au moins 59 acides nucléiques, lesdits au moins 59 acides nucléiques possédant les séquences d'acides nucléiques SEQ ID NO : 1, 3, 5, 7, 9, 11, 13, 15, 17, 19, 21, 23, 25, 27, 29, 31, 33, 35, 37, 39, 41, 43, 45, 47, 49, 51, 53, 55, 57, 59, 61, 63, 65, 67, 69, 71, 73, 75, 77, 79, 81, 83, 85, 87, 89, 91, 93, 95, 97, 99, 101, 103, 105, 107, 109, 111, 113, 385 et 386,
éventuellement, où ledit jeu d'acides nucléiques comprend au moins 93 acides nucléiques, lesdits au moins 93 acides nucléiques possédant les séquences d'acides nucléiques SEQ ID NO : 1, 3, 5, 7, 9, 11, 13, 15, 17, 19, 21, 23, 25, 27, 29, 31, 33, 35, 37, 39, 41, 43, 45, 47, 49, 51, 53, 55, 57, 59, 61, 63, 65, 67, 69, 71, 73, 75, 77, 79, 81, 83, 85, 87, 89, 91, 93, 95, 97, 99, 101, 103, 105, 107, 109, 111, 113, 385, 386, 115, 117, 119, 121, 123, 125, 127, 129, 131, 133, 135, 137, 139, 141, 143, 145, 147, 149, 151, 153, 155, 157, 159, 161, 163, 165, 167, 169, 171, 173, 175, 387, 388 et 389,
éventuellement, où ledit jeu d'acides nucléiques comprend au moins 108 acides nucléiques, lesdits au moins 108 acides nucléiques possédant les séquences d'acides nucléiques SEQ ID NO : 1, 3, 5, 7, 9, 11, 13, 15, 17, 19, 21, 23, 25, 27, 29, 31, 33, 35, 37, 39, 41, 43, 45, 47, 49, 51, 53, 55, 57, 59, 61, 63, 65, 67, 69, 71, 73, 75, 77, 79, 81, 83, 85, 87, 89, 91, 93, 95, 97, 99, 101, 103, 105, 107, 109, 111, 113, 385, 386, 115, 117, 119, 121, 123, 125, 127, 129, 131, 133, 135, 137, 139, 141, 143, 145, 147, 149, 151, 153, 155, 157, 159, 161, 163, 165, 167, 169, 171, 173, 175, 387, 388, 389, 177, 179, 181, 183, 185, 187, 189, 191, 193, 195, 197, 199, 201, 203 et 205,
éventuellement, où ledit jeu d'acides nucléiques comprend au moins 128 acides nucléiques, lesdits au moins 128 acides nucléiques possédant les séquences d'acides nucléiques SEQ ID NO : 1, 3, 5, 7, 9, 11, 13, 15, 17, 19, 21, 23, 25, 27, 29, 31, 33, 35, 37, 39, 41, 43, 45, 47, 49, 51, 53, 55, 57, 59, 61, 63, 65, 67, 69, 71, 73, 75, 77, 79, 81, 83, 85, 87, 89, 91, 93, 95, 97, 99, 101, 103, 105, 107, 109, 111, 113, 385, 386, 115, 117, 119, 121, 123, 125, 127, 129, 131, 133, 135, 137, 139, 141, 143, 145, 147, 149, 151, 153, 155, 157, 159, 161, 163, 165, 167, 169, 171, 173, 175, 387, 388, 389, 177, 179, 181, 183, 185, 187, 189, 191, 193, 195, 197, 199, 201, 203, 205, 207, 209, 211, 213, 215, 217, 219, 221, 223, 225, 227, 229, 231, 233, 235, 237, 239, 241, 390 et391,
éventuellement, où ledit jeu d'acides nucléiques comprend au moins 160 acides nucléiques, lesdits au moins 160 acides nucléiques possédant les séquences d'acides nucléiques SEQ ID NO : 1, 3, 5, 7, 9, 11, 13, 15, 17, 19, 21, 23, 25, 27, 29, 31, 33, 35, 37, 39, 41, 43, 45, 47, 49, 51, 53, 55, 57, 59, 61, 63, 65, 67, 69, 71, 73, 75, 77, 79, 81, 83, 85, 87, 89, 91, 93, 95, 97, 99, 101, 103, 105, 107, 109, 111, 113, 385, 386, 115, 117, 119, 121, 123, 125, 127, 129, 131, 133, 135, 137, 139, 141, 143, 145, 147, 149, 151, 153, 155, 157, 159, 161, 163, 165, 167, 169, 171, 173, 175, 387, 388, 389, 177, 179, 181, 183, 185, 187, 189, 191, 193, 195, 197, 199, 201, 203, 205, 207, 209, 211, 213, 215, 217, 219, 221, 223, 225, 227, 229, 231, 233, 235, 237, 239, 241, 390, 391, 243, 245, 247, 249, 251, 253, 255, 257, 259, 261, 263, 265, 267, 269, 271, 273, 275, 277, 279, 281, 283, 285, 287, 392, 393, 394, 395, 396, 397, 398, 399 et 400,
éventuellement, où ledit jeu d'acides nucléiques comprend au moins 166 acides nucléiques, lesdits au moins 166 acides nucléiques possédant les séquences d'acides nucléiques SEQ ID NO : 1, 3, 5, 7, 9, 11, 13, 15, 17, 19, 21, 23, 25, 27, 29, 31, 33, 35, 37, 39, 41, 43, 45, 47, 49, 51, 53, 55, 57, 59, 61, 63, 65, 67, 69, 71, 73, 75, 77, 79, 81, 83, 85, 87, 89, 91, 93, 95, 97, 99, 101, 103, 105, 107, 109, 111, 113, 385, 386, 115, 117, 119, 121, 123, 125, 127, 129, 131, 133, 135, 137, 139, 141, 143, 145, 147, 149, 151, 153, 155, 157, 159, 161, 163, 165, 167, 169, 171, 173, 175, 387, 388, 389, 177, 179, 181, 183, 185, 187, 189, 191, 193, 195, 197, 199, 201, 203, 205, 207, 209, 211, 213, 215, 217, 219, 221, 223, 225, 227, 229, 231, 233, 235, 237, 239, 241, 390, 391, 243, 245, 247, 249, 251, 253, 255, 257, 259, 261, 263, 265, 267, 269, 271, 273, 275, 277, 279, 281, 283, 285, 287, 392, 393, 394, 395, 396, 397, 398, 399, 400, 289, 291, 293, 295, 297 et 299,
éventuellement, où ledit jeu d'acides nucléiques comprend au moins 179 acides nucléiques, lesdits au moins 179 acides nucléiques possédant les séquences d'acides nucléiques SEQ ID NO : 1, 3, 5, 7, 9, 11, 13, 15, 17, 19, 21, 23, 25, 27, 29, 31, 33, 35, 37, 39, 41, 43, 45, 47, 49, 51, 53, 55, 57, 59, 61, 63, 65, 67, 69, 71, 73, 75, 77, 79, 81, 83, 85, 87, 89, 91, 93, 95, 97, 99, 101, 103, 105, 107, 109, 111, 113, 385, 386, 115, 117, 119, 121, 123, 125, 127, 129, 131, 133, 135, 137, 139, 141, 143, 145, 147, 149, 151, 153, 155, 157, 159, 161, 163, 165, 167, 169, 171, 173, 175, 387, 388, 389, 177, 179, 181, 183, 185, 187, 189, 191, 193, 195, 197, 199, 201, 203, 205, 207, 209, 211, 213, 215, 217, 219, 221, 223, 225, 227, 229, 231, 233, 235, 237, 239, 241, 390, 391, 243, 245, 247, 249, 251, 253, 255, 257, 259, 261, 263, 265, 267, 269, 271, 273, 275, 277, 279, 281, 283, 285, 287, 392, 393, 394, 395, 396, 397, 398, 399, 400, 289, 291, 293, 295, 297, 299, 301, 303, 305, 307, 309, 311, 313, 315, 317, 319, 321, 323 et 325,
éventuellement, où ledit jeu d'acides nucléiques comprend au moins 213 acides nucléiques, lesdits au moins 213 acides nucléiques possédant les séquences d'acides nucléiques SEQ ID NO : 1, 3, 5, 7, 9, 11, 13, 15, 17, 19, 21, 23, 25, 27, 29, 31, 33, 35, 37, 39, 41, 43, 45, 47, 49, 51, 53, 55, 57, 59, 61, 63, 65, 67, 69, 71, 73, 75, 77, 79, 81, 83, 85, 87, 89, 91, 93, 95, 97, 99, 101, 103, 105, 107, 109, 111, 113, 385, 386, 115, 117, 119, 121, 123, 125, 127, 129, 131, 133, 135, 137, 139, 141, 143, 145, 147, 149, 151, 153, 155, 157, 159, 161, 163, 165, 167, 169, 171, 173, 175, 387, 388, 389, 177, 179, 181, 183, 185, 187, 189, 191, 193, 195, 197, 199, 201, 203, 205, 207, 209, 211, 213, 215, 217, 219, 221, 223, 225, 227, 229, 231, 233, 235, 237, 239, 241, . 390, 391, 243, 245, 247, 249, 251, 253, 255, 257, 259, 261, 263, 265, 267, 269, 271, 273, 275, 277, 279, 281, 283, 285, 287, 392, 393, 394, 395, 396, 397, 398, 399, 400, 289, 291, 293, 295, 297, 299, 301, 303, 305, 307, 309, 311, 313, 315, 317, 319, 321, 323, 325, 327, 329, 331, 333, 335, 337, 339, 341, 343, 345, 347, 349, 351, 353, 355, 357, 359, 361, 363, 365, 367, 369, 371, 401, 402, 403, 404, 405, 406, 407, 408, 409, 410 et 411,
éventuellement, où ledit jeu d'acides nucléiques comprend au moins 222 acides nucléiques, lesdits au moins 222 acides nucléiques possédant les séquences d'acides nucléiques SEQ ID NO : 1, 3, 5, 7, 9, 11, 13, 15, 17, 19, 21, 23, 25, 27, 29, 31, 33, 35, 37, 39, 41, 43, 45, 47, 49, 51, 53, 55, 57, 59, 61, 63, 65, 67, 69, 71, 73, 75, 77, 79, 81, 83, 85, 87, 89, 91, 93, 95, 97, 99, 101, 103, 105, 107, 109, 111, 113, 385, 386, 115, 117, 119, 121, 123, 125, 127, 129, 131, 133, 135, 137, 139, 141, 143, 145, 147, 149, 151, 153, 155, 157, 159, 161, 163, 165, 167, 169, 171, 173, 175, 387, 388, 389, 177, 179, 181, 183, 185, 187, 189, 191, 193, 195, 197, 199, 201, 203, 205, 207; 209, 211, 213, 215, 217, 219, 221, 223, 225, 227, 229, 231, 233, 235, 237, 239, 241, 390, 391, 243, 245, 247, 249, 251, 253, 255, 257, 259, 261, 263, 265, 267, 269, 271, 273, 275, 277, 279, 281, 283, 285, 287, 392, 393, 394, 395, 396, 397, 398, 399, 400, 289, 291, 293, 295, 297, 299, 301, 303, 305, 307, 309, 311, 313, 315, 317, 319, 321, 323, 325, 327, 329, 331, 333, 335, 337, 339, 341, 343, 345, 347, 349, 351, 353, 355, 357, 359, 361, 363, 365, 367, 369, 371, 401, 402, 403, 404, 405, 406, 407, 408, 409, 410, 411, 373, 375, 377, 379, 381, 383, 412, 413 et 414.

3. Utilisation d'au moins un jeu d'acides aminés choisis parmi :
- Un jeu comprenant au moins 26 protéines
- Un jeu comprenant au moins un fragment de chacune desdites au moins 26 protéines, ledit fragment pouvant être reconnu par un anticorps spécifiquement dirigé contre une protéine à partir de laquelle ledit fragment dérive
lesdites au moins 26 protéines étant codées par au moins 26 acides nucléiques selon la revendication 1, et lesdites au moins 26 protéines possédant les séquences d'acides aminés SEQ ID NO : 2, 4, 6, 8, 10, 12, 14, 16, 18, 20, 22, 24, 26, 28, 30, 32, 34, 36, 38, 40, 42, 44, 46, 48, 50 et 52,
chaque acide aminé contenu dans un jeu donné défini ci-dessus étant spécifiquement reconnu par au moins un anticorps spécifique,
pour le diagnostic *in vitro* ou *ex vivo* de n'importe quels types de cancers somatiques ou ovariens, lesdits cancers somatiques étant des tumeurs solides ou des néoplasmes hématologiques,
où,
- un échantillon biologique d'un patient atteint par n'importe quels types de cancers somatiques ou ovariens présente un niveau anormal d'au moins un anticorps reconnaissant spécifiquement un acide aminé du jeu d'acides aminés ci-dessus, et
- au moins un anticorps qui reconnaît spécifiquement un acide aminé des susdits jeux d'acides aminés est présent à un niveau anormal dans un échantillon biologique d'un patient atteint par au moins un type de cancer somatique ou ovarien

4. Utilisation d'au moins un jeu d'acides aminés selon la revendication 3,
où ledit jeu de protéines comprend au moins 57 protéines, lesdites au moins 57 protéines possédant les séquences d'acides aminés SEQ ID NO : 2, 4, 6, 8, 10, 12, 14, 16, 18, 20, 22, 24, 26, 28, 30, 32, 34, 36, 38, 40, 42, 44, 46, 48, 50, 52, 54, 56, 58, 60, 62, 64, 66, 68, 70, 72, 74, 76, 78, 80, 82, 84, 86, 88, 90, 92, 94, 96, 98, 100, 102, 104, 106, 108, 110, 112 et 114,
éventuellement, où ledit jeu de protéines comprend au moins 88 protéines, lesdites au moins 88 protéines possédant les séquences d'acides aminés SEQ ID NO : 2, 4, 6, 8, 10, 12, 14, 16, 18, 20, 22, 24, 26, 28, 30, 32, 34, 36, 38, 40, 42, 44, 46, 48, 50, 52, 54, 56, 58, 60, 62, 64, 66, 68, 70, 72, 74, 76, 78, 80, 82, 84, 86, 88, 90, 92, 94, 96, 98, 100, 102, 104, 106, 108, 110, 112, 114, 116, 118, 120, 122, 124, 126, 128, 130, 132, 134, 136, 138, 140, 142, 144, 146, 148, 150, 152, 154, 156, 158, 160, 162, 164, 166, 168, 170, 172, 174 et 176,
éventuellement, où ledit jeu de protéines comprend au moins 103 protéines, lesdites au moins 103 protéines possédant les séquences d'acides aminés SEQ ID NO : 2, 4, 6, 8, 10, 12, 14, 16, 18, 20, 22, 24, 26, 28, 30, 32, 34, 36, 38, 40, 42, 44, 46, 48, 50, 52, 54, 56, 58, 60, 62, 64, 66, 68, 70, 72, 74, 76, 78, 80, 82, 84, 86, 88, 90, 92, 94, 96, 98, 100, 102, 104, 106, 108, 110, 112, 114, 116, 118, 120, 122, 124, 126, 128, 130, 132, 134, 136, 138, 140, 142, 144, 146, 148, 150, 152, 154, 156, 158, 160, 162, 164, 166, 168, 170, 172, 174, 176, 178, 180, 182, 184, 186, 188, 190, 192, 194, 196, 198, 200, 202, 204 et 206,
éventuellement, où ledit jeu de protéines comprend au moins 121 protéines, lesdites au moins 121 protéines possédant les séquences d'acides aminés SEQ ID NO : 2, 4, 6, 8, 10, 12, 14, 16, 18, 20, 22, 24, 26, 28, 30, 32, 34, 36, 38, 40, 42, 44, 46, 48, 50, 52, 54, 56, 58, 60, 62, 64, 66, 68, 70, 72, 74, 76, 78, 80, 82, 84, 86, 88, 90, 92, 94, 96, 98, 100, 102, 104, 106, 108, 110, 112, 114, 116, 118, 120, 122, 124, 126, 128, 130, 132, 134, 136, 138, 140, 142, 144, 146, 148, 150, 152, 154, 156, 158, 160, 162, 164, 166, 168, 170, 172, 174, 176, 178, 180, 182, 184, 186, 188, 190, 192, 194, 196, 198, 200, 202, 204, 206, 208, 210, 212, 214, 216, 218, 220, 222, 224, 226, 228, 230, 232, 234, 236, 238, 240 et 242,
éventuellement, où ledit jeu de protéines comprend au moins 144 protéines, lesdites au moins 144 protéines possédant les séquences d'acides aminés SEQ ID NO : 2, 4, 6, 8, 10, 12, 14, 16, 18, 20, 22, 24, 26, 28, 30, 32, 34, 36, 38, 40, 42, 44, 46, 48, 50, 52, 54, 56, 58, 60, 62, 64, 66, 68, 70, 72, 74, 76, 78, 80, 82, 84, 86, 88, 90, 92, 94, 96, 98, 100, 102, 104, 106, 108, 110, 112, 114, 116, 118, 120, 122, 124, 126, 128, 130, 132, 134, 136, 138, 140, 142, 144, 146, 148, 150, 152, 154, 156, 158, 160, 162, 164, 166, 168, 170, 172, 174, 176, 178, 180, 182, 184, 186, 188, 190, 192, 194, 196, 198, 200, 202, 204, 206, 208, 210, 212, 214, 216, 218, 220, 222, 224, 226, 228, 230, 232, 234, 236, 238, 240, 242, 244, 246, 248, 250, 252, 254, 256, 258, 260, 262, 264, 266, 268, 270, 272, 274, 276, 278, 280, 282, 284, 286 et 288,
éventuellement, où ledit jeu de protéines comprend au moins 150 protéines, lesdites au moins 150 protéines possédant les séquences d'acides aminés SEQ ID NO : 2, 4, 6, 8, 10, 12, 14, 16, 18, 20, 22, 24, 26, 28, 30, 32, 34, 36, 38, 40, 42, 44, 46, 48, 50, 52, 54, 56, 58, 60, 62, 64, 66, 68, 70, 72, 74, 76, 78, 80, 82, 84, 86, 88, 90, 92, 94, 96, 98, 100, 102, 104, 106, 108, 110, 112, 114, 116, 118, 120, 122, 124, 126, 128, 130, 132, 134, 136, 138, 140, 142, 144, 146, 148, 150, 152, 154, 156, 158, 160, 162, 164, 166, 168, 170, 172, 174, 176, 178, 180, 182, 184, 186, 188, 190, 192, 194, 196, 198, 200, 202, 204, 206, 208, 210, 212, 214, 216, 218, 220, 222, 224, 226, 228, 230, 232, 234, 236, 238, 240, 242, 244, 246, 248, 250, 252, 254, 256, 258, 260, 262, 264, 266, 268, 270, 272, 274, 276, 278, 280, 282, 284, 286, 288, 290, 292, 294, 296, 298 et 300,
éventuellement, où ledit jeu de protéines comprend au moins 163 protéines, lesdites au moins 163 protéines possédant les séquences d'acides aminés SEQ ID NO : 2, 4, 6, 8, 10, 12, 14, 16, 18, 20, 22, 24, 26, 28, 30, 32, 34, 36, 38, 40, 42, 44, 46, 48, 50, 52, 54, 56, 58, 60, 62, 64, 66, 68, 70, 72, 74, 76, 78, 80, 82, 84, 86, 88, 90, 92, 94, 96, 98, 100, 102, 104, 106, 108, 110, 112, 114, 116, 118, 120, 122, 124, 126, 128, 130, 132, 134, 136, 138, 140, 142, 144, 146, 148, 150, 152, 154, 156, 158, 160, 162, 164, 166, 168, 170, 172, 174, 176, 178, 180, 182, 184, 186, 188, 190, 192, 194, 196, 198, 200, 202, 204, 206, 208, 210, 212, 214, 216, 218, 220, 222, 224, 226, 228, 230, 232, 234, 236, 238, 240, 242, 244, 246, 248, 250, 252, 254, 256, 258, 260, 262, 264, 266, 268, 270, 272, 274, 276, 278, 280, 282, 284, 286, 288, 290, 292, 294, 296, 298, 300, 302, 304, 306, 308, 310, 312, 314, 316, 318, 320, 322, 324 et 326,
éventuellement, où ledit jeu de protéines comprend au moins 186 protéines, lesdites au moins 186 protéines possédant les séquences d'acides aminés SEQ ID NO : 2, 4, 6, 8, 10, 12, 14, 16, 18, 20, 22, 24, 26, 28, 30, 32, 34, 36, 38, 40, 42, 44, 46, 48, 50, 52, 54, 56, 58, 60, 62, 64, 66, 68, 70, 72, 74, 76, 78, 80, 82, 84, 86, 88, 90, 92, 94, 96, 98, 100, 102, 104, 106, 108, 110, 112, 114, 116, 118, 120, 122, 124, 126, 128, 130, 132, 134, 136, 138, 140, 142, 144, 146, 148, 150, 152, 154, 156, 158, 160, 162, 164, 166, 168, 170, 172, 174, 176, 178, 180, 182, 184, 186, 188, 190, 192, 194, 196, 198, 200, 202, 204, 206, 208, 210, 212, 214, 216, 218, 220, 222, 224, 226, 228, 230, 232, 234, 236, 238, 240, 242, 244, 246, 248, 250, 252, 254, 256, 258, 260, 262, 264, 266, 268, 270, 272, 274, 276, 278, 280, 282, 284, 286, 288, 290, 292, 294, 296, 298, 300, 302, 304, 306, 308, 310, 312, 314, 316, 318, 320, 322, 324, 326, 328, 330, 332, 334, 336, 338, 340, 342, 344, 346, 348, 350, 352, 354, 356, 358, 360, 362, 364, 366, 368, 370 et 372,
éventuellement, où ledit jeu de protéines comprend au moins 192 protéines, lesdites au moins 192 protéines possédant les séquences d'acides aminés SEQ ID NO : 2, 4, 6, 8, 10, 12, 14, 16, 18, 20, 22, 24, 26, 28, 30, 32, 34, 36, 38, 40, 42, 44, 46, 48, 50, 52, 54, 56, 58; 60, 62, 64, 66, 68, 70, 72, 74, 76, 78, 80, 82, 84, 86, 88, 90, 92, 94, 96, 98, 100, 102, 104, 106, 108, 110, 112, 114, 116, 118, 120, 122, 124, 126, 128, 130, 132, 134, 136, 138, 140, 142, 144, 146, 148, 150, 152, 154, 156, 158, 160, 162, 164, 166, 168, 170, 172, 174, 176, 178, 180, 182, 184, 186, 188, 190, 192, 194, 196, 198, 200, 202, 204, 206, 208, 210, 212, 214, 216, 218, 220, 222, 224, 226, 228, 230, 232, 234, 236, 238, 240, 242, 244, 246, 248, 250, 252, 254, 256, 258, 260, 262, 264, 266, 268, 270, 272, 274, 276, 278, 280, 282, 284, 286, 288, 290, 292, 294, 296, 298, 300, 302, 304, 306, 308, 310, 312, 314, 316, 318, 320, 322, 324, 326, 328, 330, 332, 334, 336, 338, 340, 342, 344, 346, 348, 350, 352, 354, 356, 358, 360, 362, 364, 366, 368, 370, 372, 374, 376, 378, 380, 382 et 384.

5. Utilisation d'un jeu d'au moins 26 anticorps, éventuellement un jeu de 57 anticorps, éventuellement un jeu de 88 anticorps, éventuellement un jeu de 103 anticorps, éventuellement un jeu de 121 anticorps, éventuellement un jeu de 150 anticorps, éventuellement un jeu de 163 anticorps, éventuellement un jeu de 186 anticorps, éventuellement un jeu de 192 anticorps, **caractérisé en ce que** chaque anticorps d'un jeu d'anticorps donné susmentionné reconnaisse spécifiquement un acide aminé d'un jeu d'acides aminés tel que défini dans les revendications 3 ou 4, et chaque acide aminé d'un jeu donné d'acides aminés tel que défini dans les revendications 3 ou 4 est reconnu spécifiquement par un anticorps dudit jeu d'anticorps donné.
pour le diagnostic *in vitro* ou *ex vivo* de n'importe quels types de cancers somatiques ou ovariens,
où :
- les cellules cancéreuses de chaque type de cancer somatique ou ovarien expriment anormalement au moins un acide aminé reconnu par un anticorps du jeu d'anticorps ci-dessus, et
- au moins un acide aminé reconnu par un anticorps des jeux d'anticorps ci-dessus est anormalement exprimé dans les cellules cancéreuses d'au moins un type de cancers somatiques ou ovariens

6. Puce comprenant au moins 32 sondes d'oligonucléotides possédant les séquences d'oligonucléotides SEQ ID NO 415 à 446, chacune desdi tes au moins 32 sondes d'oligonucléotides s'hybridant spécifiquement avec un acide nucléique d'au moins un jeu d'acides nucléiques selon la revendication 1, la correspondance entre les sondes d'oligonucléotides et leur séquence d'acide nucléique correspondante étant représentée dans le tableau 3a

7. Puce selon la revendication 6, comprenant au moins 70 sondes d'oligonucléotides possédant les séquences d'oligonucléotides SEQ ID NO 415 à 484, chacune desdites au moins 70 sondes d'oligonucléotides s'hybridant spécifiquement avec un acide nucléique parmi les 59 acides nucléiques tels que définis dans la revendication 2,
éventuellement, comprenant au moins 110 sondes d'oligonucléotides possédant les séquences d'oligonucléotides SEQ ID NO 415 à 524, chacune desdites au moins 110 sondes d'oligonucléotides s'hybridant spécifiquement avec un acide nucléique parmi les 93 acides nucléiques tels que définis dans la revendication 2,
éventuellement, comprenant au moins 130 sondes d'oligonucléotides possédant les séquences d'oligonucléotides SEQ ID NO 415 à 544, chacune desdites au moins 130 sondes d'oligonucléotides s'hybridant spécifiquement avec un acide nucléique parmi les 108 acides nucléiques tels que définis dans la revendication 2,
éventuellement, comprenant au moins 154 sondes d'oligonucléotides possédant les séquences d'oligonucléotides SEQ ID NO 415 à 568, chacune desdites au moins 154 sondes d'oligonucléotides s'hybridant spécifiquement avec un acide nucléique parmi les 128 acides nucléiques tels que définis dans la revendication 2,
éventuellement, comprenant au moins 197 sondes d'oligonucléotides possédant les séquences d'oligonucléotides SEQ ID NO 415 à 611, chacune desdites au moins 197 sondes d'oligonucléotides s'hybridant spécifiquement avec un acide nucléique parmi les 160 acides nucléiques tels que définis dans la revendication 2,
éventuellement, comprenant au moins 204 sondes d'oligonucléotides possédant les séquences d'oligonucléotides SEQ ID NO 415 à 618, chacune desdites au moins 204 sondes d'oligonucléotides s'hybridant spécifiquement avec un acide nucléique parmi les 166 acides nucléiques tels que définis dans la revendication 2,
éventuellement, comprenant au moins 220 sondes d'oligonucléotides possédant les séquences d'oligonucléotides SEQ ID NO 415 à 634, chacune desdites au moins 220 sondes d'oligonucléotides s'hybridant spécifiquement avec un acide nucléique parmi les 179 acides nucléiques tels que définis dans la revendication 2,
éventuellement, comprenant au moins 261 sondes d'oligonucléotides possédant les séquences d'oligonucléotides SEQ ID NO 415 à 675, chacune desdites au moins 261 sondes d'oligonucléotides s'hybridant spécifiquement avec un acide nucléique parmi les 213 acides nucléiques tels que définis dans la revendication 2,
éventuellement, comprenant au moins 270 sondes d'oligonucléotides possédant les séquences d'oligonucléotides SEQ ID NO 415 à 684, chacune desdites au moins 270 sondes d'oligonucléotides s'hybridant spécifiquement avec un acide nucléique parmi les 222 acides nucléiques tels que définis dans la revendication 2,
la correspondance entre les sondes d'oligonucléotides et leur gène correspondant étant représentée dans le tableau 3b,
ladite puce pouvant comprendre des sondes d'oligonucléotides positives et négatives s'hybridant spécifiquement avec des acides nucléiques contrôles positifs et négatifs

8. Puce selon les revendications 6 ou 7, comprenant les sondes d'oligonucléotides possédant les séquences d'oligonucléotides SEQ ID NO 415 à SEQ ID NO 684,
- éventuellement comprenant les sondes d'oligonucléotides possédant les séquences d'oligonucléotides SEQ ID NO 415 à SEQ ID NO 1617, éventuellement comprenant ou consistant en les sondes d'oligonucléotides possédant les séquences d'oligonucléotides SEQ ID NO 415 à SEQ ID NO 2989

9. Méthode pour le diagnostic *in vitro* et/ou *ex vivo* du cancer somatique ou ovarien chez un sujet, en déterminant, parmi les acides nucléiques provenant d'un échantillon biologique du sujet, la présence ou la variation du niveau d'au moins un acide nucléique parmi un groupe d'au moins 26 acides nucléiques tel que défini dans la revendication 1,
ladite présence ou variation du niveau dudit acide nucléique étant évaluée par rapport à l'absence ou au niveau donné dudit acide nucléique provenant d'un échantillon isolé d'un individu sain, comprenant:
- la mise en contact des acides nucléiques provenant de l'échantillon biologique avec un agent pour permettre la formation d'au moins un complexe d'acide nucléique entre ledit agent et ledit au moins acide nucléique provenant de l'échantillon biologique du sujet,
∘ ledit agent comprenant au moins :
▪ ledit acide nucléique
▪ une molécule complémentaire dudit acide nucléique
▪ un fragment dudit acide nucléique
▪ un fragment de ladite molécule complémentaire de chacun desdits au moins 26 acides nucléiques, et
∘ L'acide nucléique, la molécule complémentaire dudit acide nucléique ou les fragments de ceux-ci, contenus dans ledit agent étant capables de s'hybrider sélectivement avec lesdits au moins 26 acides nucléiques
- la détermination de la présence ou de la variation du niveau d'au moins un complexe d'acide nucléique indiquant le fait que le sujet est atteint d'un cancer

10. Méthode selon la revendication 9, où ledit agent contient les séquences d'acides nucléiques qui permettent une amplification par PCR d'un fragment d'un moins un acide nucléique parmi lesdits au moins 26 acides nucléiques ,
ladite amplification par PCR étant de préférence une PCR quantitative après transcription inverse (RT-PCR quantitative), PCR array

11. Méthode selon la revendication 9, comprenant
- La mise en contact des acides nucléiques provenant de l'échantillon biologique avec un agent, ledit agent étant une puce telle que définie dans n'importe lesquelles des revendications 6 à 8, pour permettre la formation d'au moins un complexe d'acide nucléique, entre ledit agent et au moins un acide nucléique provenant d'un échantillon d'un sujet,
- La détermination de la présence ou de la variation du niveau d'au moins un complexe d'acide nucléique indiquant le fait que le sujet est atteint d'un cancer

12. Méthode pour le diagnostic *in vitro* et/ou *ex vivo* du cancer chez un sujet, en déterminant la présence ou la variation du niveau d'au moins une protéine, ou d'un fragment de celle-ci, parmi un groupe d'au moins 26 protéines tel que défini dans la revendication 3, dans un échantillon biologique d'un sujet,
ladite présence ou variation du niveau de ladite protéine étant évaluée par rapport à l'absence ou au niveau donné de ladite protéine dans un échantillon isolé d'un sujet sain, comprenant :
- la mise en contact des protéines provenant de l'échantillon biologique avec un agent, pour permettre la formation d'au moins un complexe immun entre ledit agent et ladite au moins une protéine provenant de l'échantillon biologique du sujet,
ledit agent consistant en au moins un anticorps s'hybridant spécifiquement avec une protéine parmi lesdites au moins 26 protéines,
- la détermination de la présence ou de la variation du niveau dudit au moins un complexe immun indiquant le fait que le sujet est atteint d'un cancer, ledit complexe immun étant déterminé de préférence par immunohistochimie, immunocytochimie, immunofluorescence, western blot et immunoprécipitation

13. Méthode pour le diagnostic *in vitro* et/ou *ex vivo* du cancer chez un sujet, en déterminant la présence ou la variation du niveau d'au moins un anticorps parmi un groupe d'au moins 26 anticorps tel que défini dans la revendication 5 qui reconnait spécifiquement au moins 26 protéines telles que définies dans la revendication 3, dans un échantillon biologique d'un sujet,
ladite présence ou variation du niveau dudit anticorps étant évaluée par rapport à l'absence ou au niveau donné dudit anticorps dans un échantillon isolé d'un sujet sain, comprenant :
- la mise en contact de l'échantillon biologique provenant du sujet avec un agent, pour permettre la formation d'au moins un complexe immun entre ledit agent et ledit au moins un anticorps provenant de l'échantillon biologique du sujet,
ledit agent consistant en au moins 26 protéines capables de s'hybrider spécifiquement avec lesdits au moins 26 anticorps,
- la détermination de la présence ou de la variation du niveau dudit au moins un complexe immun indiquant le fait que le sujet est atteint d'un cancer, ledit complexe immun étant déterminé de préférence par immunohistochimie, immunocytochimie, immunofluorescence, western blot et immunoprécipitation

14. Kit pour le diagnostic *in vitro* et/ou *ex vivo* du cancer, comprenant:
- une puce telle que définie dans la revendication 6
- éventuellement le matériel pour la préparation des acides nucléiques de l'échantillon biologique provenant d'un sujet suspecté d'être atteint par un cancer, en particulier la préparation de cDNAs
- éventuellement des molécules marquées pour le marquage desdits acides nucléiques
- éventuellement un contrôle négatif correspondant aux acides nucléiques provenant d'un échantillon biologique d'un sujet sain

15. Kit pour le diagnostic *in vitro* et/ou *ex vivo* du cancer, comprenant :
- un support ELISA consistant en au moins 26 protéines telles que définies dans la revendication 3, ou des fragments de celles-ci
- éventuellement des anticorps marqués dirigés contre des anticorps qui reconnaissent spécifiquement lesdites protéines, ladite protéine pouvant être présente dans un échantillon provenant d'un sujet suspecté d'être atteint par un cancer
- éventuellement un contrôle négatif correspondant aux anticorps provenant d'un échantillon d'un sujet sain

16. Kit pour le diagnostic *in vitro* et/ou *ex vivo* du cancer, comprenant :
- un support ELISA consistant en au moins 26 anticorps qui reconnaissent spécifiquement au moins 26 protéines telles que définies dans la revendication 3, ou des fragments de celles-ci
- éventuellement des anticorps marqués dirigés contre une protéine spécifiquement reconnue par ledit anticorps, ledit anticorps pouvant être présent dans un échantillon provenant d'un sujet suspecté d'être atteint par un cancer
- éventuellement un contrôle négatif correspondant aux polypeptides provenant d'un échantillon d'un sujet sain
